# EUROPEAN PATENT APPLICATION

(11) **EP 2 770 052 A1**
(43) Date of publication of application: **27.08.2014**
(21) Application number: 13190312.2
(22) Date of filing: 06.06.2008
(51) Int. Cl.: C12N 9/22

(54) **Method for enhancing the cleavage activity of I-CreI derived meganucleases**

(30) Priority: 06.06.2007 WO PCT/IB2007/002830; 25.06.2007 WO PCT/IB2007/002881
(62) Divisional of application: 08807170.9
(71) Applicant: Cellectis, 75013 Paris (FR)
(72) Inventor: Grizot, Sylvestre, 92250 La Garenne Colombes (FR); Gouble, Agnès, 75017 Paris (FR); Perez-Michaut, Christophe, 75016 Paris (FR)
(74) Representative: Leblois-Préhaud, Hélène Marthe Georgette

(57) **Abstract**

A method for enhancing the cleavage activity of an I-*Cre*I derived meganuclease, comprising the site-specific mutation of at least one amino acid residue which is selected in the group consisting of: the glycine at position 19, the phenylalanine at position 54, the phenylalanine at position 87, the serine at position 79, the valine at position 105 and the isoleucine at position 132 of I-*Cre*I, and its application for the manufacturing of meganuclease cleaving a DNA target of interest, for use in genome therapy (treatment of genetic diseases) and genome engineering (making of transgenic animals, transgenic plants and recombinant cell lines).

## Description

The invention relates to a method for enhancing the cleavage activity of I-*Cre*I derived meganucleases, and its application for the manufacturing of meganuclease cleaving a DNA target of interest, for use in genome therapy (treatment of genetic diseases) and genome engineering (making of transgenic animals, transgenic plants and recombinant cell lines).

Homologous recombination is the best way to precisely engineer a given locus. Homologous gene targeting strategies have been used to knock out endogenous genes (Capecchi, M. R., Science, 1989, 244, 1288-1292; Smithies, O. Nat. Med., 2001, 7, 1083-1086) or knock-in exogenous sequences in the chromosome. It can as well be used for gene correction, and in principle, for the correction of mutations linked with monogenic diseases. However, this application is in fact difficult, due to the low efficiency of the process (10⁻⁶ to 10⁻⁹ of transfected cells). In the last decade, several methods have been developed to enhance this yield. (De Semir et al., J; Gene Med. 2003, 5, 625-639; De Semir D. and Aran J., Gene Ther. 2002, 9, 683-685; Sangiuolo et al., BMC Med. Genet., 2002, 3, 8-; Goncz et al., Gene Ther., 2001, 8, 961-965). An elegant strategy to enhance the efficiency of recombination is to deliver a DNA double-strand break in the targeted locus, using meganucleases.

In the wild, meganucleases are essentially represented by homing endonucleases. Homing Endonucleases (HEs) are a widespread family of natural meganucleases including hundreds of proteins families (Chevalier, B. S. and B.L. Stoddard, Nucleic Acids Res., 2001, 29, 3757-3774). These proteins are encoded by mobile genetic elements which propagate by a process called "homing": the endonuclease cleaves a cognate allele from which the mobile element is absent, thereby stimulating a homologous recombination event that duplicates the mobile DNA into the recipient locus. Pioneer works have shown that they can be used to cleave unique site in living cells, thereby enhancing gene targeting by 1000-fold or more in the vicinity of the cleavage site (Rouet et al., Mol. Cell. Biol., 1994, 14, 8096-8106; Puchta et al., Proc. Natl. Acad. Sci. U. S. A., 1996, 93, 5055-5060; Puchta et al., Nucleic Acids Res., 1993, 21, 5034-5040; Elliott et al., Mol. Cell. Biol., 1998, 18, 93-101; Cohen-Tannoudji et al., Mol. Cell. Biol., 1998, 18, 1444-1448; Choulika et al., Mol. Cell. Biol., 1995, 15, 1968-1973; Donoho et al., Mol. Cell. Biol., 1998, 18, 4070-4078; Sargent et al., Mol. Cell. Biol., 1997, 17, 267-277). However, the use of this technology is limited by the repertoire of natural meganucleases. Therefore, the making of meganucleases with tailored specificities is under intense investigation, and several laboratories have tried to alter the specificity of natural meganucleases (Chevalier et al., Mol. Cell., 2002, 10, 895-905; Epinat et al., Nucleic Acids Res., 2003, 31, 2952-2962; Arnould et al., J. Mol. Biol., 2006, 355, 443-458; Sussman et al., J. Mol. Biol., 2004, 342, 31-41; Smith et al., Nucleic Acids Res., 2006, 34, e149; Seligman et al., Genetics, 1997, 147, 1653-1664; International PCT Applications WO 031078619, WO 2004/031346, WO 2006/097784, WO 2006/097853.).

Enzymes properties can be modified by evolutionary molecular engineering, which is also called directed evolution or *in vitro* evolution (Arnold, F. H. and J.C. Moore, Adv. Biochem. Eng. Biotechnol., 1997, 58, 1-14; Rubingh, D. N., Curr. Opin. Biotechnol., 1997, 8, 417-422) and several studies have described successful optimization of stability (Giver et al., Proc. Natl. Acad. Sci. U.S.A., 1998, 95, 12809-12813; Zhao, H and F.H. Arnold, Protein. Eng., 1999, 12, 47-53.), activity (Taguchi et al., Appl. Environ. Microbiol., 1998, 64, 492-495), altered substrate specificity (Yano et al., Proc. Natl. Acad. Sci. USA., 1998, 95, 5511-5515), and the ability to interact correctly with surfaces (Egmond et al., Adv. Exp. Med. Biol., 1996, 379, 219-228). Usually, directed evolution relies on a more or less random mutagenesis, by PCR (Cadwell R. C. and G.F. Joyce, PCR Methods Applic., 1992, 2, 28-33.) and DNA shuffling (Temmer, W. P., Proc. Natl. Acad. Sci. USA., 1994, 91, 10747-10751), the variants of interest being identified by an adapted screening process.

Rational design is a totally different strategy that relies on in depth knowledge of structural features and to structure/function relationships (Scrutton et al., Nature, 1990, 343, 38-43; Craik et al., Science, 1985, 228, 291-297). The soaring of computational biology, with the development of powerful software for energy calculation, has given a new impetus to this kind of approach (Schueler-Furman et al., Science, 2005, 310, 638-642). Computational studies could be used to design novel proteins, including meganucleases (Chevalier et al., Mol. Cell. 2002, 10, 895-905; Ashworth et al., Nature, 2006, 441, 656-659). However, many protein engineering studies today are based on an hybrid strategy, sometimes referred to as semi-rational (Chica et al., Curr. Opin. Biotechnol., 2005, 16, 378-384). These studies rely on structural information (Arnould et al., J. Mol. Biol., 2006, 355, 443-458; Santoro, S. W. and P.G. Schultz, Proc. Natl. Acad. Sci. U. S. A., 2002, 99, 4185-4190; Rui et al., J. Biol. Chem., 2004, 279, 46810-46817) and/or computational studies (Hayes et al., Proc. Natl. Acad. Sci. U.S.A., 2002, 99, 15926-15931), to dramatically decrease the complexity of the libraries to be processed.

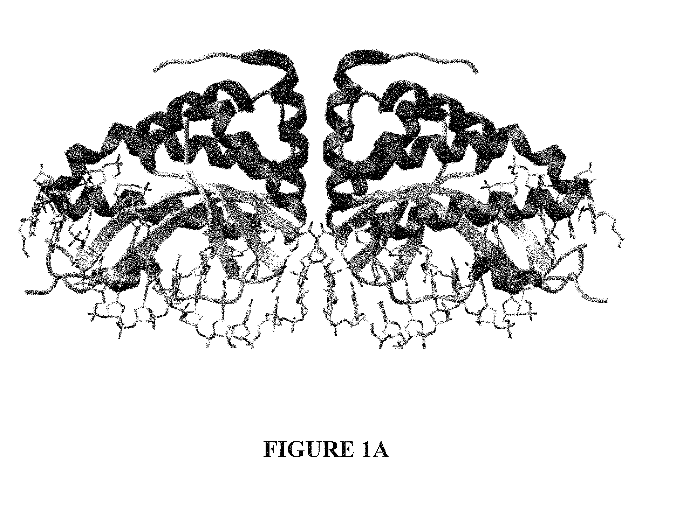

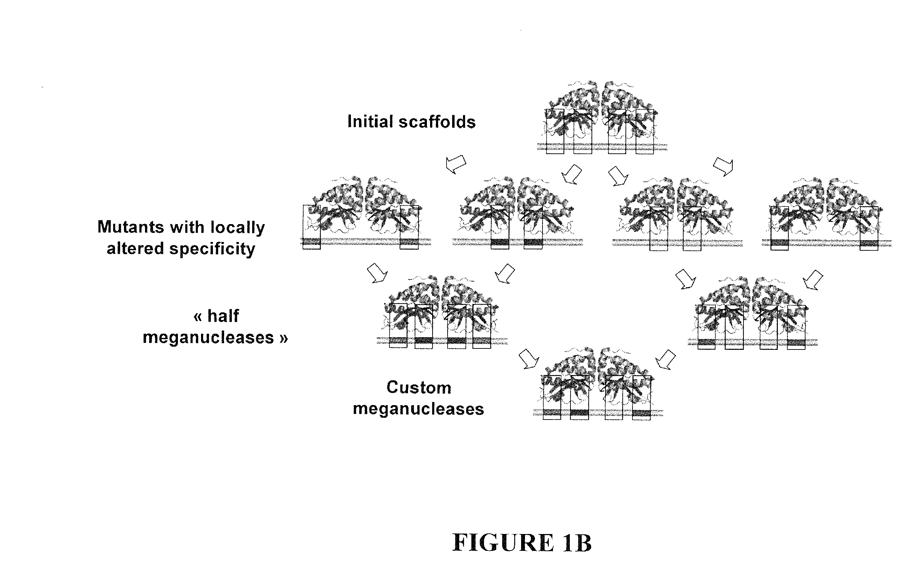

Recently, a two steps strategy was used to tailor the specificity of LAGLIDADG meganucleases, both steps relying on a semi-rational approach (Figure 1). The first step is to locally mutagenize specific' residues in the DNA-binding domain the protein and to identify collections of variants with altered specificity by screening (Arnould et al., J. Mol. Biol., 2006, 355, 443-458; Smith et al., Nucleic Acids Res., 2006, 34, e149; International PCT Applications WO 2006/097784, WO 2006/097853, WO 2007/049156). The second step relies on the modularity of these proteins: it is based on a combinatorial approach, wherein sets of mutations from different locally engineered variants are assembled in order to create globally engineered proteins with predictable specificity (Smith et al., Nucleic Acids Res., 2006, 34, e149; International PCT Application WO 2007/049156). The subdomains that are combined are not totally independent functional units, therefore many different combinations have to be screened in order to find a functional protein with the predicted activity. Therefore, this second step can also be considered as a semi-rational approach.

Although this strategy has shown its power to generate new meganucleases with new specificity towards chosen targets, the cleavage activity of the engineered meganucleases can be weak and therefore may need further engineering steps in order to enhance this activity Although many previous studies have shown that directed evolution, based on random mutagenesis can result in substantial improvement of protein properties (Arnould et al., J. Mol. Biol., Epub, 10 May 2007), such experiment can be time consuming and labour intensive. Thus, the identification of "portable" specific mutations, improving the activity of natural or engineered meganucleases, independently of their substrate specificity would be extremely helpful for manufacturing novel efficient meganucleases.

In addition to the efficacy issue, specificity is another important feature for many applications, and especially for therapeutic ones. Although the I-*Sce*I homing endonuclease has been shown to be less toxic than ZFPs (Alwin et al., Mol. Ther., 2005, 12, 610-617; Porteus M.H. and Baltimore D., Science, 2003, 300, 763-; Porteus M.H. and Carroll D., Nat. Biotechnol., 2005, 23, 967-973), probably because of better specificity, it can still be harmful at very high doses (Gouble et al., J. Gene Med., 2006, 8, 616-622).

Most engineered endonucleases (ZFNs and HEs) so far are heterodimers, and include two separately engineered monomers, each binding one half of the target. Heterodimer formation is obtained by co-expression of the two monomers in the same cells (Porteus H.M., Mol. Ther., 2006, 13, 438-446; Smith et al., Nucleic acids Res.,2006, 34, e149; International PCT Applications WO 2007/097854 and WO 2007/049156). However, it is actually associated with the fonnation of two homodimers (Arnould et al., J. Mol. Biol., 2006, 355, 443-458; Bibikova et al., Genetics, 2002, 161, 1169-1175), recognizing different targets, and individual homodimers can sometimes result in an extremely high level of toxicity (Bibikova et al., Genetics, 2002, 161, 1169-1175). Thus, a limiting factor that still remains for the widespread use of the single-LAGLIDADG homing endonucleases such as I-*Cre*I, is the fact that the proteins can form homodimers in addition to engineered heterodimers (Arnould et al., J. Mol. Biol., 2006, 355, 443-458; International PCT Applications WO 2006/097853, WO 2006/097854, WO 2006/097784; Smith et al., Nucleic Acids Res., 2006, 34, e149), resulting in potential off_site cleavage.

This issue can be solved only by the suppression of functional homodimer formation, which could, in theory, be achieved by the fusion of the two monomers in a single chain molecule (Chevalier et al., Mol. Cell., 2002, 10, 895-905; Epinat et al., Nucleic Acids Res., 2005, 33, 5978-5990). However, this kind of design is relatively perilous, and can result in badly folded proteins (Epinat et al., Nucleic Acids Res., 2005, 33, 5978-5990). Impairing the functionnality of individual homodimers would be another solution.

The present invention discloses specific mutations that can enhance the activity of engineered meganucleases derived from the I-*Cre*I homodimeric meganucleases. In addition, one of these mutations, the G19S substitution, impairs the formation of functional homodimers. Engineered proteins derived from I-*Cre*I are generally heterodimers, containing two different monomers engineered separately. Such heterodimers are obtained by co-expression of the two different monomers in the targeted cells. Since these monomers can also homodimerize, there are actually three molecular species in the cells, the only useful one being the heterodimer, while the two other can result in additional off-site cleavage. Thus, the G19S mutation does not only improve protein activity, but also improve specificity.

Therefore, the invention relates to a method for enhancing the cleavage activity of an I-*Cre*I derived meganuclease (initial meganuclease), comprising the site-specific mutation of at least one amino acid residue which is selected in the group consisting of: the glycine at position 19 (G19), the phenylalanine at position 54 (F54), the phenylalanine at position 87 (F87), the serine at position 79 (S79), the valine at position 105 (V105) and the isoleucine at position 132 (I132) of I-*Cre*I*.*

### Definitions

- Amino acid refers to a natural or synthetic amino acid including enantiomers and stereoisomers of the preceding amino acids.

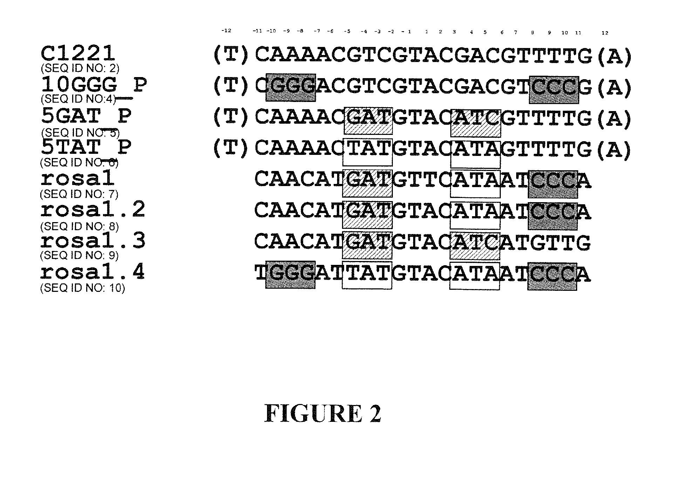

Amino acid residues in a polypeptide sequence are designated herein according to the one-letter code, in which, for example, Q means Gln or Glutamine residue, R means Arg or Arginine residue and D means Asp or Aspartic acid residue.
- Acidic amino acid refers to aspartic acid (D) and Glutamic acid (E).
- Basic amino acid refers to lysine (K), arginine (R) and histidine (H).
- Small amino acid refers to glycine (G) and alanine (A).
- Aromatic amino acid refers to phenylalanine (F), tryptophane (W) and tyrosine (Y).
- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.
- by "meganuclease", is intended an endonuclease having a double-stranded DNA target sequence of 12 to 45 bp. Said meganuclease is either a dimeric enzyme, wherein each domain is on a monomer or a monomeric enzyme comprising the two domains on a single polypeptide.
- by "I-*Cre*I" is intended the wild-type I-*Cre*I having the sequence SWISSPROT P05725, corresponding to the sequence SEQ ID NO: 1 in the sequence listing or pdb accession code 1g9y, corresponding to the sequence SEQ ID NO: 48 in the sequence listing
- by "meganuclease variant" or "variant" is intented a meganuclease obtained by replacement of at least one residue in the amino acid sequence of the wild-type meganuclease (natural meganuclease) with a different amino acid.
- by "functional variant" is intended a variant which is able to cleave a DNA target sequence, preferably said target is a new target which is not cleaved by the parent meganuclease. For example, such variants have amino acid variation at positions contacting the DNA target sequence or interacting directly or indirectly with said DNA target.
- by "meganuclease variant with novel specificity" is intended a variant having a pattern of cleaved targets different from that of the parent meganuclease. The terms "novel specificity", "modified specificity", "novel cleavage specificity", "novel substrate specificity" which are equivalent and used indifferently, refer to the specificity of the variant towards the nucleotides of the DNA target sequence.
- by "meganuclease domain" is intended the region which interacts with one half of the DNA target of a meganuclease and is able to associate with the other domain of the same meganuclease which interacts with the other half of the DNA target to form a functional meganuclease able to cleave said DNA target.
- by "domain" or "core domain" is intended the "LAGLIDADG homing endonuclease core domain" which is the characteristic α₁β₁β₂α₂β₃β₄α₃ fold of the homing endonucleases of the LAGLIDADG family, corresponding to a sequence of about one hundred amino acid residues. Said domain comprises four beta-strands (β₁β₂α₂β₃β₄) folded in an antiparallel beta-sheet which interacts with one half of the DNA target. This domain is able to associate with another LAGLIDADG homing endonuclease core domain which interacts with the other half of the DNA target to form a functional endonuclease able to cleave said DNA target. For example, in the case of the dimeric homing endonuclease I-*Cre*I (163 amino acids), the LAGLIDADG homing endonuclease core domain corresponds to the residues 6 to 94.
- by "single-chain meganuclease" is intended a meganuclease comprising two LAGLIDADG homing endonuclease domains or core domains linked by a peptidic spacer. The single-chain meganuclease is able to cleave a chimeric DNA target sequence comprising one different half of each parent meganuclease target sequence. The single-chain meganuclease is also named single-chain derivative, single-chain meganuclease, single-chain meganuclease derivative or chimeric meganuclease.
- by "I-*Cre*I derived meganuclease" is intended both a functional variant of I-*Cre*I and a single-chain meganuclease derived from said variant.
- by "subdomain" is intended the region of a LAGLIDADG homing endonuclease core domain which interacts with a distinct part of a homing endo-nuclease DNA target half-site. Two different subdomains behave independently and the mutation in one subdomain does not alter the binding and cleavage properties of the other subdomain. Therefore, two subdomains bind distinct part of a homing endonuclease DNA target half-site.
- by "beta-hairpin" is intended two consecutive beta-strands of the antiparallel beta-sheet of a LAGLIDADG homing endonuclease core domain ((β₁β₂ or,β₃β₄) which are connected by a loop or a turn,
- by "I-*Cre*I site" is intended a 22 to 24 bp double-stranded DNA sequence which is cleaved by I-*Cre*I. I-*Cre*I sites include the wild-type (natural) non-palindromic I-*Cre*I homing site and the derived palindromic sequences such as the sequence 5'- t₋₁₂c₋₁₁a₋₁₀a₋₉a₋₈a₋₇c₋₆g₋₅t₋₄c₋₃g₋₂t₋₁a₊₁c₊₂g₊₃a₊₄c₊₅g₊₆t₊₇t₊₈t₊₉t₊₁₀g₊₁₁a₊₁₂ also called C1221 (SEQ ID NO :2; figure 2).
- by "DNA target", "DNA target sequence", "target sequence" , "target-site", "target" , "site"; "site of interest"; "recognition site", "recognition sequence", "homing recognition site", "homing site", "cleavage site" is intended a 20 to 24 bp double-stranded palindromic, partially palindromic (pseudo-palindromic) or non-palindromic polynucleotide sequence that is recognized and cleaved by a LAGLIDADG homing endonuclease such as I-*Cre*I, or a variant, or a single-chain chimeric meganuclease derived from I-*Cre*I*.* These terms refer to a distinct DNA location, preferably a genomic location, at which a double stranded break (cleavage) is to be induced by the meganuclease. The DNA target is defined by the 5' to 3' sequence of one strand of the double-stranded polynucleotide, as indicated above for C1221. Cleavage of the DNA target occurs at the nucleotides at positions +2 and -2, respectively for the sense and the antisense strand. Unless otherwiwe indicated, the position at which cleavage of the DNA target by an I-*Cre* I meganuclease variant occurs, corresponds to the cleavage site on the sense strand of the DNA target.
- by "DNA target half-site", "half cleavage site" or half-site" is intended the portion of the DNA target which is bound by each LAGLIDADG homing endonuclease core domain.
- by "chimeric DNA target" or "hybrid DNA target" is intended the fusion of a different half of two parent meganuclease target sequences. In addition at least one half of said target may comprise the combination of nucleotides which are bound by at least two separate subdomains (combined DNA target).
- by "vector" is intended a nucleic acid molecule capable of transporting another nucleic acid to which it has been linked.
- by "homologous" is intended a sequence with enough identity to another one to lead to a homologous recombination between sequences, more particularly having at least 95 % identity, preferably 97 % identity and more preferably 99 %.
- "identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default settings.
- "individual" includes mammals, as well as other vertebrates (e.g., birds, fish and reptiles). The terms "mammal" and "mammalian", as used herein, refer to any vertebrate animal, including monotremes, marsupials and placental, that suckle their young and either give birth to living young (eutharian or placental mammals) or are egg-laying (metatharian or nonplacental mammals). Examples of mammalian species include humans and other primates (e.g., monkeys, chimpanzees), rodents (e.g., rats, mice, guinea pigs) and others such as for example: cows, pigs and horses.
- by "mutation" is intended the substitution, deletion, insertion of one or more nucleotides/amino acids in a polynucleotide (cDNA, gene) or a polypeptide sequence. Said mutation can affect the coding sequence of a gene or its regulatory sequence. It may also affect the structure of the genomic sequence or the structure/stability of the encoded mRNA.
- by "site-specific mutation" is intended the mutation of a specific nucleotide/codon in a nucleotidic sequence as opposed to random mutation.

The method according to the invention is performed according to standard site-directed mutagenesis methods which are well-known in the art and commercially available. It may be advantageously performed by amplifying overlapping fragments comprising the mutated position(s), as defined above, according to well-known overlapping PCR techniques.

The cleavage activity of the improved meganuclease obtainable by the method according to the invention may be measured by any well-known, *in vitro* or *in vivo* cleavage assay, such as those described in the International PCT Application WO 2004/067736; Epinat et al., Nucleic Acids Res., 2003, 31, 2952-2962; Chames et al., Nucleic Acids Res., 2005, 33, e178; Arnould et al., J. Mol. Biol., 2006, 355, 443-458; Arnould et al., J. Mol. Biol., Epub 10 May 2007.

For example, the cleavage activity of the improved meganuclease obtainable by the method according to the present invention may be measured by a direct repeat recombination assay, in yeast or mammalian cells, using a reporter vector, by comparison with that of the initial meganuclease. The reporter vector comprises two truncated, non-functional copies of a reporter gene (direct repeats) and the genomic DNA target sequence which is cleaved by the initial meganuclease, within the intervening sequence, cloned in a yeast or a mammalian expression vector. Expression of the meganuclease results in cleavage of the genomic DNA target sequence. This cleavage induces homologous recombination between the direct repeats, resulting in a functional reporter gene (LacZ, for example), whose expression can be monitored by appropriate assay. A stronger signal is observed with the improved meganuclease, as compared to the initial meganuclease.

Alternatively, the activity of the improved meganuclease towards its genomic DNA target can be compared to that of I-*Cre*I towards the I-*Cre*I site, at the same genomic locus, using a chromosomal assay in mammalian cells (Arnould et al., J. Mol. Biol., Epub 10 May 2007).

In a preferred embodiment of the method according to the invention:
- the glycine at position 19 is changed to serine (G19S) or alanine (G19A),
- the phenylalanine at position 54 is changed to leucine (F54L),
- the phenylalanine at position 87 is changed to leucine (F87L),
- the serine at position 79 is changed to glycine (S79G),
- the valine at position 105 is changed to alanine (V105A), and
- the isoleucine at position 132 is changed to valine (I132V).

In another embodiment of the method according to the invention, both I-*Cre*I monomers are mutated; the mutation(s) in each monomer may be identical or different.

For example the G19S or the F87L mutation is introduced in one monomer and the V105A or the I132V mutation is introduced in the other monomer.

In another preferred embodiment, at least two residues are mutated in the same monomer; the double mutant has a higher cleavage activity compared to each of the single mutants. For example, one monomer has both V105A and I132V mutations.

In another preferred embodiment of said method, said mutation further impairs the formation of a functional homodimer. More preferably, said mutation is the G19S mutation. The G19S mutation is advantageously introduced in one of the two monomers of a heterodimeric I-*Cre*I variant, so as to obtain a meganuclease having enhanced cleavage activity and enhanced cleavage specificity.

In addition, to enhance the cleavage specificity further, the other monomer may carry a distinct mutation that impairs the formation of a functional homodimer or favors the formation of the heterodimer.

The initial meganuclease may be derived from the wild-type I-*Cre*I (SEQ ID NO: 1 or 48) or an I-*Cre*I scaffold protein having at least 85 % identity, preferably at least 90 % identity, more preferably at least 95 % identity with SEQ ID NO: 48, such as the scaffold consisting of SEQ ID NO: 3 (167 amino acids) having the insertion of an alanine at position 2, the substitution D75N and the insertion of AAD at the C-terminus (positions 164 to 166) of the I-*Cre*I sequence.

The initial meganuclease may comprise one or more mutations at positions of amino acid residues which contact the DNA target sequence or interact with the DNA backbone or with the nucleotide bases, directly or via a water molecule; these residues are well-known in the art (Jurica et al., Molecular Cell., 1998, 2, 469-476; Chevalier et al., J. Mol. Biol., 2003, 329, 253-269). Preferably said mutations modify the cleavage specificity of the meganuclease and result in a meganuclease with novel specificity, which is able to cleave a DNA target from a gene of interest. More preferably, said mutations are substitutions of one or more amino acids in a first functional subdomain corresponding to that situated from positions 26 to 40 of I-*Cre*I amino acid sequence, that alter the specificity towards the nucleotide at positions ± 8 to 10 of the DNA target, and/or substitutions in a second functional subdomain corresponding to that situated from positions 44 to 77 of I-*Cre*I amino acid sequence, that alter the specificity towards the nucleotide at positions ± 3 to 5 of the DNA target, as described previously (International PCT Applications WO 2006/097784, WO 2006/097853 and WO 2007/049156; Arnould et al., J. Mol. Biol., 2006, 355, 443-458; Smith et al., Nucleic Acids Res., 2006, 34, e149). The substitutions correspond advantageously to positions 26, 28, 30, 32, 33, 38, and/or 40, 44, 68, 70, 75 and/or 77 of I-*Cre*I amino acid sequence. Said substitutions may be replacement of the initial amino acids with amino acids selected from the group consisting of: A, D, E, G, H, K, N, P, Q, R, S, T, Y, C, V, L and W. For cleaving a DNA target, wherein n₋₄ is t or n₊₄ is a, said variant has advantageously a glutamine (Q) at position 44; for cleaving a DNA target, wherein n₋₄ is a or n₊₄ is t, said variant has an alanine (A) or an asparagine at position 44, and for cleaving a DNA target, wherein n₋₉ is g or n₋₉ is c, said variant has advantageously an arginine (R) or a lysine (K) at position 38.

The initial meganuclease may be a homodimer which is able to cleave a palindromic or pseudo-palindromic DNA target sequence.

Alternatively, said initial meganuclease is a heterodimer, consisting of two monomers, each monomer comprising different mutations at positions 26 to 40 and/or 44 to 77 of I-*Cre*I, and said meganuclease being able to cleave a non-palindromic genomic DNA target sequence of interest.

The heterodimeric meganuclease is advantageously an obligate heterodimer variant having at least one pair of mutations interesting corresponding residues of the first and the second monomers which make an intermolecular interaction between the two **I**-*Cre*I monomers, wherein the first mutation of said pair(s) is in the first monomer and the second mutation of said pair(s) is in the second monomer and said pair(s) of mutations prevent the formation of functional homodimers from each monomer and allow the formation of a functional heterodimer, able to cleave a genomic DNA target of interest.

To form an obligate heterodimer, the monomers have advantageously at least one of the following pairs of mutations, respectively for the first and the second monomer:
a) the substitution of the glutamic acid at position 8 with a basic amino acid, preferably an arginine (first monomer) and the substitution of the lysine at position 7 with an acidic amino acid, preferably a glutamic acid (second monomer) ; the first monomer may further comprise the substitution of at least one of the lysine residues at positions 7 and 96, by an arginine.
b) the substitution of the glutamic acid at position 61 with a basic amino acid, preferably an arginine (first monomer) and the substitution of the lysine at position 96 with an acidic amino acid, preferably a glutamic acid (second monomer) ; the first monomer may further comprise the substitution of at least one of the lysine residues at positions 7 and 96, by an arginine
c) the substitution of the leucine at position 97 with an aromatic amino acid, preferably a phenylalanine (first monomer) and the substitution of the phenylalanine at position 54 with a small amino acid, preferably a glycine (second monomer) ; the first monomer may further comprise the substitution of the phenylalanine at position 54 by a tryptophane and the second monomer may further comprise the substitution of the leucine at position 58 or lysine at position 57, by a methionine, and
d) the substitution of the aspartic acid at position 137 with a basic amino acid, preferably an arginine (first monomer) and the substitution of the arginine at position 51 with an acidic amino acid, preferably a glutamic acid (second monomer).

For example, the first monomer may have the mutation D137R and the second monomer, the mutation R51D. Alternatively, the first monomer may have the mutations K7R, E8R, E61R, K96R and L97F or K7R, E8R, F54W, E61R, K96R and L97F and the second monomer, the mutations K7E, F54G, L58M and K96E or K7E, F54G, K57M and K96E.

Preferably, one monomer comprises the substitution of the lysine residue at position 7 by an acidic amino acid, preferably an aspartic acid (K7E) and the other monomer comprises the substitution of the glutamic acid residue at position 8 by a basic amino acid, preferably a lysine (E8K).

More preferably, one monomer comprises the G19S mutation and the K7E mutation and the other monomer comprises the E8K mutation or one monomer comprises the G19S mutation and the E8K mutation and the other monomer comprises the K7E mutation.

Other substitutions may also be introduced at positions contacting the phosphate backbone, for example in the final C-terminal loop (positions 137 to 143; Prieto et al., Nucleic Acids Res., Epub 22 April 2007). Preferably said residues are involved in binding and cleavage of said DNA cleavage site. More preferably, said residues are at positions 138, 139, 142 or 143 of I-*Cre*I*.* Two residues may be mutated in one variant provided that each mutation is in a different pair of residues chosen from the pair of residues at positions 138 and 139 and the pair of residues at positions 142 and 143. The mutations which are introduced modify the interaction(s) of said amino acid(s) of the final C-terminal loop with the phosphate backbone of the I-*Cre*I site. Preferably, the residue at position 138 or 139 is substituted by an hydrophobic amino acid to avoid the formation of hydrogen bonds with the phosphate backbone of the DNA cleavage site. For example, the residue at position 138 is substituted by an alanine or the residue at position 139 is substituted by a methionine. The residue at position 142 or 143 is advantageously substituted by a small amino acid, for example a glycine, to decrease the size of the side chains of these amino acid residues. More, preferably, said substitution in the final C-terminal loop modify the specificity of the variant towards the nucleotide at positions ± 1 to 2, ± 6 to 7 and/or ± 11 to 12 of the I-*Cre*I site.

Furthermore, other residues may be mutated on the entire sequence of the monomer(s). Example of mutations include the following mutations, by reference to I-*Cre*I amino acid sequence: I24V, R70S, the mutation of the aspartic acid at position 75, in an uncharged amino acid, preferably an asparagine (D75N) or a valine (D75V) and substitutions in the C-terminal half of the monomer sequence (positions 80 to 163 of I-*Cre*I);

In addition, one or more residues may be inserted at the NH₂ terminus and/or COOH terminus of the monomer(s). For example, a methionine residue is introduced at the NH₂ terminus, a tag (epitope HA-tag (YPYDVPDYA; SEQ ID NO: 49) or S-tag (KETAAAKFERQHMDS; SEQ ID NO: 50) or polyhistidine sequence) is introduced at the NH₂ terminus and/or COOH terminus; said tag is useful for the detection and/or the purification of the meganuclease. When the tag is introduced at the NH₂ terminus, the sequence of the tag may either replace the first amino acids of the variant (at least the first methionine and eventually the second amino acid of the variant; tag starting with a methionine) or be inserted between the first (methionine) and the second amino acids or the first and the third amino acids of the variant (tag with no methionine).

The variant may also comprise a nuclear localization signal (NLS); said NLS is useful for the importation of said variant into the cell nucleus. An example of NLS is KKKRK (SEQ ID NO: 51). The NLS may be inserted just after the first methionine of the variant or just after an N-terminal tag.

The invention relates also to an I-*Cre*I derived meganuclease (improved meganuclease) which is obtainable by the method as defined above, said meganuclease comprising at least a mutation selected from the group consisting of G19S, G19A, F54L, F87L, S79G, V105A and I132V, with the exclusion of the I-*Cre*I variants selected in the group consisting of:
- I-*Cre*I G19A, K28A, Y33S, Q38R, S40K, R70S, D75N
- I-*Cre*I G19A, K28A, Q38R, S40K, R70S, D75N, F87L
- I-*Cre*I G19A, K28A, Y33S, Q38R, S40K, D69G, R70S, D75N
- I-*Cre*I Y33R, S40Q, Q44A, R70H, D75N, F87L, I132T, V151A
- I-*Cre*I Y33R, S40Q, Q44A, R70H, D75N, F87L, F94L, V125A, E157G, K160R,
- I-*Cre*I Y33H, F54L, N86D, K100R, L104M, V105A, N136S, K159R
- I-*Cre*I S32T, Y33H, Q44K, R68Y, R70S, I77R, Q92R, K96R, K107R, I132V, T140A, T143A
- I-*Cre*I S32A, Y33H, Q44A, R68Y, R70S, D75Y, I77K, I132V
- I-*Cre*I N2I, S32G, Y33H, Q44A, R68Y, R70S, D75Y, I77K, K96R, V105A
- I-*Cre*I S32A, Y33H, F43L, Q44A, R68Y, R70S, D75Y, I77K, V105A, K159R
- I-*Cre*I G19S, N30Q, Y33G, Q38C, R68N, R70S, S72F, I77R
- I-*Cre*I Y33G, Q38C, R68N, R70S, I77R, F87L
- I-*Cre*I N30Q, Y33G, Q38C, F54L, R68N, R70S, I77R
- I-*Cre*I N30Q, Q31L, Y33G, Q38C, R68N, R70S, I77R, P83Q, F87L
- I-*Cre*I N30Q, Y33G, Q38C, R68N, R70S, I77R, V105A.

The invention encompasses I-*Cre*I derived meganucleases having at least 85 % identity, preferably at least 90 % identity, more preferably at least 95 % (96%, 97 %, 98 %, 99 %) identity with the sequences as defined above, said meganuclease having improved cleavage activity as compared to the initial meganuclease (I-Crel or I-*Cre*I variant as defined above).

The invention relates also to a method for making an I-*Cre*I derived heterodimeric meganuclease substantially free of at least one of the two homodimers resulting from the association of each monomer of said heterodimeric meganuclease, comprising the co-expression of the two monomers of an I-*Cre*I derived heterodimeric meganuclease in a cell, wherein one of the two monomers comprises comprises the G 19S mutation.

According to an advantageous embodiment of said method, the other monomer carries another mutation that impairs the formation of a functional homodimer or favors the formation of the heterodimer, so as to produce a heterodimeric meganuclease substantially free of homodimers. The I-*Cre*I derived heterodimeric meganuclease which is produced by said method is more specific since at least one of the two homodimers resulting from the association of the two monomers is not functional. In addition said meganuclease has enhanced cleavage activity due to the presence of the G19 mutation, as mentioned above.

The invention relates also to an I-*Cre*I derived heterodimeric meganuclease substantially free of at least one of the two homodimers resulting from the association of each monomer of said heterodimeric meganuclease, which is obtainable by the method as defined above.

The subject-matter of the present invention is also a single-chain chimeric meganuclease (fusion protein) derived from a meganuclease as defined above. The single-chain meganuclease may comprise two I-*Cre*I monomers, two I-*Cre*I core domains (positions 6 to 94 of I-*Cre*I) or a combination of both. Preferably, the two monomers /core domains or the combination of both, are connected by a peptidic linker. Examples of peptidic linkers are SEQ ID NO: 52 and 68.

The meganuclease of the invention includes the improved meganuclease, the heterodimeric meganuclease and the single-chain chimeric derivative, as defined above. The meganuclease of the invention may comprise at least one NLS and/or one tag as defined above; said NLS and/or tag may be in the first and/or the second monomer.

The subject-matter of the present invention is also a polynucleotide fragment encoding a meganuclease as defined above; said polynucleotide may encode one monomer of a homodimeric or heterodimeric variant, or two domains/monomers of a single-chain derivative.

The subject-matter of the present invention is also a recombinant vector for the expression of a meganuclease according to the invention. The recombinant vector comprises at least one polynucleotide fragment encoding a variant or a single-chain meganuclease, as defined above.

In a preferred embodiment, said vector comprises two different polynucleotide fragments, each encoding one of the monomers of a heterodimeric variant.

A vector which can be used in the present invention includes, but is not limited to, a viral vector, a plasmid, a RNA vector or a linear or circular DNA or RNA molecule which may consists of a chromosomal, non chromosomal, semisynthetic or synthetic nucleic acids. Preferred vectors are those capable of autonomous replication (episomal vector) and/or expression of nucleic acids to which they are linked (expression vectors). Large numbers of suitable vectors are known to those of skill in the art and commercially available.

Viral vectors include retrovirus, adenovirus, parvovirus (e. g. adeno-associated viruses), coronavirus, negative strand RNA viruses such as orthomyxovirus (e. g., influenza virus), rhabdovirus (e. g., rabies and vesicular stomatitis virus), para-myxovirus (e. g. measles and Sendai), positive strand RNA viruses such as picornavirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example. Examples of retroviruses include: avian leukosissarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996).

Preferred vectors include lentiviral vectors, and particularly self inactivacting lentiviral vectors.

Vectors can comprise selectable markers, for example: neomycin phosphotransferase, histidinol dehydrogenase, dihydrofolate reductase, hygromycin phosphotransferase, herpes simplex virus thymidine kinase, adenosine deaminase, glutamine synthetase, and hypoxanthine-guanine phosphoribosyl transferase for eukaryotic cell culture; TRP1 for *S. cerevisiae*; tetracycline, rifampicin or ampicillin resistance in *E. coli.*

Preferably said vectors are expression vectors, wherein the sequence(s) encoding the variant/single-chain derivative of the invention is placed under control of appropriate transcriptional and translational control elements to permit production or synthesis of said meganuclease. Therefore, said polynucleotide is comprised in an expression cassette. More particularly, the vector comprises a replication origin, a promoter operatively linked to said encoding polynucleotide, a ribosome-binding site, an RNA-splicing site (when genomic DNA is used), a polyadenylation site and a transcription termination site. It also can comprise an enhancer. Selection of the promoter will depend upon the cell in which the polypeptide is expressed. Preferably, when said variant is a heterodimer, the two polynucleotides encoding each of the monomers are included in one vector which is able to drive the expression of both polynucleotides, simultaneously. Suitable promoters include tissue specific and/or inducible promoters. Examples of inducible promoters are: eukaryotic metallothionine promoter which is induced by increased levels of heavy metals, prokaryotic lacZ promoter which is induced in response to isopropyl-β-D-thiogalactopyranoside (IPTG) and eukaryotic heat shock promoter which is induced by increased temperature. Examples of tissue specific promoters are skeletal muscle creatine kinase, prostate-specific antigen (PSA), α-antitrypsin protease, human surfactant (SP) A and B proteins, β-casein and acidic whey protein genes.

According to another advantageous embodiment of said vector, it includes a targeting DNA construct comprising sequences sharing homologies with the region surrounding the genomic DNA target cleavage site as defined above.

Alternatively, the vector coding for the meganuclease and the vector comprising the targeting DNA construct are different vectors.

More preferably, the targeting DNA construct comprises:
a) sequences sharing homologies with the region surrounding the genomic DNA cleavage site as defined above, and
b) a sequence to be introduced flanked by sequences as in a).

Preferably, homologous sequences of at least 50 bp, preferably more than 100 bp and more preferably more than 200 bp are used. Indeed, shared DNA homologies are located in regions flanking upstream and downstream the site of the break and the DNA sequence to be introduced should be located between the two arms. The sequence to be introduced is preferably a sequence which repairs a mutation in the gene of interest (gene correction or recovery of a functional gene), for the purpose of genome therapy. Alternatively, it can be any other sequence used to alter the chromosomal DNA in some specific way including a sequence used to modify a specific sequence, to attenuate or activate the endogenous gene of interest, to inactivate or delete the endogenous gene of interest or part thereof, to introduce a mutation into a site of interest or to introduce an exogenous gene or part thereof.

The invention also concerns a prokaryotic or eukaryotic host cell which is modified by a polynucleotide or a vector as defined above, preferably an expression vector.

The invention also concerns a non-human transgenic animal or a transgenic plant, characterized in that all or parts of their cells are modified by a polynucleotide or a vector as defined above.

As used herein, a cell refers to a prokaryotic cell, such as a bacterial cell, or eukaryotic cell, such as an animal, plant or yeast cell.

The subject-matter of the present invention is further the use of an improved meganuclease having a mutation at position 19 obtainable by the method as defined above, one or two derived polynucleotide(s), preferably included in expression vector(s), a cell, a transgenic plant, a non-human transgenic mammal, as defined above, for molecular biology, for *in vivo* or *in vitro* genetic engineering, and for *in vivo* or *in vitro* genome engineering for non-therapeutic purposes, in a locus which is different from that of the human beta-2-microglobulin gene and the human XPC gene.

The subject-matter of the present invention is further the use of an improved meganuclease having a mutation at position 54 or 105 obtainable by the method as defined above, one or two derived polynucleotide(s), preferably included in expression vector(s), a cell, a transgenic plant, a non-human transgenic mammal, as defined above, for molecular biology, for *in vivo* or *in vitro* genetic engineering, and for *in vivo* or *in vitro* genome engineering for non-therapeutic purposes, in a locus which is different from that of the Chinese Hamster Hypoxanthine Phosphoribosyltransferase gene and the human beta-2-microglobulin gene.

The subject-matter of the present invention is further the use of an improved meganuclease having a mutation at position 87 obtainable by the method as defined above, one or two derived polynucleotide(s), preferably included in expression vector(s), a cell, a transgenic plant, a non-human transgenic mammal, as defined above, for molecular biology, for *in vivo* or *in vitro* genetic engineering, and for *in vivo* or *in vitro* genome engineering for non-therapeutic purposes, in a locus which is different from that of the human beta-2-microglobulin gene, the human RAG2 gene and the human XPC gene.

The subject-matter of the present invention is further the use of an improved meganuclease having a mutation at position 132 obtainable by the method as defined above, one or two derived polynucleotide(s), preferably included in expression vector(s), a cell, a transgenic plant, a non-human transgenic mammal, as defined above, for molecular biology, for *in vivo* or *in vitro* genetic engineering, and for *in vivo* or *in vitro* genome engineering for non-therapeutic purposes, in a locus which is different from that of the Chinese Hamster Hypoxanthine Phosphoribosyltransferase gene, the human RAG2 gene and the human beta-2-mieroglobulin gene.

The subject-matter of the present invention is further the use of an improved meganuclease having a mutation at position 79 obtainable by the method as defined above, one or two derived polynucleotide(s), preferably included in expression vector(s), a cell, a transgenic plant, a non-human transgenic mammal, as defined above, for molecular biology, for *in vivo* or *in vitro* genetic engineering, and for *in vivo* or *in vitro* genome engineering, for non-therapeutic purposes.

The subject-matter of the present invention is further the use of a heterodimeric meganuclease having the G19S mutation, obtainable by the method as defined above, one or two derived polynucleotide(s), preferably included in expression vector(s), a cell, a transgenic plant, a non-human transgenic mammal, as defined above, for molecular biology, for *in vivo* or *in vitro* genetic engineering, and for *in vivo* or *in vitro* genome engineering, for non-therapeutic purposes.

Non therapeutic purposes include for example (i) gene targeting of specific loci in cell packaging lines for protein production, (ii) gene targeting of specific loci in crop plants, for strain improvements and metabolic engineering, (iii) targeted recombination for the removal of markers in genetically modified crop plants, (iv) targeted recombination for the removal of markers in genetically modified microorganism strains (for antibiotic production for example).

According to an advantageous embodiment of said use, it is for inducing a double-strand break in a site of interest comprising a DNA target sequence, thereby inducing a DNA recombination event, a DNA loss or cell death.

According to the invention, said double-strand break is for: repairing a specific sequence, modifying a specific sequence, restoring a functional gene in place of a mutated one, attenuating or activating an endogenous gene of interest, introducing a mutation into a site of interest, introducing an exogenous gene or a part thereof, inactivating or detecting an endogenous gene or a part thereof, translocating a chromosomal arm, or leaving the DNA unrepaired and degraded.

The subject-matter of the present invention is also a method of genetic engineering, characterized in that it comprises a step of double-strand nucleic acid breaking in a site of interest located on a vector comprising a DNA target as defined hereabove, by contacting said vector with a meganuclease as defined above, thereby inducing an homologous recombination with another vector presenting homology with the sequence surrounding the cleavage site of said meganuclease.

The subjet-matter of the present invention is also a method of genome engineering, characterized in that it comprises the following steps: 1) double-strand breaking a genomic locus comprising at least one DNA target of a meganuclease as defined above, by contacting said target with said meganuclease; 2) maintaining said broken genomic locus under conditions appropriate for homologous recombination with a targeting DNA construct comprising the sequence to be introduced in said locus, flanked by sequences sharing homologies with the targeted locus.

The subject-matter of the present invention is also a method of genome engineering, characterized in that it comprises the following steps: 1) double-strand breaking a genomic locus comprising at least one DNA target of a meganuclease as defined above, by contacting said cleavage site with said meganuclease; 2) maintaining said broken genomic locus under conditions appropriate for homologous recombination with chromosomal DNA sharing homologies to regions surrounding the cleavage site.

The subject-matter of the present invention is also the use of at least one meganuclease as defined above, or one or two derived polynucleotide(s), preferably included in expression vector(s), as defined above, for the preparation of a medicament for preventing, improving or curing a genetic disease in an individual in need thereof, said medicament being administrated by any means to said individual.

The subject-matter of the present invention is also a method for preventing, improving or curing a genetic disease in an individual in need thereof, said method comprising the step of administering to said individual a composition comprising at least a meganuclease as defined above, by any means.

In this case, the use of the meganuclease as defined above, comprises at least the step of (a) inducing in somatic tissue(s) of the individual a double stranded cleavage at a site of interest of a gene comprising at least one recognition and cleavage site of said meganuclease, and (b) introducing into the individual a targeting DNA, wherein said targeting DNA comprises (1) DNA sharing homologies to the region surrounding the cleavage site and (2) DNA which repairs the site of interest upon recombination between the targeting DNA and the chromosomal DNA. The targeting DNA is introduced into the individual under conditions appropriate for introduction of the targeting DNA into the site of interest.

According to the present invention, said double-stranded cleavage is induced, either *in toto* by administration of said meganuclease to an individual, or *ex vivo* by introduction of said meganuclease into somatic cells removed from an individual and returned into the individual after modification.

In a preferred embodiment of said use, the meganuclease is combined with a targeting DNA construct comprising a sequence which repairs a mutation in the gene flanked by sequences sharing homologies with the regions of the gene surrounding the genomic DNA cleavage site of said meganuclease, as defined above. The sequence which repairs the mutation is either a fragment of the gene with the correct sequence or an exon knock-in construct.

For correcting a gene, cleavage of the gene occurs in the vicinity of the mutation, preferably, within 500 bp of the mutation. The targeting construct comprises a gene fragment which has at least 200 bp of homologous sequence flanking the genomic DNA cleavage site (minimal repair matrix) for repairing the cleavage, and includes the correct sequence of the gene for repairing the mutation. Consequently, the targeting construct for gene correction comprises or consists of the minimal repair matrix; it is preferably from 200 pb to 6000 pb, more preferably from 1000 pb to 2000 pb.

For restoring a functional gene, cleavage of the gene occurs upstream of a mutation. Preferably said mutation is the first known mutation in the sequence of the gene, so that all the downstream mutations of the gene can be corrected simultaneously. The targeting construct comprises the exons downstream of the genomic DNA cleavage site fused in frame (as in the cDNA) and with a polyadenylation site to stop transcription in 3'. The sequence to be introduced (exon knock-in construct) is flanked by introns or exons sequences surrounding the cleavage site, so as to allow the transcription of the engineered gene (exon knock-in gene) into a mRNA able to code for a functional protein. For example, the exon knock-in construct is flanked by sequences upstream and downstream.

The subject-matter of the present invention is also the use of at least one meganuclease as defined above, or one or two derived polynucleotide(s), preferably included in expression vector(s), as defined above for the preparation of a medicament for preventing, improving or curing a disease caused by an infectious agent that presents a DNA intermediate, in an individual in need thereof, said medicament being administrated by any means to said individual.

The subject-matter of the present invention is also a method for preventing, improving or curing a disease caused by an infectious agent that presents a DNA intermediate, in an individual in need thereof, said method comprising at least the step of administering to said individual a composition as defined above, by any means.

The subject-matter of the present invention is also the use of at least one meganuclease as defined above, or one or two polynucleotide(s), preferably included in expression vector(s), as defined above, *in vitro,* for inhibiting the propagation, inactivating or deleting an infectious agent that presents a DNA intermediate, in biological derived products or products intended for biological uses or for disinfecting an object.

The subject-matter of the present invention is also a composition characterized in that it comprises at least one meganuclease, or one or two derived polynucleotide(s), preferably included in expression vector(s), as defined above.

In a preferred embodiment of said composition, it comprises a targeting DNA construct comprising the sequence which repairs the site of interest flanked by sequences sharing homologies with the targeted locus as defined above. Preferably, said targeting DNA construct is either included in a recombinant vector or it is included in an expression vector comprising the polynucleotide(s) encoding the meganuclease, as defined in the present invention.

The subject-matter of the present invention is also a method for decontaminating a product or a material from an infectious agent that presents a DNA intermediate, said method comprising at least the step of contacting a biological derived product, a product intended for biological use or an object, with a composition as defined above, for a time sufficient to inhibit the propagation, inactivate or delete said infectious agent.

In a particular embodiment, said infectious agent is a virus. For example said virus is an adenovirus (Ad11, Ad21), herpesvirus (HSV, VZV, EBV, CMV, herpesvirus 6, 7 or 8), hepadnavirus (HBV), papovavirus (HPV), poxvirus or retrovirus (HTLV, HIV).

The subject-matter of the present invention is also products containing at least a meganuclease, or one or two expression vector(s) encoding said meganuclease, and a vector including a targeting construct, as defined above, as a combined preparation for simultaneous, separate or sequential use in the prevention or the treatment of a genetic disease.

For purposes of therapy, the meganuclease and a pharmaceutically acceptable excipient are administered in a therapeutically effective amount. Such a combination is said to be administered in a "therapeutically effective amount" if the amount administered is physiologically significant. An agent is physiologically significant if its presence results in a detectable change in the physiology of the recipient. In the present context, an agent is physiologically significant if its presence results in a decrease in the severity of one or more symptoms of the targeted disease and in a genome correction of the lesion or abnormality.

In one embodiment of the uses according to the present invention, the meganuclease is substantially non-immunogenic, i.e., engenders little or no adverse immunological response. A variety of methods for ameliorating or eliminating deleterious immunological reactions of this sort can be used in accordance with the invention.

In a preferred embodiment, the meganuclease is substantially free of N-formyl methionine.

Another way to avoid unwanted immunological reactions is to conjugate meganucleases to polyethylene glycol ("PEG") or polypropylene glycol ("PPG") (preferably of 500 to 20,000 daltons average molecular weight (MW)). Conjugation with PEG or PPG, as described by Davis et al. (US 4,179,337) for example, can provide non-immunogenic, physiologically active, water soluble endonuclease conjugates with anti-viral activity. Similar methods also using a polyethylene-polypropylene glycol copolymer are described in Saifer et al. (US 5,006,333).

The meganuclease can be used either as a polypeptide or as a polynucleotide construct/vector encoding said polypeptide. It is introduced into cells, *in vitro, ex vivo* or *in vivo,* by any convenient means well-known to those in the art, which are appropriate for the particular cell type, alone or in association with either at least an appropriate vehicle or carrier and/or with the targeting DNA. Once in a cell, the meganuclease and if present, the vector comprising targeting DNA and/or nucleic acid encoding a meganuclease are imported or translocated by the cell from the cytoplasm to the site of action in the nucleus.

The meganuclease (polypeptide) may be advantageously associated with: liposomes, polyethyleneimine (PEI), and/or membrane translocating peptides (Bonetta, The Scientist, 2002, 16, 38; Ford *et al.,* Gene Ther., 2001, 8, 1-4 ; Wadia and Dowdy, Curr. Opin. Biotechnol., 2002, 13, 52-56); in the latter case, the sequence of the meganuclease fused with the sequence of a membrane translocating peptide (fusion protein).

Vectors comprising targeting DNA and/or nucleic acid encoding a meganuclease can be introduced into a cell by a variety of methods (e.g., injection, direct uptake, projectile bombardment, liposomes, electroporation). Meganucleases can be stably or transiently expressed into cells using expression vectors. Techniques of expression in eukaryotic cells are well known to those in the art. (See Current Protocols in Human Genetics: Chapter 12 "Vectors For Gene Therapy" & Chapter 13 "Delivery Systems for Gene Therapy"). Optionally, it may be preferable to incorporate a nuclear localization signal into the recombinant protein to be sure that it is expressed within the nucleus.

The I-*Cre*I derived meganuclease (initial meganuclease) may be obtained by a method for engineering variants able to cleave a genomic DNA target sequence of interest, as described previously in Smith et al., Nucleic Acids Res., 2006, 34, e149, said method comprising at least the steps of:
(a) constructing a first series of I-*Cre*I variants having at least one substitution in a first functional subdomain of the LAGLIDADG core domain situated from positions 26 to 40 of I-*Cre*I,
(b) constructing a second series of I-*Cre*I variants having at least one substitution in a second functional subdomain of the LAGLIDADG core domain situated from positions 44 to 77 of I-*Cre*I,
(c) selecting and/or screening the variants from the first series of step (a) which are able to cleave a mutant I-*Cre*I site wherein (i) the nucleotide triplet at positions -10 to -8 of the I-*Cre*I site has been replaced with the nucleotide triplet which is present at positions -10 to -8 of said genomic target and (ii) the nucleotide triplet at positions +8 to +10 has been replaced with the reverse complementary sequence of the nucleotide triplet which is present at positions -10 to -8 of said genomic target,
(d) selecting and/or screening the variants from the second series of step (b) which are able to cleave a mutant I-*Cre*I site wherein (i) the nucleotide triplet at positions -5 to -3 of the I-*Cre*I site has been replaced with the nucleotide triplet which is present at positions -5 to -3 of said genomic target and (ii) the nucleotide triplet at positions +3 to +5 has been replaced with the reverse complementary sequence of the nucleotide triplet which is present at positions -5 to -3 of said genomic target,
(e) selecting and/or screening the variants from the first series of step (a) which are able to cleave a mutant I-*Cre*I site wherein (i) the nucleotide triplet at positions +8 to +10 of the I-*Cre*I site has been replaced with the nucleotide triplet which is present at positions +8 to +10 of said genomic target and (ii) the nucleotide triplet at positions -10 to -8 has been replaced with the reverse complementary sequence of the nucleotide triplet which is present at positions +8 to +10 of said genomic target,
(f) selecting and/or screening the variants from the second series of step (b) which are able to cleave a mutant I-*Cre*I site wherein (i) the nucleotide triplet at positions +3 to +5 of the I-*Cre*I site has been replaced with the nucleotide triplet which is present at positions +3 to +5 of said genomic target and (ii) the nucleotide triplet at positions -5 to -3 has been replaced with the reverse complementary sequence of the nucleotide triplet which is present at positions +3 to +5 of said genomic target,
(g) combining in a single variant, the mutation(s) at positions 26 to 40 and 44 to 77 of two variants from step (c) and step (d), to obtain a novel homodimeric I-*Cre*I variant which cleaves a sequence wherein (i) the nucleotide triplet at positions -10 to -8 is identical to the nucleotide triplet which is present at positions -10 to -8 of said genomic target, (ii) the nucleotide triplet at positions +8 to +10 is identical to the reverse complementary sequence of the nucleotide triplet which is present at positions -10 to -8 of said genomic target, (iii) the nucleotide triplet at positions -5 to -3 is identical to the nucleotide triplet which is present at positions -5 to -3 of said genomic target and (iv) the nucleotide triplet at positions +3 to +5 is identical to the reverse complementary sequence of the nucleotide triplet which is present at positions -5 to -3 of said genomic target,
(h) combining in a single variant, the mutation(s) at positions 26 to 40 and 44 to 77 of two variants from step (e) and step (f), to obtain a novel homodimeric I-*Cre*I variant which cleaves a sequence wherein (i) the nucleotide triplet at positions +3 to +5 is identical to the nucleotide triplet which is present at positions +3 to +5 of said genomic target, (ii) the nucleotide triplet at positions -5 to -3 is identical to the reverse complementary sequence of the nucleotide triplet which is present at positions +3 to +5 of said genomic target, (iii) the nucleotide triplet at positions +8 to +10 of the I-*Cre*I site has been replaced with the nucleotide triplet which is present at positions +8 to +10 of said genomic target and (iv) the nucleotide triplet at positions -10 to -8 is identical to the reverse complementary sequence of the nucleotide triplet at positions +8 to +10 of said genomic target,
(i) combining the variants obtained in steps (g) and (h) to form heterodimers, and
(j) selecting and/or screening the heterodimers from step (i) which are able to cleave said genomic DNA target.

Steps (a) and (b) may comprise the introduction of additional mutations in order to improve the binding and/or cleavage properties of the mutants, particularly at other positions contacting the DNA target sequence or interacting directly or indirectly with said DNA target. These steps may be performed by generating combinatorial libraries as described in the International PCT Application WO 2004/067736 and Arnould et al. (J. Mol. Biol., 2006, 355, 443-458).

The selection and/or screening in steps (c), (d), (c), (f) and/or (j) may be performed by using a cleavage assay *in vitro* or *in vivo,* as described in the International PCT Application WO 2004/067736, Epinat et al. (Nucleic Acids Res., 2003, 31, 2952-2962), Chames et al. (Nucleic Acids Res., 2005, 33, e178), and Arnould et al. (J. Mol. Biol., 2006, 355, 443-458).

Preferably, steps (c), (d), (e), (f) and/or (j) are performed *in vivo,* under conditions where the double-strand break in the mutated DNA target sequence which is generated by said variant leads to the activation of a positive selection marker or a reporter gene, or the inactivation of a negative selection marker or a reporter gene, by recombination-mediated repair of said DNA double-strand break, as described in the International PCT Application WO 2004/067736, Epinat et al. (Nucleic Acids Res., 2003, 31, 2952-2962), Chames et al. (Nucleic Acids Res., 2005, 33, e178), and Arnould et al. (J. Mol. Biol., 2006, 355, 443-458).

The (intramolecular) combination of mutations in steps (g) and (h) may be performed by amplifying overlapping fragments comprising each of the two subdomains, according to well-known overlapping PCR techniques.

The mutation(s) at positions 19, 54, 79, 105 and /or 132 as defined above, are introduced by directed mutagenesis on the combined variants of step (g) or step (h).

The (intermolecular) combination of the variants in step (i) is performed by co-expressing one variant from step (g) with one variant from step (h), so as to allow the formation of heterodimers. For example, host cells may be modified by one or two recombinant expression vector(s) encoding said variant(s). The cells are then cultured under conditions allowing the expression of the variant(s), so that heterodimers are formed in the host cells, as described previously in the International PCT Application WO 2006/097854 and Arnould et al. (J. Mol. Biol., 2006, 355, 443-458).

Alternatively, the heterodimeric meganuclease of the invention may be obtained by a method derived from the hereabove method of engineering meganuclease variants, by introducing the following modifications:
- step (a) and step (b) are performed on two types of initial scaffold proteins: a first I-*Cre*I scaffold having the G19S mutation (monomer A) and a second I-*Cre*I scaffold (monomer B) not having the mutation G19S; said second scaffold may have another mutation that impairs the formation of a functional homodimer as defined above, and
- the selection/screening of steps (c) to (f) is performed by transforming the library of variants of monomer A or B as defined above in a host cell that expresses a I-*Cre*I mutant having the corresponding mutations (from monomer B or A, respectively) to allow the formation of heterodimers and selecting the functional heterodimeric variants by using a non-palindromic DNA target wherein one half of the I-*Cre*I site is modified at positions ± 3 to 5 or ± 8 to 10 and the other half is not modified.

The steps (g) and (h) are performed by combining in a single variant, the mutations of two variants derived from the same monomer (A) or (B).

Step (i) is performed by combining the variants derived from one of the monomers (A or B), obtained in step (g) with the variants derived from the other monomer, obtained in step (h) to form heterodimers.

The uses of the meganuclease and the methods of using said meganucleases according to the present invention include also the use of the polynucleotide(s), vector(s), cell, transgenic plant or non-human transgenic mammal encoding said meganuclease, as defined above.

According to another advantageous embodiment of the uses and methods according to the present invention, said meganuclease, polynucleotide(s), vector(s), cell, transgenic plant or non-human transgenic mammal are associated with a targeting DNA construct as defined above. Preferably, said vector encoding the monomer(s) of the meganuclease, comprises the targeting DNA construct, as defined above.

The polynucleotide sequence(s) encoding the two monomers of the I-*Cre*I derived meganuclease as defined in the present invention may be prepared by any method known by the man skilled in the art. For example, they are amplified from a cDNA template, by polymerase chain reaction with specific primers. Preferably the codons of said cDNA are chosen to favour the expression of said protein in the desired expression system.

I-*Cre*I derived single-chain meganucleases able to cleave a DNA target from a genomic sequence of interest are prepared by methods well-known in the art (Epinat et al., Nucleic Acids Res., 2003, 31, 2952-62; Chevalier et al., Mol. Cell., 2002, 10, 895-905; Steuer et al., Chembiochem., 2004, 5, 206-13; International PCT Applications WO 03/078619 and WO 2004/031346). Any of such methods, may be applied for constructing the single-chain meganuclease as defined in the invention.

The recombinant vector comprising said polynucleotides may be obtained and introduced in a host cell by the well-known recombinant DNA and genetic engineering techniques.

The I-*Cre*I derived meganucleases as defined in the invention are produced by co-expressing two I-*Cre*I monomers as defined above, in a host cell or a transgenic animal/plant modified modified by one or two expression vector(s), under conditions suitable for the co-expression of the monomers, and the heterodimeric meganuclease is recovered from the host cell culture or from the transgenic animal/plant, by any appropriate means.

The single-chain meganuclease as defined in the invention is produced by expressing a fusion protein comprising the two monomers as defined above, in a host cell or a transgenic animal/plant modified by one expression vector, under conditions suitable for the expression of said fusion protein, and the single-chain meganuclease is recovered from the host cell culture or from the transgenic animal/plant, by any appropriate means.

The subject-matter of the present invention is also the use of at least one I-*Cre*I derived meganuclease obtainable by the method as defined above, as a scaffold for making other meganucleases. For example another round of mutagenesis and selection/screening can be performed on the monomers, for the purpose of making a novel generation of homing endonucleases.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986).

In addition to the preceding features, the invention further comprises other features which will emerge from the description which follows, which refers to examples illustrating the method for enhancing the cleavage activity of I-*Cre*I derived meganuclease according to the invention, as well as to the appended drawings in which:
- figure 1 represents the structure of homing endonucleases from the LAGLIDADG family and combinatorial approach for engineering them.
   A. Tridimensional structure of the I-*Cre*I homing endonuclease bound to its DNA target. The catalytic core is surrounded by two αββαββα folds forming a saddle-shaped interaction interface above the DNA major groove.
   B. A two-step approach to engineer the specificity of I-*Cre*I and other LAGLIDADG homing endonucleases. A large collection of novel endonucleases is generated by semi-rational mutagenesis of an initial scaffold and screening for functionalk variants variants with with locally altered specificity. Then, a combinatorial approach is used to assemble these mutants into into meganucleases with fully redesigned specificity. Homodimeric proteins ("half-meganucleases") are created by combination of two sets of mutations within a same αββαββα fold, and the co-expression of two such "half-meganuclease" can result in a heterodimeric species ("custom meganucleases") cleaving the target of interest.
- figure 2 represents the Rosal target sequence and derivatives. 10GGG_P, 5GAT_P and 5TAT_P are close derivatives found to be cleaved by previously obtained I-*Cre*I mutants. They differ from C1221 (palindromic sequence cleaved by the I-*Cre*I scaffold protein) by the boxed motives. C1221, 10GGG_P, 5GAT_P and 5TAT_P were first described as 24 bp sequences, but structural data suggest that only the 22 bp are relevant for protein/DNA interaction. However, positions ±12 are indicated in parenthesis, rosal is the DNA sequence located in the mouse ROSA26 locus at position 8304. In the rosa1.2 target, the GTTC sequence in the middle of the target is replaced with GTAC, the bases found in C1221. rosa1.3 is the palindromic sequence derived from the left part of rosa1.2, and rosa1.4 is the palindromic sequence derived from the right part of rosa1.2. As shown in the figure, the boxed motives from 10GGG_P, 5GAT_P and 5TAT_P are found in the rosa1 series of targets.
- figure 3 represents the map of pCLS1055, a plasmid for gateway cloning of DNA targets in yeast reporter vector.
- figure 4 represents the map of pCLS0542, a *LEU2* marked plasmid for meganuclease ORF cloning and expression in yeast.
- figure 5 illustrates the cleavage of rosa1.3 DNA target by I-*Cre*I mutants. The 63 positives found in primary screen were rearranged in one 96-well plate and validated by a secondary screen (in a quadraplicate format). The 22 mutants chosen in example 1 are circled.
- figure 6 illustrates the cleavage of rosa1.4 target by I-*Cre*I combinatorial mutants. The 69 positives found in primary screen were rearranged in one 96-well plate and validated by a secondary screen (in a quadriplicate format). The 15 mutants chosen in example 2 are circled.
- figure 7 represents the map of pCLS1107, a Kan^{R} marked plasmid for meganuclease ORF cloning and expression in yeast.
- figure 8 illustrates the cleavage of rosa1.2 and rosa1 targets by heterodimeric I-*Cre*I combinatorial mutants. **A.** Example of screening of combinations of I-*Cre*I mutants with the rosa1.2 target. **B.** Screening of the same combinations of I-*Cre*I mutants with the rosa1 target. B5, B6, D5, D6, F5, F6, H5 and H6: yeast strains expressing rosa1.3 cutting I-*Cre*I mutants transformed with pCLS1107 empty plasmid DNA.

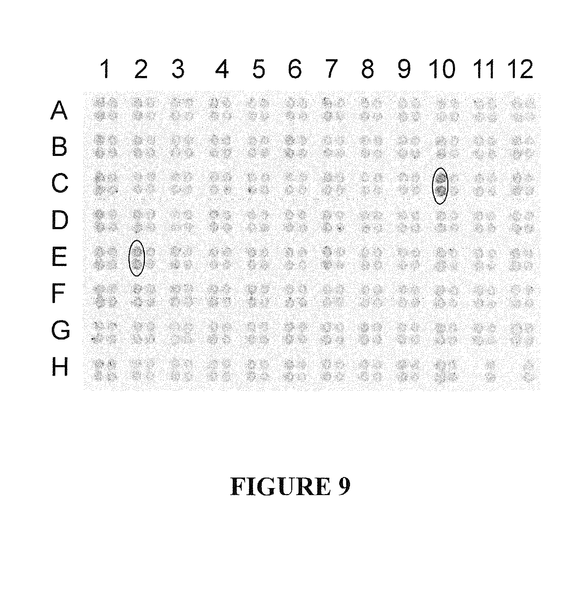

- figure 9 illustrates the cleavage of the rosa1 target. A series of I-*Cre*I mutants cutting rosa1.4 were randomly mutagenized and co-expressed with a mutant cutting rosa1.3. Cleavage is tested with the rosa1 target. In each four dots cluster, the two dots on the right correspond to one of the original heterodimers cleaving rosa1 in duplicate, whereas the tow left dots correspond to a same mutated rosa1.4 cleaver co-expressed with a non mutated rosa1.3 cleaver (mutant m14, described in Tables III and IV). The two optimized mutants displaying improved cleavage of rosa1 are circled, and correspond to co-expression of mutants m13 and MO_1 (C10) or of m13 and MO_2 (E2). MO_1 and MO_2 are further described in Table V.
- figure 10 illustrates the cleavage of the rosa1 target. A series of I-*Cre*I mutants cutting rosa1.3 were randomly mutagenized and co-expressed with a refined mutant cutting rosa1.4. Cleavage is tested with the rosa1 target. Mutants displaying efficient cleavage of rosa1 are circled. In the filter:
   - B11 corresponds to the heterodimer S19 V24 Y44 R68 S70 N75 V77 / E28 R33 R38 K40 A44 H68 Q70 A105 R107 A151 G153 E158 ;
   - C9 corresponds to the heterodimer S19 V24 Y44 R68 S70 Q75 177 / E28 R33 R38 K40 A44 H68 Q70 A105 R107 A151 G153 E158 ;
   - C11 and E8 correspond to the heterodimer V24 Y44 S68 S70 R75 I77 A105 / E28 R33 R38 K40 A44 H68 Q70 A105 R107 A151 G153 E158, and
   - E6 corresponds to the heterodimer V24 Y44 S68 S70 R75 I77 G79 / E28 R33 R38 K40 A44 H68 Q70 A105 R107 A151 G153 E158.
      H10 is a negative control, H11 and H12 are positive controls of different intensity. To compare the activity of the heterodimers against the rosa1 target before and after the improvement of mutants cutting the rosa1.3 target: in each cluster, the two right points correspond to one of the heterodimers described in example 4 and the two left points to heterodimers with additional mutations, as described in example 5.

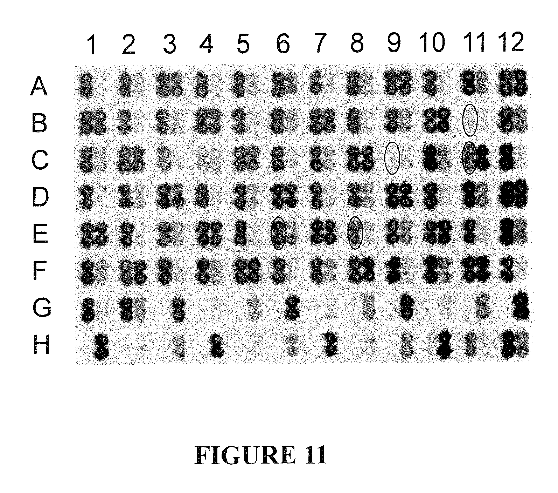

- figure 11 illustrates the screening of refined mutants displaying efficient cleavage of rosa1 in example 5 (circled), as homodimers in yeast, against the rosa1.3 palindromic target. In the filter:
   - B11corresponds to the mO_1 mutant (S19 V24 Y44 R68 S70 N75 V77); C9 corresponds to the mO_2 mutant (S 19 V24 Y44 R68 S70 Q75 I77); C11 and E8 correspond to the mO_3 mutant (V24 Y44 S68 S70 R75 I77 A105) and E6 correspond to the mO_ 4 mutant (V24 Y44 S68 S70 R75 I77 G79). H10 is a negative control, H11 and H12 are positive controls of different intensity. In each cluster, the two right points are one of the homodimers described in example 6 screened against the rosa1.3 target and the two left points are negative or positive controls of different intensity.

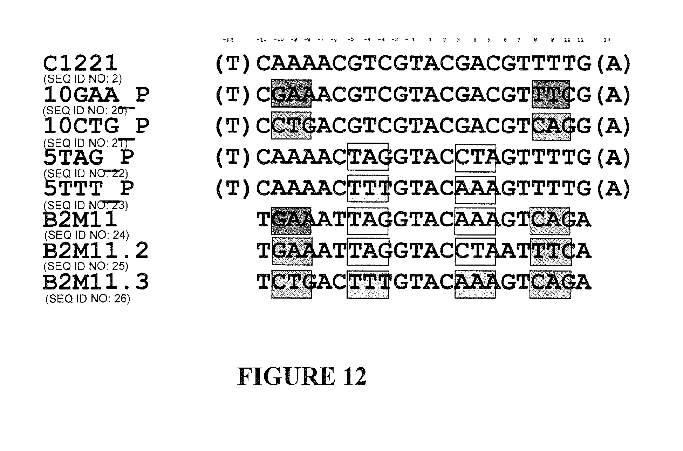

- figure 12 represents the B2M series of target. 10GAA_P, 10CTG_P, 5TAG_P and 5TTT_P are close derivatives found to be cleaved by previously obtained I-*Cre*I mutants. They differ from C1221 (palindromic sequence cleaved by the I-*Cre*I scaffold protein) by the boxed motives. C1221, 10GAA_P, 10CTG_P, 5TAG_P and 5TTT_P were first described as 24 bp sequences, but structural data suggest that only the 22 bp are relevant for protein/DNA interaction. However, positions ±12 are indicated in parenthesis. B2M11.2 and B2M11.3 are two palindromic sequences derived from the B2M11 target by mirror duplication of one half of the target. These two targets can in turn be considered as combinations of 10NNN and 5NNN targets found to be cleaved by I-CreI targets, if it is considered that nucleotides at positions ±11, ±7 and ±6 in the B2M11.2 and B2M11.3 targets have no impact on cleavage. All targets are aligned with the C1221 target, a palindromic sequence cleaved by I-*Cre*I.

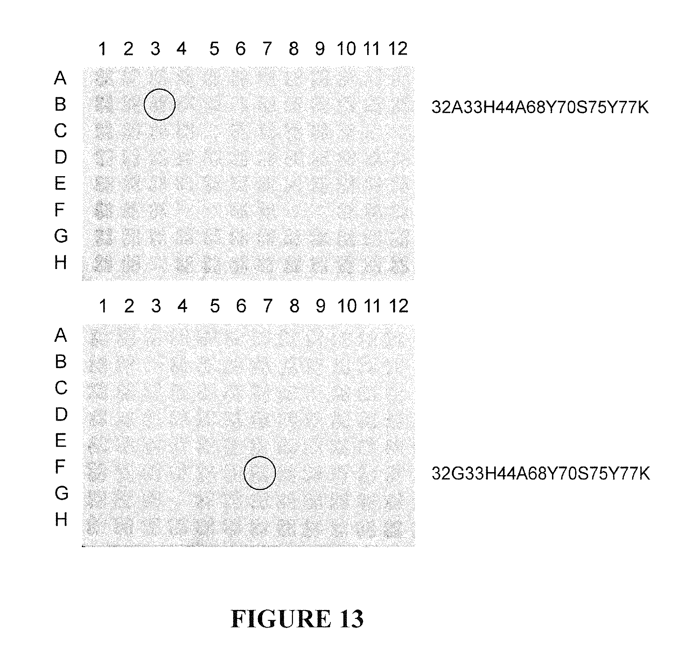

- figure 13 illustrates cleavage of the B2M11.2 target by combinatorial mutants. The figure displays an example of primary screening of I-*Cre*I combinatorial mutants with the B2M11.2 target. In the first top filter, the sequence of positive mutant at position B3 (circled) is KNAHQS/AYSYK (same nomenclature as for Table VIII). In the second filter (bottom), the sequence of positive mutant at position F7 is KNGHQS/AYSYK. In both panels, H12 correspond to a weak positive control (c).

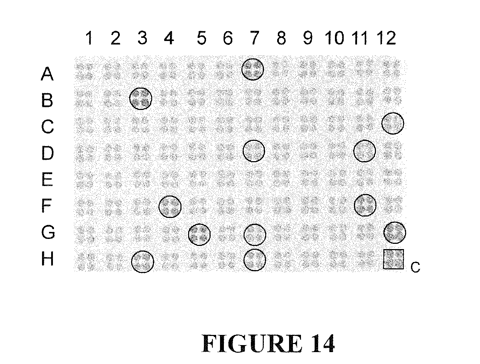

- figure 14 illustrates cleavage of the B2M11.2 target by optimized mutants. A series of I-*Cre*I N75 optimized mutants cutting B2M11.2 are obtained from random mutagenesis of the two mutants KNAHQS/AYSYK and KNGHQS/AYSYK. Cleavage is tested with the B2M11.2 target. Mutants cleaving B2M11.2 are circled, as example B3 is corresponding to 32A33H 44A68Y70S75Y77K/2Y53R66C (same nomenclature as for Table IX). H12 is a positive control.

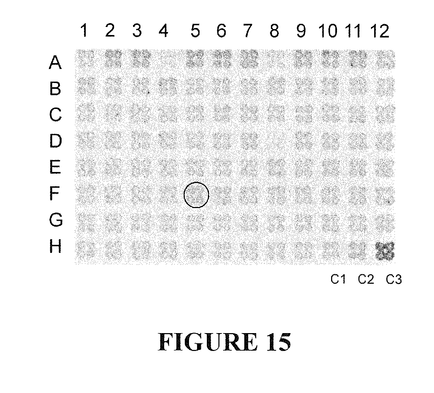

- figure 15 illustrates cleavage of the B2M11.3 target by combinatorial mutants. The figure displays an example of primary screening of I-*Cre*I combinatorial mutants with the B2M 11.3 target. H10, H11 and H12 are respectively negative (C1) and two positive controls (C2 and C3) of different strength. In the filter, the sequence of positive mutant at position G5 (circle) is KQSGCS/QNSNR (same nomenclature as for Table X).

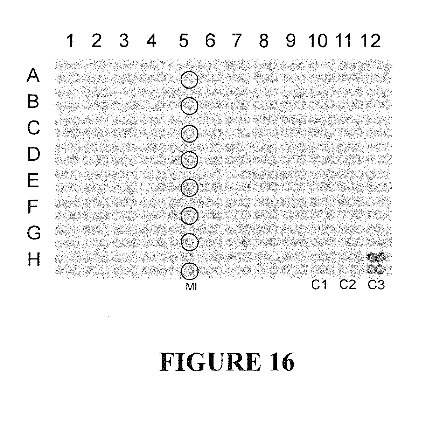

- figure 16 illustrates cleavage of B2M11 target by heterodimeric combinatorial mutants. The figure displays screening of combinations of I-*Cre*I mutants with the B2M11 target. A series of positive heterodimeric combinatorial mutants are circled on column 5. They all correspond to the 32A33H44A68Y70S75Y77K132V /30Q33G38C68N70S75N77R heterodimer listed in Table XI.

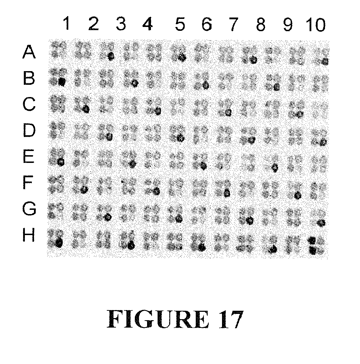

- figure 17 illustrates cleavage of B2M11 target by optimized heterodimeric combinatorial mutants. A series of I-*Cre*I N75 optimized mutants cutting B2M11.3 are coexpressed with mutants cutting B2M11.2. For example G9, corresponding to a heterodimer of 30Q33G38C68N70S75N77R vs 32A33H44A68Y70S75Y77K**2Y53R66C**. Cleavage is tested with the B2M11 target. The same heterodimeric combination is tested twice (two left dots) in each four dot cluster, whereas dot (top right) corresponds to the 32A33H44A68Y70S75Y77K2Y53R66C/30Q33G38C68N70S75N77R control (before random mutagenesis of the meganucleases cleaving B2M11.3). The fourth dot from each cluster corresponds either to a negative control (no meganuclease), either to a strong positive control (I-*Sce*I with I-*Sce*I target), either to a medium strength positive control (an I-*Sce*I protein expressed from an ORF with an altered codon usage, which in this assay, results in a lower signal with the I-*Sce*I target).

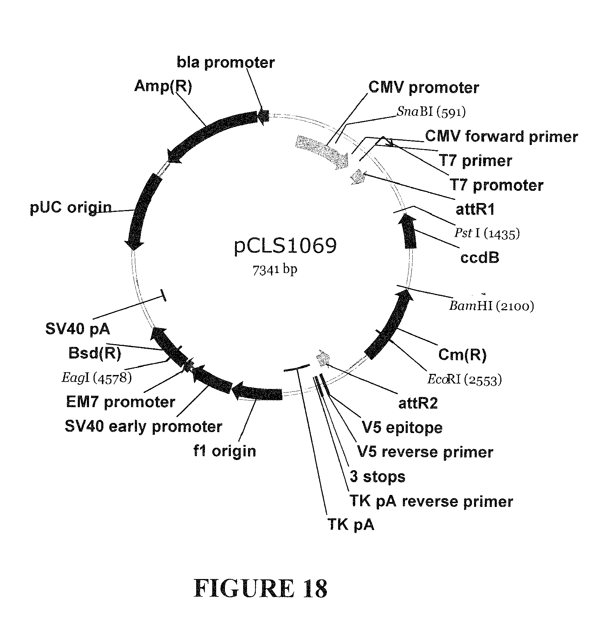

- figure 18 represents the map of pCLS1069, a plasmid for meganuclease expression in mammalian cells after Gateway cloning.

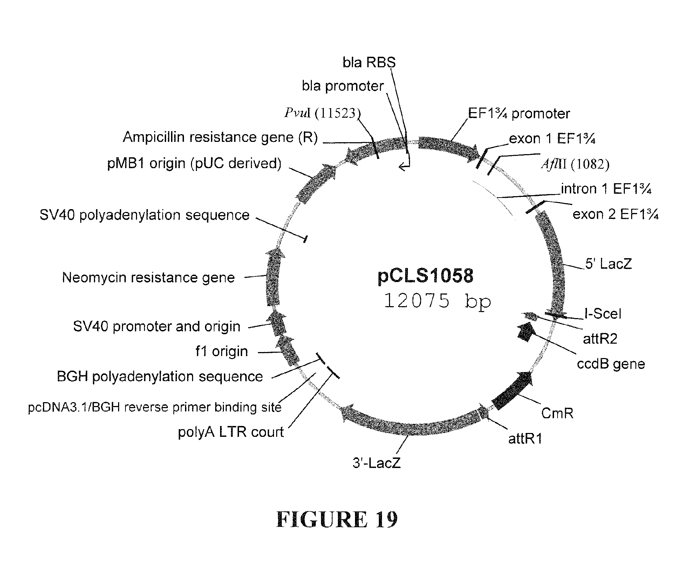

- figure 19 represents the map of pCLS1058, a plasmid for gateway cloning of DNA targets in a reporter vector for mammalian cells.

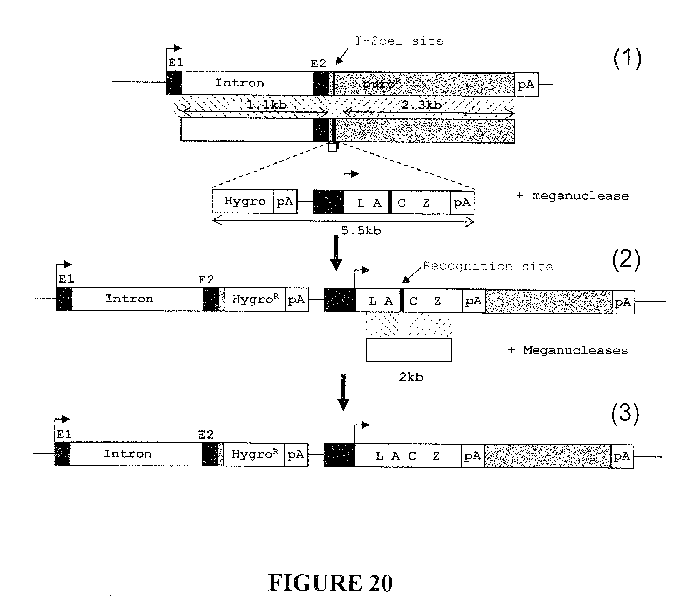

- figure 20 illustrates the design of reporter system in mammalian cells. The puromycin resistance gene, interrupted by an I-*Sce*I cleavage site 132bp downstream of the start codon, is under the control of the EFIα promoter (1). The transgene has been stably expressed in CHO-K1 cells in single copy. In order to introduce Meganuclease target sites in the same chromosomal context, the repair matrix is composed of i) a promoterless hygromycin resistance gene, ii) a complete lacZ expression cassette and iii) two arms of homologous sequences (1.1 kb and 2.3 kb). Several repair matrixes have been constructed differing only by the recognition site that interrupts the lacZ gene (2). Thus, very similar cell lines have been produced as A1 cell line, I-*Sce*I cell line and I-*Cre*I cell line. A functional lacZ gene is restored when a lacZ repair matrix (2kb in length) is co-transfected with vectors expressing a meganuclease cleaving the recognition site (3). The level of meganuclease-induced recombination can be inferred from the number of blue colonies or foci after transfection.

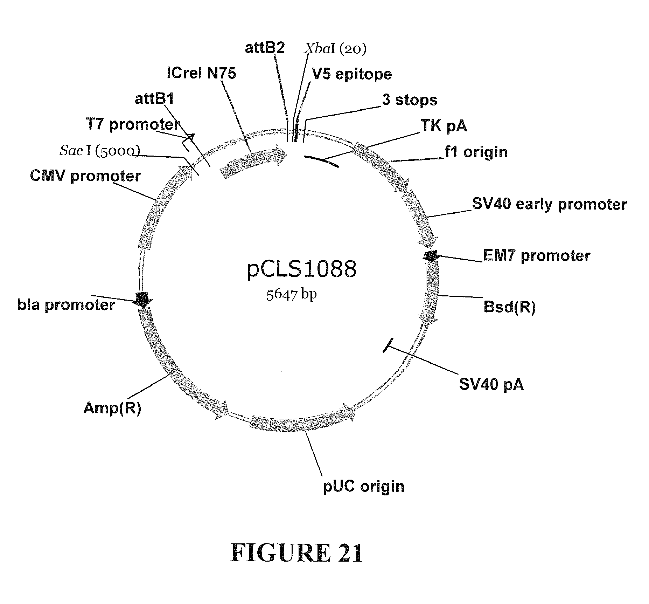

- figure 21 represents the map of pCLS1088, a plasmid for expression of I-*Cre*I N75 in mammalian cells.

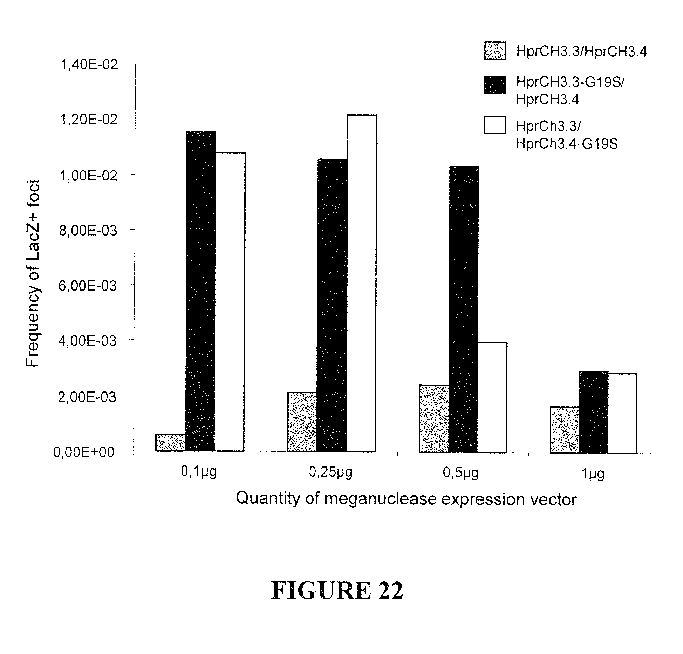

- figure 22 illustrates cleavage efficiency of meganucleases cleaving the HprCH3 DNA target sequence. The frequency of repair of the LacZ gene is detected after transfection of CHO cells containing a HprCH3 chromosomal reporter system, with a repair matrix and various quantities of meganuclease expression vectors, coding for the initial engineered heterodimers (HprCH3.3 / HprCH3.4) or their G19S derivatives (HprCH3.3 / HprCh3.4 G19S or HprCH3.3 G19S / HprCh3.4).

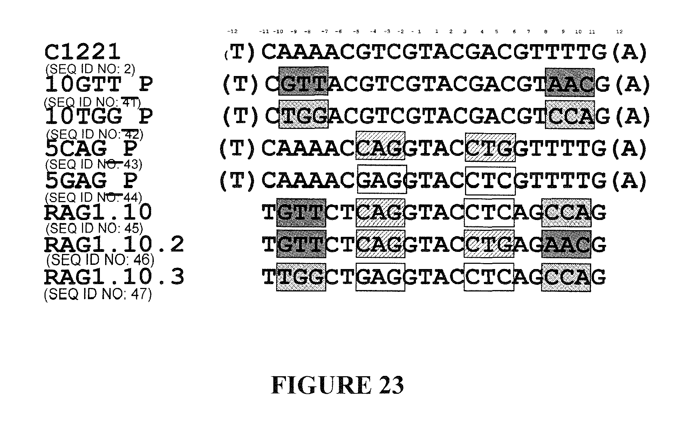

- figure 23 illustrates the RAG1.10 series of target. 10GTT_P, 10TGG_P, 5CAG_P and 5GAG_P are close derivatives found to be cleaved by previously obtained I-*Cre*I mutants. They differ from C1221 (palindromic sequence cleaved by the I-CreI scaffold protein) by the boxed motives. C1221, 10GTT_P, 10TGG_P, 5CAG_P and 5GAG_P were first described as 24 bp sequences, but structural data suggest that only the 22 bp are relevant for protein/DNA interaction. However, positions ±12 are indicated in parenthesis. RAG1.10.2 and RAG1.10.3 are two palindromic sequences derived from the RAG1.10 target by mirror duplication of one half of the target. These two targets can in turn be considered as combinations of 10NNN and 5NNN targets found to be cleaved by I-CreI targets, if we consider that nucleotides at positions ±11, ±7 and ±6 in the RAG1.10.2 and RAG1.10.3 targets have no impact on cleavage. All targets are aligned with the C1221 target, a palindromic sequence cleaved by I-*Cre*I.

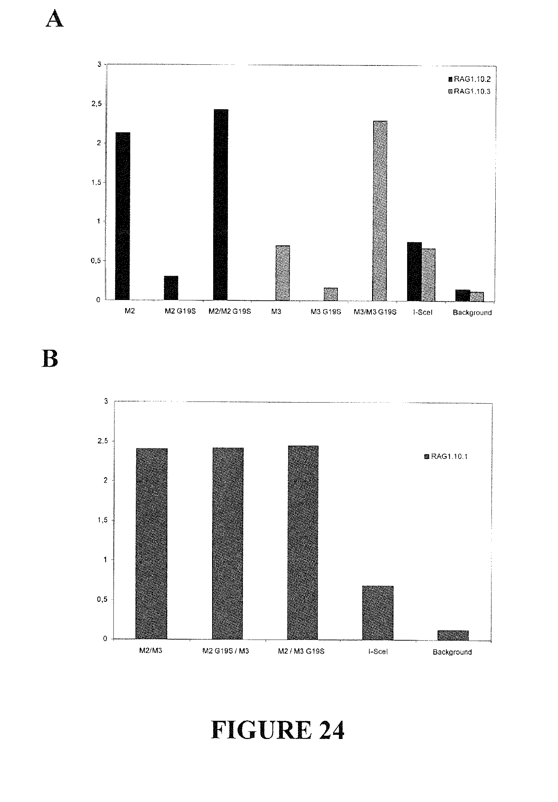

- figure 24 illustrates cleavage of the RAG1.10, RAG1.10.2 and RAG1.10.3 targets by M2 and M3 I-*Cre*I mutants with or without the G19S mutation in an extrachromosomal assay in CHO cells. The cleavage of the palindromic targets RAG1.10.2 and RAG1.10.3 is shown in panel A, while RAG1.10 cleavage is by heterodimeric meganucleases is shown in panel B. Cleavage of I-*Sce*I target by in the same experiments is shown as positive control.

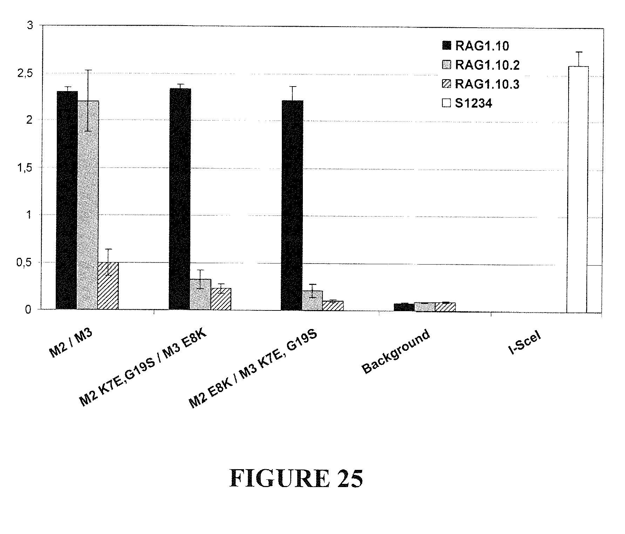

- figure 25: illustrates the activity of three RAG1.10 heterodimers against the three RAG1.10 targets is monitored in an extrachromosomal assay in CHO cells. Background corresponds to the transfection of the cells with an empty expression vector. Cleavage of the S 1234 target by I-*Sce*I in the same experiment is shown as a positive control.

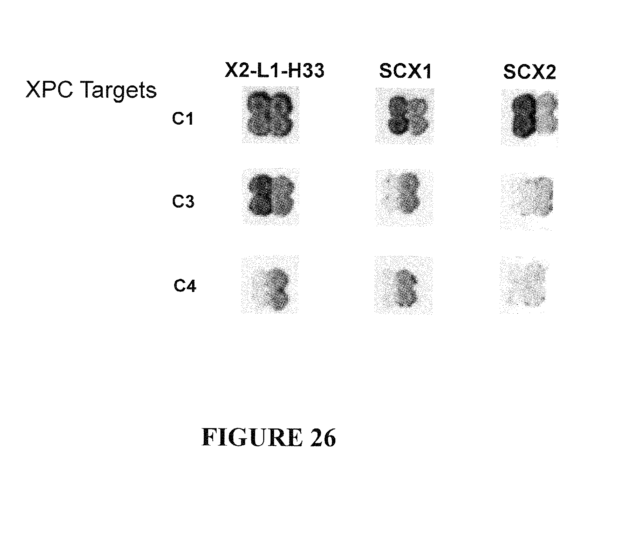

- figure 26 illustrates the yeast screen of three XPC single chain molecules X2-L1-H33, SCX1 and SCX2 against the three XPC targets (C1, C3 and C4). SCX1 is the X2(K7E)-L1-H33(E8K,G19S) molecule and SCX2 stands for the X2(E8K)-L1-H33(K7E,G19S) molecule. For each 4 dots yeast cluster, the two left dots are the result of the experiment, while the two right dots are various internal controls to assess the experiment quality and validity.

### Example 1: Making of meganucleases cleaving rosa1.3

This example shows that I*-Cre*I mutants can cut the rosa1.3 DNA target sequence derived from the left part of the rosa1 target in a palindromic form (Figure 2). Targets sequences described in this example are 22 bp palindromic sequences. Therefore, they will be described only by the first 11 nucleotides, followed by the suffix _P. For example, target rosa1.3 will be noted also caacat**gat**gt_P; SEQ ID NO: 9).

The rosa1.3 target is similar to 5GAT_P at positions ±1, ±2, ±3, ±4, ±5, ±7, ±9, ±10 and ±11, the two sequences differing only at positions ±6 and ±8. It was hypothesized that positions ±6 would have little effect on the binding and cleavage activity. Mutants able to cleave 5GAT_P (caaaac**gat**gt_P; SEQ ID NO: 5) were previously obtained by mutagenesis on I*-Cre*I N75 at positions 24, 44, 68, 70, 75 and 77, as described in Arnould et al., J Mol Biol. 2006; 355, 443-458 and International PCT Applications WO 2006/097784 and WO 2006/097853. In this example, it was checked whether mutants cleaving the 5GAT_P target could also cleave the rosa1.3 target.

### 1) Material and Methods

The method for producing meganuclease variants and the assays based on cleavage-induced recombination in mammal or yeast cells, which are used for screening variants with altered specificity are described in the International PCT Application WO 2004/067736; Epinat et al., Nucleic Acids Res., 2003, 31, 2952-2962; Chames et al., Nucleic Acids Res., 2005, 33, e178, and Arnould et al., J. Mol. Biol., 2006, 355, 443-458. These assays result in a functional LacZ reporter gene which can be monitored by standard methods.

### a) Construction of target vector

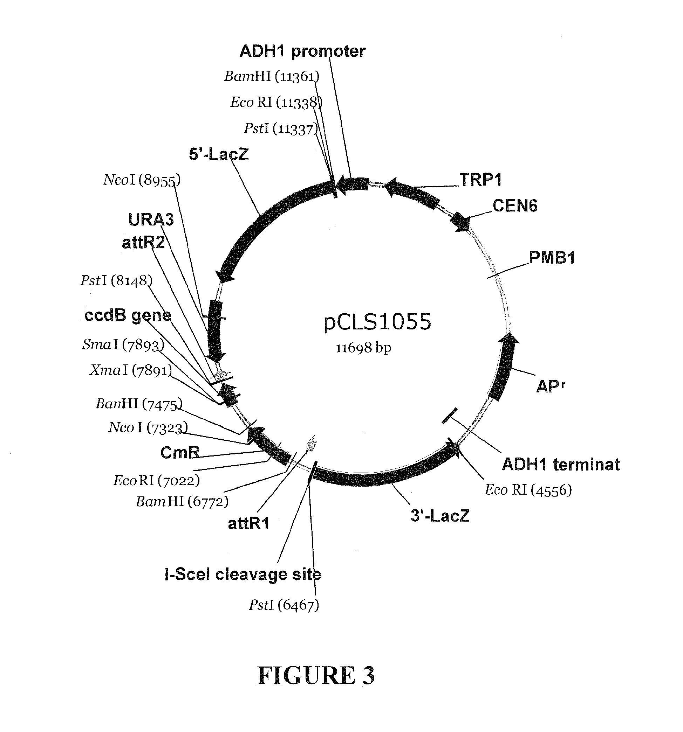

The target was cloned as follow: oligonucleotide corresponding to the target sequence flanked by gateway cloning sequence was ordered from Proligo: 5' tggcatacaagtttcaacatgatgtacatcatgttgacaatcgtctgtca 3'(SEQ ID NO: 11). Doublestranded target DNA, generated by PCR amplification of the single stranded oligonucleotide, was cloned using the Gateway protocol (INVITROGEN) into yeast reporter vector (pCLS1055, Figure 3). Yeast reporter vector was transformed into S. *cerevisiae* strain FYBL2-7B (*MAT a*, *ura3Δ851, trp1Δ63, leu2Δ1, lys2Δ202*).

### b) I-CreI mutants

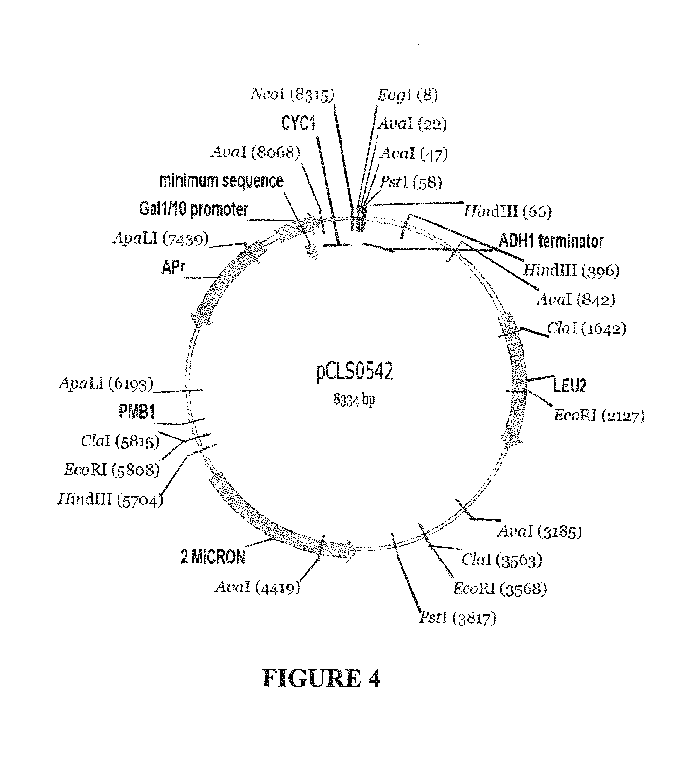

I-*Cre*I mutants cleaving 5GAT_P were identified in a library where positions 24, 44, 68, 70, 75 and 77 of I*-Cre*I are mutated, as described previously in Arnould et al., J. Mol. Biol., 2006, 355, 443-458 and International PCT Applications WO 2006/097784 and WO 2006/097853. They are cloned in the DNA vector (pCLS0542, Figure 4) and expressed in the yeast *Saccharomyces cerevisiae* strain FYC2-6A (MATα, trp1Δ63, leu2Δ1, his3Δ200).

### c) Mating of meganuclease expressing clones and screening in yeast

Screening was performed as described previously (Arnould et al., J. Mol. Biol. 2006, 355, 443-458). Mating was performed using a colony gridder (QpixII, Genetix). Mutants were gridded on nylon filters covering YPD plates, using a low gridding density (about 4 spots/cm²). A second gridding process was performed on the same filters to spot a second layer consisting of different reporter-harboring yeast strains for each target. Membranes were placed on solid agar YPD rich medium, and incubated at 30 °C for one night, to allow mating. Next, filters were transferred to synthetic medium, lacking leucine and tryptophan, with galactose (2 %) as a carbon source, and incubated for five days at 37 °C, to select for diploids carrying the expression and target vectors. After 5 days, filters were placed on solid agarose medium with 0.02 % X-Gal in 0.5 M sodium phosphate buffer, pH 7.0, 0.1% SDS, 6% dimethyl formamide (DMF), 7mM β-mercaptoethanol, 1 % agarose, and incubated at 37°C, to monitor β-galactosidase activity. Results were analyzed by scanning and quantification was performed using appropriate software.

### d) Sequencing of mutants

To recover the mutant expressing plasmids, yeast DNA was extracted using standard protocols and used to transform *E*. *coli.* Sequence of mutant ORF were then performed on the plasmids by MILLEGEN SA. Alternatively, ORFs were amplified from yeast DNA by PCR (Akada et al., Biotechniques, 2000, 28, 668-670, 672, 674), and sequence was performed directly on PCR product by MILLEGEN SA.

### 2) Results

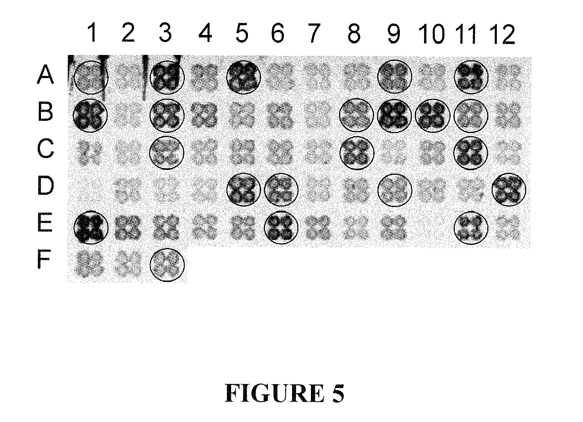

I-*Cre*I mutants cleaving the 5GAT_P target, previously identified in a library where positions 24, 44, 68, 70, 75 and 77 of I*-Cre*I are mutated, were screened for cleavage against the rosa1.3 DNA target (caacat**gat**gt_P; SEQ ID NO: 9). A total of 63 positive clones were found, rearranged in a 96-well plate and validated by secondary screening (Figure 5). Among those positive clones, 22 (circled in Figure 5). were chosen. Those 22 positives clones were sequenced. They turned out to correspond to 18 different proteins described in Table I.

**Table I: I-CreI mutants capable of cleaving the rosa1.3 DNA target.**

| **I-*Cre*I mutant position on** **figure 5** | **name** | **amino acids at positions 24, 44, 68, 70, 75 and 77 (ex: VYRSYI stands for V24, Y44, R68, S70, Y75 and 177)** |
|---|---|---|
| A1 and F3 | m1 | VYRSYI |
| A3 | m2 | VYRSNI |
| A5 and B1 | m3 | VYDSRR |
| A9 | m4 | ITYSYR |
| A11 | m5 | VYRSYQ |
| B3, D5 and E6 | m6 | VYYSYR |
| B8 | m7 | VYYSRA |
| B9 | m8 | VYRSNV |
| B10 | m9 | VNYSYR |
| B11 | m10 | VNYSYR + 82T* |
| C3 | m11 | VYSSRV |
| C8 | m12 | VYNSRI |
| C11 | m13 | VYSSRI |
| D6 | m14 | VYRSQI |
| D9 | m15 | IYRSNI |
| D12 | m16 | VYYSRV |
| E1 | m17 | VYRSYT |
| E11 | m18 | VNSSRV |

| | | |
|---|---|---|
| * 82T in m 10 is unexpected mutation may be due to PCR reaction before sequencing of yeast DNA. | | |

### Example 2: Making of meganucleases cleaving rosa1.4

This example shows that I*-Cre*I mutants can cleave the rosa1.4 DNA target sequence derived from the right part of the rosa1 target in a palindromic form (Figure 2). All targets sequences described in this example are 22 bp palindromic sequences. Therefore, they will be described only by the first 11 nucleotides, followed by the suffix _P. For example, rosa1.4 will be called t**ggg**at**tat**gt_P (SEQ ID NO: 10).

The rosa1.4 target is similar to 5TAT_P at positions ±1, ±2, ±3, ±4, ±5 and ±7 and to 10GGG_P at positions ±1, ±2, ±7, ±8, ±9 and ±10. It was hypothesized that positions ±6 and ±11 would have little effect on the binding and cleavage activity. Mutants able to cleave 5TAT_P were previously obtained by mutagenesis on *I-CreI* N75 at positions 44, 68, 70, as described in Arnould et al., J Mol. Biol., 2006; 355, 443-458 and INternational PCT Applications WO 2006/097784 and WO 2006/097853. Mutants able to cleave the 10GGG_P target were obtained by mutagenesis on I*-Cre*I N75 at positions 28, 30, 33, 38, 40 and 70 as described in Smith et al., Nucleic Acids Research, 2006, 34, e149 and International PCT Application WO 2007/049156.

Both sets of proteins are mutated at position 70. However, it was hypothesized that two separable functional subdomains exist. That implies that this position has little impact on the specificity towards the bases ± 8 to 10 of the target.

Therefore, to check whether combined mutants could cleave the rosa1.4 target, mutations at positions 44, 68 and 70 from proteins cleaving 5TAT_P (caaaac**tat**gt_P; SEQ ID NO: 6) were combined with the 28, 30, 33, 38 and 40 mutations from proteins cleaving 10GGG_P (c**ggg**acgtcgt_P; SEQ ID NO: 4)

### 1) Material and Methods

The experimental procedures are as described in example 1 and as follows:

### Construction of combinatorial mutants

I*-Cre*I mutants cleaving 10GGG_P or 5TAT_P were identified in Smith et al, Nucleic Acids Res., 2006, 34, e149; International PCT Application WO 2007/049156, and Arnould et al., J. Mol. Biol., 2006, 355, 443-458; International PCT Applications WO 2006/097784 and WO 2006/097853, respectively for the 10GGG_P or 5TAT_P targets. In order to generate I-*Cre*I derived coding sequence containing mutations from both series, separate overlapping PCR reactions were carried out that amplify the 5' end (aa positions 1-43) or the 3' end (positions 39-167) of the I*-Cre*I coding sequence. For both the 5' and 3' end, PCR amplification is carried out using primers Gal10F 5'-gcaactttagtgctgacacatacagg-3' (SEQ ID NO: 12) or Gal10R 5'-acaaccttgattggagacttgacc-3' (SEQ ID NO:13 ), specific to the vector (pCLS0542, Figure 4) and primers assF 5'-ctannnttgaccttt-3' (SEQ ID NO: 14) or assR 5'-aaaggtcaannntag-3' (SEQ ID NO: 15) where nnn code for residue 40, specific to the I-*Cre*I coding sequence for amino acids 39-43. The PCR fragments resulting from the amplification reaction realized with the same primers and with the same coding sequence for residue 40 were pooled. Then, each pool of PCR fragments resulting from the reaction with primers Gal10F and assR or assF and Gal10R was mixed in an equimolar ratio. Finally, approximately 25ng of each final pool of the two overlapping PCR fragments and 75 ng of vector DNA (pCLS0542) linearized by digestion with *Nco*I and *Eag*I were used to transform the yeast *Saccharomyces cerevisiae* strain FYC2-6A (MATα, trp1Δ63, leu2Δ1, his3Δ200) using a high efficiency LiAc transformation protocol (Gietz, R. D. and R.A. Woods, Methods Enzymol. 2002, 350, 87-96). An intact coding sequence containing both groups of mutations is generated by *in vivo* homologous recombination in yeast.

### 2) Results

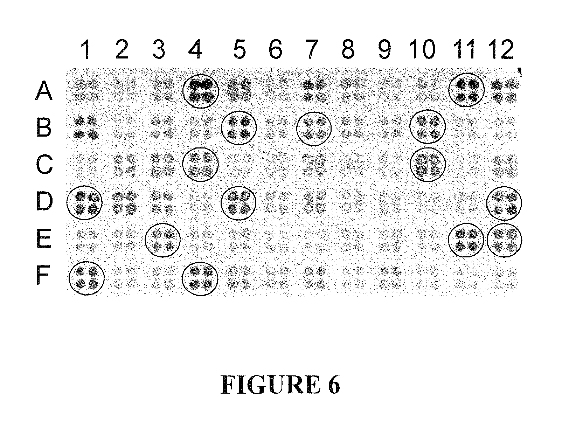

I-*Cre*I combinatorial mutants were constructed by associating mutations at positions 44, 68 and 70 with the 28, 30, 33, 38 and 40 mutations on the I-*Cre*I N75 scaffold, resulting in a library of complexity 2208. Examples of combinatorial mutants are displayed in Table II. This library was transformed into yeast and 3456 clones (1.5 times the diversity) were screened for cleavage against the rosa1.4 DNA target (t**ggg**at**tat**gt_P; SEQ ID NO:10 ). A total of 69 positive clones were found and were rearranged in a 96-well plate and validated by secondary screening (Figure 6). Among those positives, 15 clones (circled in Figure 6) were chosen. After sequencing, these 15 clones turned out to correspond to 8 different novel endonucleases cleaving the rosa1.4 DNA target (Table II).

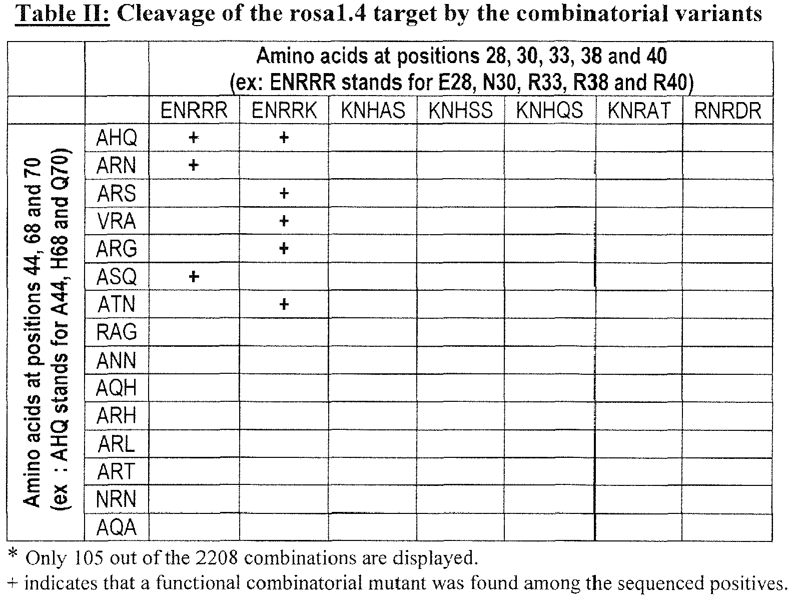

### Example 3: Making of meganucleases cleaving rosa1

I-*Cre*I mutants able to cleave each of the palindromic rosa1 derived targets (rosa1.3 and rosa1.4) were identified in examples 1 and 2. Pairs of such mutants (one cutting rosa1.3 and one cutting rosa1.4) were co-expressed in yeast. Upon coexpression, there should be three active molecular species, two homodimers, and one heterodimer. It was assayed whether the heterodimers that should be formed cut the non palindromic rosa1 and rosa1.2 DNA targets.

### 1) Material and Methods

### a) Cloning of mutants in kanamycin resistant vector

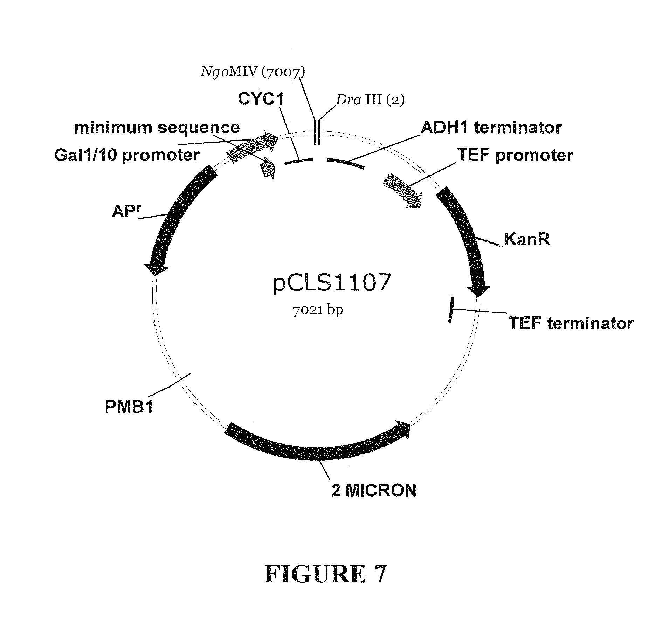

To coexpress two I*-Cre*I mutants in yeast, mutants cutting the rosa1.4 sequence were subcloned in yeast expression vector marked with a kanamycin resistance gene (pCLS1107, Figure 7). Mutants were amplified by PCR reaction using primers common for pCLS0542 and pCLS1107: Gal10F 5'-gcaactttagtgctgacacatacagg-3' (SEQ ID NO: 12) and Gal10R 5'-acaaccttgattggagacttgacc-3' (SEQ ID NO:13 ). Approximately 25 ng of PCR fragment and 25 ng of vector DNA (pCLS1107) linearized by digestion with *DraIII* and *NgoMIV* are used to transform the yeast *Saccharomyces cerevisiae* strain FYC2-6A (MATα, trp1Δ63, leu2Δ1, his3Δ200) using a high efficiency LiAc transformation protocol. An intact coding sequence for the I*-Cre*I mutant is generated by *in vivo* homologous recombination in yeast. Each yeast strain containing a mutant cutting the rosa1.4 target subcloned in pCLS1107 vector was then mated with yeast expressing the rosa1.4 target to validate it. To recover the mutant expressing plasmids, yeast DNA was extracted using standard protocols and used to transform *E*. *coli.* and prepare *E*. *coli* DNA.

### b) Mutants coexpression

Yeast strain expressing a mutant cutting the rosa1.3 target in pCLS0542 expression vector was transformed with DNA coding for a mutant cutting the rosa1.4 target in pCLS1107 expression vector. Transformants were selected on -L Glu + G418 medium.

### c) Mating of meganucleases coexpressing clones and screening in yeast

Mating was performed using a colony gridder (QpixII, Genetix). Mutants were gridded on nylon filters covering YPD plates, using a low gridding density (about 4 spots/cm²). A second gridding process was performed on the same filters to spot a second layer consisting of different reporter-harbouring yeast strains for each target. Membranes were placed on solid agar YPD rich medium, and incubated at 30 °C for one night, to allow mating. Next, filters were transferred to synthetic medium, lacking leucine and tryptophan, adding G418, with galactose (2 %) as a carbon source, and incubated for five days at 37 °C, to select for diploids carrying the expression and target vectors. After 5 days, filters were placed on solid agarose medium with 0.02 % X-Gal in 0.5 M sodium phosphate buffer, pH 7.0, 0.1 % SDS, 6% dimethyl formamide (DMF), 7mM β-mercaptoethanol, 1 % agarose, and incubated at 37 °C, to monitor β-galactosidase activity. Results were analyzed by scanning and quantification was performed using proprietary software.

### 2) Results

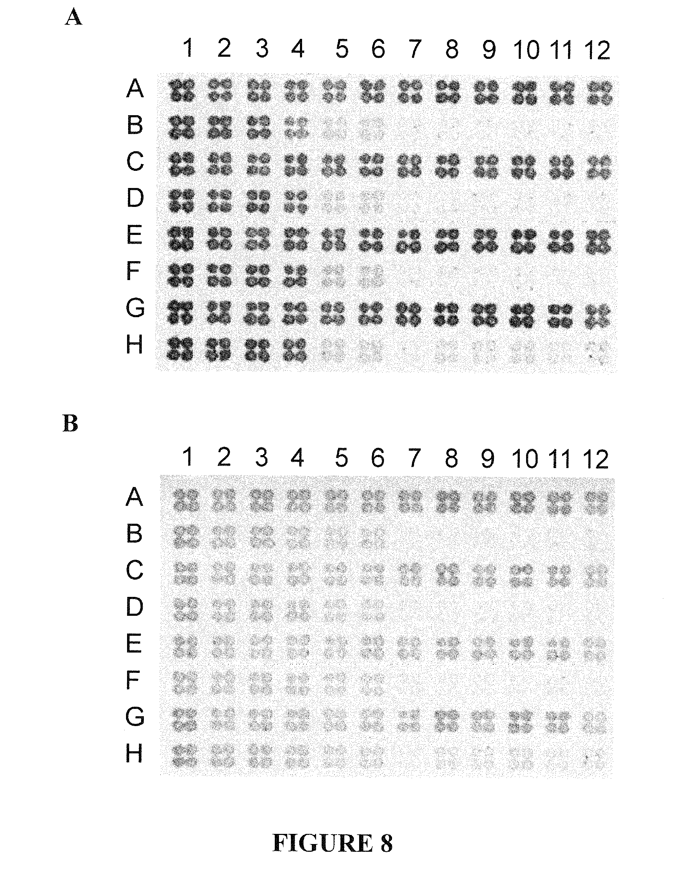

Coexpression of mutants cleaving the rosa1.3 target (m1 to m18 described in Table I) and the eight mutants cleaving the rosa1.4 target (described in Table II) resulted in efficient cleavage of the rosa1.2 target in all the cases (screen examples are shown in Figure 8A). All combinations tested are summarized in Table III. Most of these combinations are also able to cut the rosa1 natural target that differs from the rosa1.2 sequence just by 1 bp at position +1 (Figure 2). As shown on Figure 8B, the signal observed on rosa1 natural target is weak compared to the one observed on rosa1.2 target. The combinations cleaving the rosa1 DNA target are presented in Table IV.

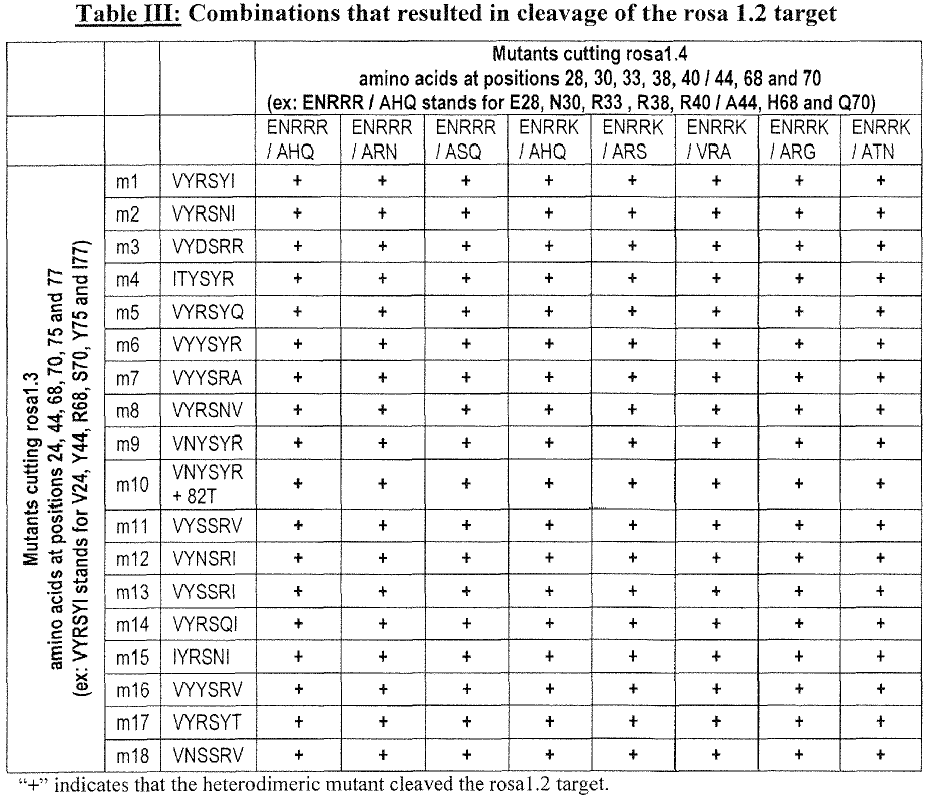

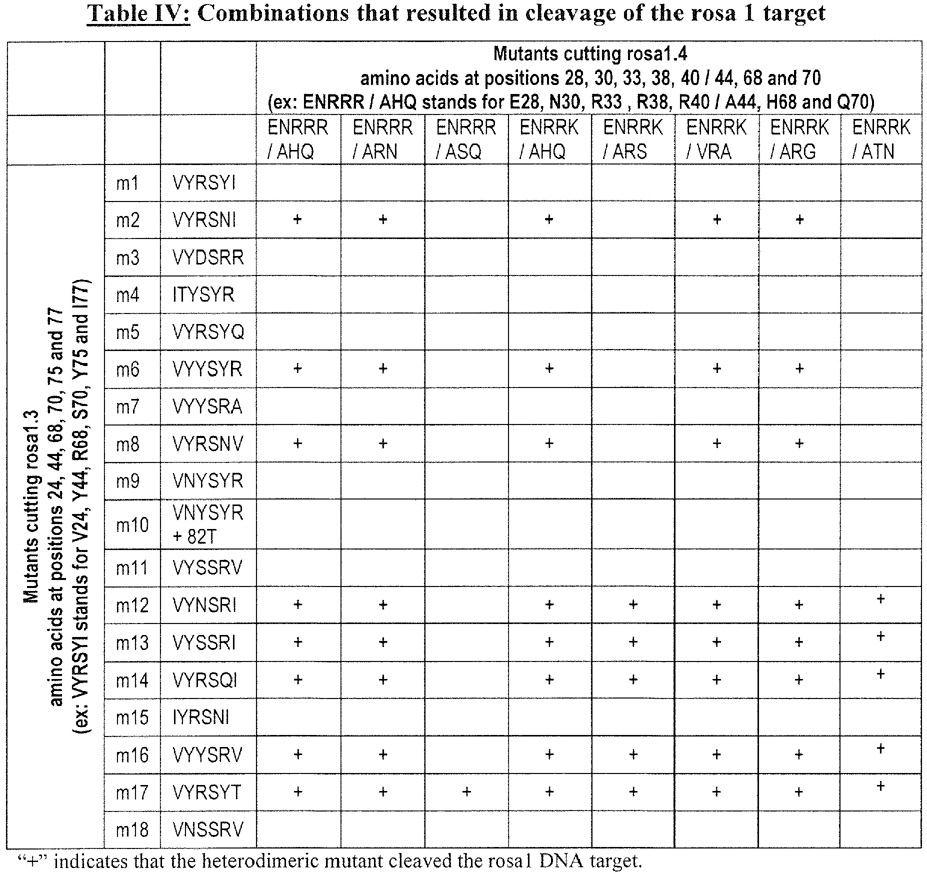

### Example 4: Refinement of meganucleases cleaving rosa1 by random mutagenesis of proteins cleaving rosa1.4 and assembly with proteins cleaving rosa1.3

I*-Cre*I mutants able to cleave the non palindromic rosa1.2 and rosa1 targets by assembly of mutants cleaving the palindromic rosa1.3 and rosa1.4 targets. However, the combinations were able to efficiency cleave rosa1.2 but weakly cleave rosa1, which differs from rosa1.2 only by 1 bp at position 1. The signal observed on rosa1 is not sufficient.

Therefore, protein combinations cleaving rosa1 were mutagenized at random, and mutants cleaving rosa1 efficiently were screened. According to the structure of the I*-Cre*I protein bound to its target, there is no contact between the 4 central base pairs (positions -2 to 2) and the I*-Cre*I protein (Chevalier, B. S. and B.L. Stoddard, Nucleic Acids Res., 2001, 29, 3757-3774; Chevalier et al., Nat. Struct. Biol., 2001, 8, 312-316; Chevalier et al., J. Mol. Biol. 2003, 329, 253-269). Thus, it is difficult to rationally choose a set of positions to mutagenize, and mutagenesis was done on the C-terminal part of the protein (83 last amino acids) or on the whole protein. Random mutagenesis results in high complexity libraries, and the complexity of the variants libraries to be tested was limited by mutagenizing only one of the two components of the heterodimers cleaving rosa1.

Thus, proteins cleaving rosa1.4 were mutagenized randomly, and it was tested whether they could efficiently cleave rosa1 when co-expressed with proteins cleaving rosa1.3.

### 1) Material and Methods

### a) Random mutagenesis

Random mutagenesis libraries were created on pools of chosen mutants, by PCR using Mn²⁺ or derivatives of dNTPs as 8-oxo-dGTP and dPTP in two-step PCR process as described in the protocol from JENA BIOSCIENCE GmbH in JBS dNTP-Mutageneis kit. For random mutagenesis on the whole protein, primers used are: preATGCreFor 5'-gcataaattactatacttctatagacacgcaaacacaaatacacagcggcctt gccacc-3'; SEQ ID NO: 16) and ICreIpostRev 5'-ggctcgaggagctcgtctagaggatcgctcgag ttatcagtcggccgc -3' (SEQ ID NO: 17). For random mutagenesis on the C-terminal part of the protein, primers used are AA78a83For (5'-ttaagcgaaatcaagccg-3'; SEQ ID NO: 18) and ICreIpostRev with dNTPs derivatives; the rest of the protein is amplified with a high fidelity taq polymerase and without dNTPs derivatives using primers preATGCreFor and AA78a83Rev (5'-cggcttgatttcgcttaa-3'; SEQ ID NO: 19).

Pools of mutants were amplified by PCR reaction using these primers common for pCLS0542 (Figure 4) and pCLS1107 (Figure 7). Approximately 75 ng of PCR fragment and 75 ng of vector DNA (pCLS1107) linearized by digestion with *DraIII* and *NgoMIV* are used to transform the yeast *Saccharomyces cerevisiae* strain FYC2-6A (MATα, trp1Δ63, leu2Δ1, his3Δ200) using a high efficiency LiAc transformation protocol. A library of intact coding sequence for the I*-Cre*I mutant is generated by *in vivo* homologous recombination in yeast. Positives resulting clones were verified by sequencing as described in example 1.

### b) Cloning of mutants in leucine expression vector in the yeast strain containing the rosa1 target

The yeast strain FYBL2-7B (MAT a, ura3Δ851, trp1Δ63, leu2Δ1, lys2Δ202) containing the rosa1 target into yeast reporter vector pCLS1055 (Figure 3) is transformed with mutants cutting rosa1.3 target, in the pCLS0542 vector marked with *LEU2* gene, using a high efficiency LiAc transformation protocol. The resulting yeast strains are used as targets for mating assays as described in example 3.

### 2) Results

Four mutants cleaving rosa1.4 (ERRR / AHQ, ERRR / ARN, ERRK / AHQ and ERRK / VRA according to Table IV) were pooled, randomly mutagenized on all proteins or on the C terminal part of proteins and transformed into yeast. 4464 transformed clones were then mated with a yeast strain that (i) contains the rosa1 target in a reporter plasmid (ii) expresses a variant cleaving the rosa1.3 target, chosen among those described in example 1. Three such strains were used expressing the I-*Cre*I V24 Y44 S68 S70 R75 I77 (or VYSSRI) mutant, or the I-CreI V24 Y44 R68 S70 Q75 I77 (or VYRSQI) mutant, or the I*-Cre*I V24 Y44 R68 S70 Y75 T77 (or VYRSYT) mutant (see Table I). Two clones were found to trigger a better cleavage of the rosa1 target when mated with such yeast strain compared to the mutants before mutagenesis with the same yeast strain. In conclusion, two proteins able to efficiently cleave rosa1 when forming heterodimers with VYSSRI, VYRSQI or VYRSYT (Table V and Figure 9). Interestingly, both proteins contain the A105 and R107 residues.

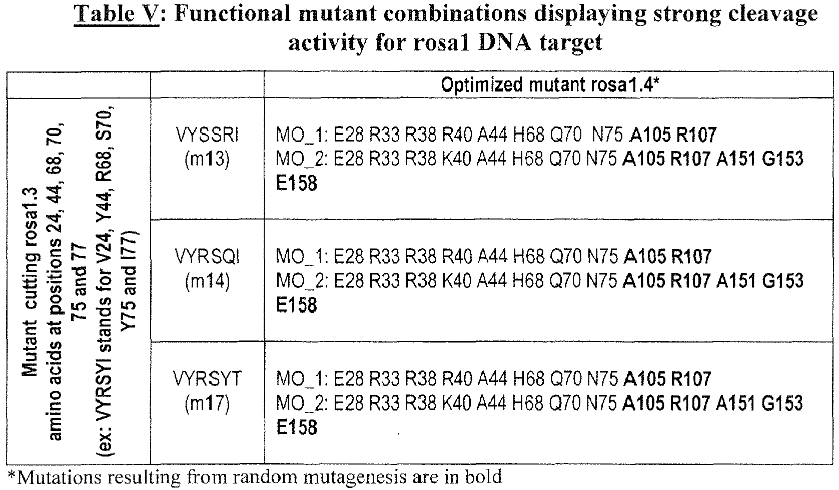

### Example 5: Refinement of meganucleases cleaving rosa1 by random mutagenesis of proteins cleaving rosa1.3 and assembly with refined proteins cleaving rosa1.4

I*-Cre*I mutants able to cleave the rosa1 target were identified by assembly of mutants cleaving rosa1.3 and refined mutants cleaving rosa1.4 To increase the activity of the meganucleases, the second component of the heterodimers cleaving rosa1 was randomly mutagenized. In this example, mutants cleaving rosa1.3 were randomly mutagenized, followed by screening of more efficient variants cleaving rosa1 in combination with the refined mutants cleaving rosa1.4 identified in example 4.

### 1) Material and Methods

The experimental procedure is as described in example 4, except that PCR product was cloned in pCLS0542 vector (Figure 4) linearized by digestion with *NcoI* and *EagI.*

### Cloning of mutants in Kan^{R} expression vector in the yeast strain containing the rosa1 target

The yeast strain FYBL2-7B (MAT a, ura3Δ851, trp1Δ63, 1eu2Δ1, lys2Δ202) containing the rosa1 target into yeast reporter vector (pCLS1055, Figure 3) is transformed with MO_1 and MO_2 refined mutants cutting rosa1.4 target, in pCLS1107 vector, using a high efficiency LiAc transformation protocol. Mutant-target yeasts are used as targets for mating assays as described in example 3.

### 2) Results

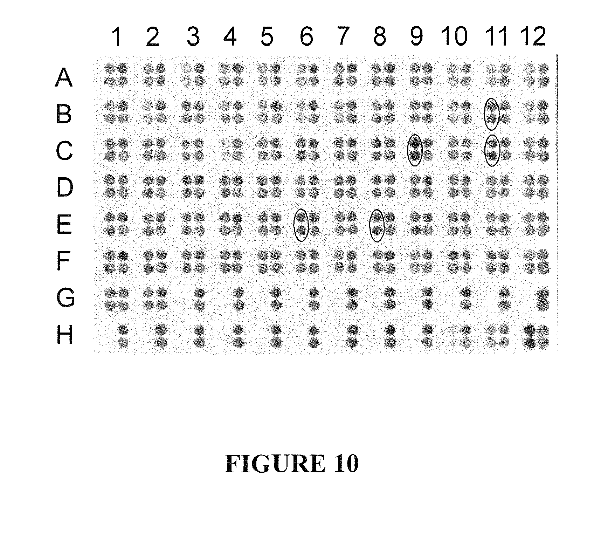

Two pools of four mutants cleaving rosa1.3 (pool 1: VYRSNI, VYYSYR, VYRSNV and VYNSRI and pool 2: VYYSYR, VYSSRI, VYRSQI and VYRSYT according to Table IV) were randomly mutagenized on all proteins or on the C terminal part of proteins and transformed into yeast. 8928 transformed clones were then mated with a yeast strain that (i) contains the rosa1 target in a reporter plasmid (ii) expresses a variant cleaving the rosa1.4 target. Two such strains were used expressing either the I*-Cre*I E28 R33 R38 R40 A44 H68 Q70 N75 A105 R107 (or MO_1) mutant, either the I-*Cre*I E28 R33 R38 K40 A44 H68 Q70 N75 A105 R107 A151 G153 E158 (or MO_2) mutant. Five clones were found to trigger a better cleavage of the rosa1 target when mated with such yeast strain compared to the mutants before mutagenesis with the same yeast strain (Figure 10). After sequencing, they turned out to correspond to four proteins. In conclusion, four proteins able to efficiently cleave rosa1 when forming heterodimers with MO_1 or MO_2 were identified (Table VI).

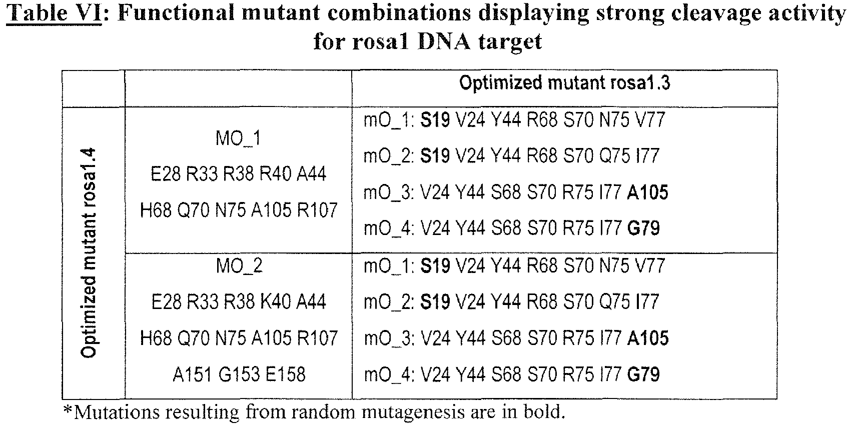

Those 4 new proteins were obtained by random mutagenesis on a pool of I*-Cre*I mutants cleaving in homodimers the rosa1.3 target (described in example 1 and Table I). Interestingly, they have each only one single amino-acid mutation (G19S, V105A or S79G) compared to initial I-*Cre*I mutants (Table VII). For example, mO_2 differs from m14 by a single G19S mutation. Similarly, the A105 mutation described in example 6, was found again in this example, but this time, not associated with any other mutation.

Those 3 amino-acid mutations increase the activity of heterodimers against the rosa1 target (Figure 10). This effect is especially significant with the G19S mutation. The improvement by the introduction of the G19S mutation is best illustrated by the comparison of activity against the rosa1 target of heterodimers formed by the co-expression in yeast of mutant m14 with MO_1 or MO_2 (Figure 9) and mutant mO_2 with MO_2 (Figure 10). Thus, the G19S mutation seems to be sufficient to increase activity by itself.

**Table VII: I-CreI mutants described in example 1 used for random mutagenesis and refined mutants described in example 5.**

| **Initials mutants described in example 1** | **Refined mutants described in example 5** |
|---|---|
| m2: VYRSNI | |
| m6:VYYSYR | |
| m8: VYRSNV | mO_1: VYRSNV + **G19S** |
| m12: VYNSRI | |
| m13: VYSSRI | mO_3: VYSSRI + **V105A** |
| | mO_4: VYSSRI + **S79G** |
| m14: VYRSQI | mO_2: VYRSQI + **G19S** |
| m17:VYRSYT | |

| | |
|---|---|
| The amino acids at positions 24, 44, 68, 70, 75 and 77 are indicated (ex: VYRSYI stands for V24, Y44, R68, S70, Y75 and 177. *Mutations resulting from random mutagenesis are in bold. | |

### Example 6: The G19S mutation, that enhances activity of heterodimers against the rosa1 target, abolishes activity of homodimers against the rosa1.3 palindromic target.

I*-Cre*I refined mutants able to efficiently cleave the rosa1 target when they are co-expressed in yeast to form heterodimers, were identified in example 5 (Figure 10). Example 5 shows that the G19S mutation added in one component of heterodimers enhances their activity on the rosa1 target (Table VI, Table VII, Figure 9 and Figure 10). This example shows that the G19S mutation abolishes the activity of homodimers against the palindromic rosa1.3 target.

### 1) Materials and methods

The experimental procedures are as described in example 1.

### 2) Results

The refined I-*Cre*I mutants described in example 5 expressed in yeast are now screened in homodimers against the palindromic rosa1.3 target (Figure 11). Mutants mO_3 and mO_4 bearing the mutations V105A or S79G are active in homodimers against the rosa1.3 target. In contrast, mutants mO_1 and mO_2 bearing the G19S mutation are inactive in homodimers against the rosa1.3 palindromic target. Since the VYRSNV and VYRSQI mutants, which efficiently cleave the rosa1.3 target (see example 1), differ from mO_1 and mO_2, respectively, only by a G at position 19, these results show that the G19S mutation is sufficient to significantly impair the activity of the homodimeric protein. These results contrast with the effect of the G19S position, when mO_1 and mO_2 form heterodimers with other proteins, which in this case, results in an increase of the activity (example 5). It is impossible by this step to determine whether this loss of activity results from a defect in homodimer formation, binding or cleavage. However, position 19, is part of the catalytic site (Chevalier et al., Biochemistry, 2004, 43, 14015-14026) and, with the adjacent Asp20, Gly19 is involved in metal cation binding, which suggest a direct impact on the cleavage step.

In addition to the efficacy issue, specificity is another important feature for many applications, and especially for therapeutic ones. Thus, the impact of the G19S mutation, which abolishes or strongly decreases the activity of a homodimer carrying this substitution, is to strongly improve specificity. Since this mutation also increases activity in heterodimers having only one such substitution, it would prove an invaluable tool to engineer meganucleases with better general properties, provided it confers this properties to any, or at least several I-CreI derived meganucleases, and not only to the ones listed in this example.

### Example 7: Making of meganucleases cleaving B2M11.2

This example shows that I*-Cre*I mutants can cut the B2M11.2 DNA target sequence derived from the left part of the B2M11 target in a palindromic form (Figure 12).

Target sequences described in this example are 22 bp palindromic sequences. Therefore, they will be described only by the first 11 nucleotides, followed by the suffix _P. For example, target B2M11.2 will be noted also tgaaattaggt_P (SEQ ID NO:25)

B2M11.2 is similar to 5TAG_P at positions ±1, ±2, ±3, ±4, ±5 and ±7 and to 10GAA_P at positions ±1, ±2, ±7, ±8, ±9 and ±10. It was hypothesized that positions ±6 and ±11 would have little effect on the binding and cleavage activity. Mutants able to cleave 5TAG_P target (caaaac**tag**gt_P; SEQ ID NO: 22) were previously obtained by mutagenesis on *I-CreI* N75 at positions 44, 68, 70, 75 and 77 (Arnould et al., J. Mol. Biol., 2006, 355, 443-458 and International PCT Applications WO 2006/097784 and WO 2006/097853). Mutants able to cleave the 10GAA_P target (c**gaa**acgtcgt _P; SEQ ID NO: 20) were obtained by mutagenesis on I*-Cre*I N75 and D75 at positions 28, 30, 32, 33, 38, 40, as described previously in Smith *et al,* Nucleic Acids Research, 2006, 34, e149). Thus,combining such pairs of mutants would allow for the cleavage of the B2M11.2 target.

Therefore, to check whether combined mutants could cleave the B2M11.2 target, mutations at positions 44, 68, 70, 75 and 77 from proteins cleaving 5TAG_P (caaaac**tag**gt_P; SEQ ID NO: 22) were combined with the 28, 30, 32, 33, 38 and 40 mutations from proteins cleaving 10GAA_P (c**gaa**acgtcgt_P; SEQ ID NO: 20).

### 1) Material and Methods

### a) Construction of target vector

The target was cloned as described in example 1, using the oligonucleotide: 5' tggcatacaagttttgttctcaggtacctgagaacaacaatcgtctgtca 3' (SEQ ID NO: 27), corresponding to the target sequence flanked by gateway cloning sequence, ordered from PROLIGO.

### b) Construction of combinatorial mutants

I-*Cre*I mutants cleaving 10GAA_P or 5TAG_P were identified in Smith et al, Nucleic Acids Res. 2006, 34, e149; International PCT Application WO 2007/049156, and Arnould et al., J. Mol. Biol., 2006, 355, 443-458; International PCT Applications WO 2006/097784 and WO 2006/097853, respectively for the 10GAA_P or 5TAG_P targets. I-*Cre*I derived coding sequence containing mutations from both series were generated by separate overlapping PCR reactions as described in example 2.

### c) Mating of meganuclease expressing clones and screening in yeast

The experimental procedure is as described in example 1.

### d) Sequencing of mutants

The experimental procedure is as described in example 1.

### 2) Results

I*-Cre*I combinatorial mutants were constructed by associating mutations at positions 44, 68, 70, 75 and 77 with the 28, 30, 33, 38 and 40 mutations on the I-CreI N75 or D75 scaffold, resulting in a library of a complexity of 2014. Examples of combinations are displayed on Table VIII. These libraries were transformed into yeast and 4464 clones (2.2 times the diversity) were screened for cleavage against B2M11.2 DNA target (t**gaa**at**tag**gt _P; SEQ ID NO: 25). Two positives clones were found with a very low level of activity, which after sequencing and validation by secondary screening turned out to correspond to two different novel endonucleases (see Table VIII). Positives are shown in Figure 13.

**Table VIII: Cleavage of the B2M11.2 target by the combinatorial variants**

| Amino acids at positions 44, 68, 70, 75 and 77 (AYSYK stands for A44, Y68, 570,Y75 and K77) | Amino acids at positions 28, 30, 32, 33, 38 and 40 (KNGHQS stands for K28, N30, G32, H33, Q38 and S40) | | | | | | | |
|---|---|---|---|---|---|---|---|---|
| | KNGHQS | KNAHQS | KNSHQS | KNAQQS | KYSHQS | KNPRQS | KPSHQS | KRSWQS |
| ARSYY | | | | | | | | |
| ANNNI | | | | | | | | |
| SRSYT | | | | | | | | |
| AYSYK | + | + | | | | | | |
| AQNNI | | | | | | | | |
| ARSYV | | | | | | | | |
| NRSYN | | | | | | | | |
| ARNNI | | | | | | | | |
| ARTNI | | | | | | | | |
| AKSYI | | | | | | | | |
| NYSYV | | | | | | | | |
| ARSYQ | | | | | | | | |
| SRSYS | | | | | | | | |
| NQSSV | | | | | | | | |
| NRSYS | | | | | | | | |
| AKSYR | | | | | | | | |
| TRSYI | | | | | | | | |
| TNSYK | | | | | | | | |
| ARSYN | | | | | | | | |
| SRSYI | | | | | | | | |
| ARSYI | | | | | | | | |
| TYSYK | | | | | | | | |
| NRSNI | | | | | | | | |
| NRSYI | | | | | | | | |
| ATNNI | | | | | | | | |

| | | | | | | | | |
|---|---|---|---|---|---|---|---|---|
| *Only 176 out of the 2014 combinations are displayed + indicates that the functional combinatorial mutant was found among the identified positives. | | | | | | | | |

### Example 8: Making of meganucleases cleaving B2M11.2 with higher efficacy by random mutagenesis of meganucleases cleaving B2M11.2.

I-*Cre*I mutants able to cleave the palindromic B2M11.2 target were identified by assembly of mutants cleaving the palindromic 10GAA_P and 5TAG_P target (example 7). However, only 2 of these combinations were able to cleave B2M11.2 and with a minimal efficiency.

Therefore, the 2 protein combinations cleaving B2M11.2, were randomly mutagenized and variants cleaving B2M11.2 with better efficiency were screened. According to the structure of the I*-Cre*I protein bound to its target, there is no contact between the residues used for the first combinatorial approach (28. 30, 32, 33, 38 and 40 vs 44, 68, 70, 75 and 77) in the I*-Cre*I protein (Chevalier, B. S. and B.L. Stoddard, Nucleic Acids Res. 2001, 29, 3757-3774; Chevalier et al., Nat. Struct. Biol. 2001, 8, 312-316, Chevalier et al., J. Mol. Biol., 2003, 329, 253-269). Thus, it is difficult to rationally choose a set of positions to mutagenize, and mutagenesis was done on the C-terminal part of the protein (83 last amino acids) or on the whole protein.

### 1) Material and Methods

The experimental procedures are as described in example 5.

### 2) Results

Two mutants cleaving B2M11.2 (I-*Cre*I 32G33H44A68Y70S75Y77K and I*-Cre*I 32A33H44A68Y70S75Y77K, also called KNGHQS/AYSYK and KNAHQS/AYSYK according to nomenclature of Table VIII) were pooled, randomly mutagenized and transformed into yeast. 4464 transformed clones were then mated with a yeast strain that contains the B2M11.2 target in a reporter plasmid. Thirty-two clones were found to trigger cleavage of the B2M11.2 target when mated with such yeast strain, corresponding at least to 14 different novel endonucleases (see Table IX). Example of positives is shown on Figure 14.

**Table IX: Optimized mutant towards the B2M11.2 target**

| Optimized Mutant B2M11.2* | B2M11.2 target |
|---|---|
| I-CreI 32A33H44A68Y70S75Y77K/**2Y53R** | |
| I-CreI 32A33H44A68Y70S75Y77K/**2Y53R66C** | + |
| I-CreI 32A33H44A68Y70S75Y77K/**132V** | + |
| I-CreI 32G33H44A68Y70S75Y77K/**2196R105A** | + |
| I-CreI 32G33H44A68Y70S75Y77K/**120G** | + |
| I-CreI 32A33H44A68Y70S75Y77K/**43L105A159R** | + |
| I-CreI 32G33H44A68Y70S75Y77K/**50R** | + |
| I-CreI 32G33H44A68Y70S75Y77K/**49A50R** | + |
| I-CreI 32G33H44A68Y70S75Y77K/**81V129A154G** | + |
| I-CreI 32G33H44A68Y70S75Y77K/**129A161P** | + |
| I-CreI 32G33H44A68Y70S75Y77K/**117G** | + |
| I-CreI 32G33H44A68Y70S75Y77K/**81T** | + |
| I-CreI 32G33H44A68Y70S75Y77K/**103T** | |

| | |
|---|---|
| + indicates cleavage of the B2M11.2 target *Mutations resulting from random mutagenesis are in bold. | |

### Example 9: Making of meganucleases cleaving B2M11.3

This example shows that I*-Cre*I mutants can cleave the B2M11.3 DNA target sequence derived from the right part of the B2M11.1 target in a palindromic form (Figure 12). All target sequences described in this example are 22 bp palindromic sequences. Therefore, they will be described only by the first 11 nucleotides, followed by the suffix _P. For example, B2M11.3 will be called tctgactttgt_P; SEQ ID NO:26).

B2M11.3 is similar to 5TTT_P at positions ±1, ±2, ±3, ±4, ±5, ±6 and ±7 and to 10CTG_P at positions ±1, ±2, ±6, ±7, ±8, ±9 and ±10. It was hypothesized that position ±11 would have little effect on the binding and cleavage activity. Mutants able to cleave 5TTT_P target (caaaac**ttt**gt_P; SEQ ID NO: 23) were previously obtained by mutagenesis on I*-Cre*I N75 at positions 44, 68, 70, 75 and 77 (Arnould et al., J. Mol. Biol. 2006, 355, 443-458 and International PCT Applications WO 2006/097784, WO 2006/097853). Mutants able to cleave the 10CTG_P target (c**ctg**acgtcgt_P; SEQ ID NO: 21) were obtained by mutagenesis on I*-Cre*I N75 and D75 at positions 28, 30, 32, 33, 38, 40 and 70, as described in Smith et al., Nucleic Acids Res., 2006, 34, e149. Thus, combining such pairs of mutants would allow for the cleavage of the B2M11.3 target.

Both sets of proteins are mutated at position 70. However, it was previously demonstrated that two separable functional domains exist in I*-Cre*I (Smith et al., Nucleic Acids Res., 2006, 34, e149). That implies that this position has little impact on the specificity towards the bases at position ± 10 to 8 of the target. Therefore, to check whether combined mutants could cleave the B2M11.3 target, mutations at positions 44, 68, 70, 75 and 77 from proteins cleaving 5TTT_P (caaaac**ttt**gt_P; SEQ ID NO:23 ) were combined with the 28, 30, 32, 33, 38, 40 mutations from proteins cleaving 10CTG_P (c**ctg**acgtcgt_P; SEQ ID NO: 21).

### 1) Material and Methods

### Construction of combinatorial mutants

I*-Cre*I mutants cleaving 10GAA_P or 5TAG_P were identified in Smith et al, Nucleic Acids Res., 2006, 34, e149; International PCT Application WO 2007/049156, and Arnould et al., J. Mol. Biol., 2006, 355, 443-458; International PCT Applications WO 2006/097784 and WO 2006/097853, respectively for the 10GAA_P or 5TAG_P targets. I-*Cre*I derived coding sequence containing mutations from both series were generated by separate overlapping PCR reactions as described in example 2, with the exception that cloning was performed in the yeast expression vector, pCLS1107 (Kan^{R}; Figure 7), linearized by digestion with *DraIII* and *NgoMIV.*

### 2) Results

I-*Cre*I combinatorial mutants were constructed by associating mutations at positions 44, 68, 70, 75and 77 with the 28, 30, 33, 38 and 40 mutations on the I*-Cre*I N75 or D75 scaffold, resulting in a library of complexity 1600. Examples of combinatorial mutants are displayed on Table X. This library was transformed into yeast and 3348 clones (2.1 times the diversity) were screened for cleavage against B2M11.3 DNA target (t**ctg**ac**ttt**gt_P; SEQ ID NO: 26). A single positive clone was found, which after sequencing and validation by secondary screening turned out to be correspond to a novel endonuclease (see Table X). Cleavage of the B2M11.3 target by this positive is shown in Figure 15.

**Table X: Cleavage of the B2M11.3 target by the combinatorial mutants***

| Amino acids at positions 44, 68, 70, 75 and 77 (KNANI stands for K44, N68, A70, N75 and I77) | Amino acids at positions 28, 30, 32, 33, 38 and 40 (KNSTQA stands for K28, N30, S32, T33, Q38 and A40) | | | | | | | | | |
|---|---|---|---|---|---|---|---|---|---|---|
| | KNSTQA | KQSGCS | KNSGQA | KNSSQP | KDSRGS | KSSNQS | KNTTQS | KNSGCS | KQSTQS | KCSGQS |
| KNANI | | | | | | | | | | |
| KRDNI | | | | | | | | | | |
| QNSNR | | | | | | | | | | |
| QRDNI | | | | | | | | | | |
| KGSNI | | | | | | | | | | |
| NHNNI | | | | | | | | | | |
| THHNI | | | | | | | | | | |
| KNSNI | | | | | | | | | | |
| QRRNI | | | | | | | | | | |
| QRSDK | | | | | | | | | | |
| KASNT | | | | | | | | | | |
| QESNR | | | | | | | | | | |
| TRSYI | | | | | | | | | | |
| TSSKN | | | | | | | | | | |
| QRSNT | | | | | | | | | | |
| TYSYR | | | | | | | | | | |
| QASDR | | | | | | | | | | |
| KYSNI | | | | | | | | | | |
| KYSNQ | | | | | | | | | | |
| TTSYR | | | | | | | | | | |
| KQSNT | | | | | | | | | | |
| QNSNR | | + | | | | | | | | |
| QSSNR | | | | | | | | | | |
| KYSDT | | | | | | | | | | |
| TYSYK | | | | | | | | | | |

| | | | | | | | | | | |
|---|---|---|---|---|---|---|---|---|---|---|
| * Only 240 out of the 1600 combinations are displayed. + indicates cleavage of the B2M11.3 target by the combinatorial mutant. | | | | | | | | | | |

### Example 10: Making of meganucleases cleaving B2M11 by coexpression of meganucleases cleaving B2M11.2 assembly with proteins cleaving B2M11.3

I*-Cre*I mutants able to cleave each of the palindromic B2M11 derived targets (B2M11.2 and B2M11.3) were identified in examples 7, 8 and 9. Pairs of such mutants (one cutting B2M11.2 and one cutting B2M11.3) were co-expressed in yeast. Upon coexpression, there should be three active molecular species, two homodimers, and one heterodimer. It was assayed whether the heterodimers that should be formed cut the B2M11 target.

### 1) Material and Methods

### a) Cloning of optimized mutants in pCLS0542, in B2M11 target veast

The yeast strain FYBL2-7B (MAT a, ura3Δ851, trp1Δ63, leu2Δ1, lys2Δ202) containing the B2M11 target into yeast reporter vector (pCLS1055, Figure 3) is transformed with optimised mutants cutting B2M11.2 target that were cloned in in the pCLS0542 vector, marked with the *LEU2* gene (Figure 4), using a high efficiency LiAc transformation protocol. Mutant-target yeasts are used as target for mating assays as described in examples 1 and 3 against the mutant cutting B2M11.3, in the pCLS 1107 vector marked by kanamycin (Figure 7).

### b) Mating of meganucleases coexpressing clones and screening in yeast

The experimental procedure is as described in example 3.

### 2) Results:

Coexpression of mutants cleaving the B2M11.2 and B2M11.3 resulted in the cleavage of the B2M11 target in most cases (Figure 16). Functional combinations are summarized in Table XI.

**Table XI: Combinations that resulted in cleavage of the B2M11 target**

| **Mutant B2M11.3** | **Optimized Mutant B2M11.2** | **B2M11 target cleavage** |
|---|---|---|
| | I-CreI 28K30N32A33H38Q40S44Ab8Y70S75Y77K/**2Y53R66C** | + |
| | I-CreI 28K30N32A33H38Q40S44A68Y70S75Y77K/**2Y53R** | + |
| | I-CreI 28K30N32A33H38Q40S 44A68Y70S75Y77K/**132V** | + |
| | I-CreI 28K30N32G33H38Q40S 44A68Y70S75Y77K/**2I96R105A** | + |
| | I-CreI 28K30N32G33H38Q40S 44A68Y70S75Y77K/**120G** | + |
| | I-CreI 28K30N32A33H38Q40S 44A68Y70575Y77K/**43L105A159R** | |
| I-*Cre*I K28Q30S32G33C38S40 Q44N68S70N75R77 (KQSGCS/QNSNR) | I-CreI 28K30N32G33H38Q40S 44A68Y70S75Y77K/**50R** | |
| | I-CreI 28K30N32033H38Q40S 44A68Y70S75Y77K/**49A50R** | |
| | I-CreI 28K30N32G33H38Q40S 44A68Y70S75Y77K/**81V129A154G** | |
| | I-CreI 28K30N32G33H38Q40S 44A68Y70S75Y77K/**129A161P** | |
| | I-CreI 28K30N32G331138Q40S 44A68Y7OS75Y77K/**117G** | |
| | I-CreI 28K30N32G33H38Q40S 44A68Y70S75Y77K/**81T** | |
| | I-CreI 28K30N32G33H38Q40S 44A68Y7OS75Y77K/**103T** | |

| | | |
|---|---|---|
| + indicates that the heterodimeric mutant is cleaving the B2M11 target * mutations resulting from random-mutagenesis are in bold | | |

### Example 11: Making of meganucleases cleaving B2M11 with higher efficacy by random mutagenesis of meganucleases cleaving B2M11.3 and co-expression with proteins cleaving B2M11.2

I-*Cre*I mutants able to cleave the palindromic B2M11 target were identified by co-expression of mutants cleaving the palindromic B2M11.2 and B2M11.3 targets (Examples 7, 8, 9 and 10). However, efficiency and number of positive combinations able to cleave B2M11 were minimal.

Therefore, the protein cleaving B2M11.3 was randomly mutagenized and variants cleaving B2M11 with better efficiency, when combined to optimized mutants for B2M 11.2, were screened. According to the structure of the I-*CreI* protein bound to its target, there is no contact between the residus used for the first combinatorial approach (28, 30, 32, 33, 38 and 40 vs 44, 68, 70, 75 and 77) in the I-*Cre*I protein (Chevalier, B. S. and B.L. Stoddard B L, Nucleic Acids Res., 2001, 29, 3757-3774; Chevalier et al., Nat. Struct. Biol. 2001, 8, 312-316; Chevalier et al., J. Mol. Biol. 2003, 329, 253-269). Thus, it is difficult to rationally choose a set of positions to mutagenize, and mutagenesis was done on the C-terminal part of the protein (83 last amino acids) or on the whole protein.

### 1) Material and Methods

Random mutagenesis is performed as decribed in example 4. Mutanttarget yeasts are prepared and used as target for mating assays as described in example 10.

### 2) Results

The mutant cleaving B2M11.3 (I-CreI 30Q33G38C68N70S75N77R also called KQSGCS/QNSNR according to nomenclature of Table XI) was randomly mutagenized and transformed into yeast. 6696 transformed clones were then mated with a yeast strain that (i) contains the B2M11 target in a reporter plasmid (ii) expresses a optimized variant cleaving the B2M11.2 target, chosen among those described in example 8. Two such strains were used, expressing either the I-CreI 32A33H44A68Y70S75Y77K/132V mutant, either the I-CreI 32G33H44A68Y70S75Y77K/2I96R105A mutant (see Table XII). One hundred and one clones were found to trigger cleavage of the B2M11 target when mated with such yeast strain. In a control experiment, none of these clones was found to trigger cleavage of B2M11 without coexpression of the KQSGCS/QNSNR protein. We concluded that 101 positives were containing proteins able to cleave B2M11 when forming heterodimers with KQSGCS/QNSNR. Examples of such heterodimeric mutants are listed in Table XII. Example of cleavage of the B2M11 target by such positives is shown on Figure 17.

**Table XII : Combinations that resulted in cleavage of B2M11 target**

| **Optimized Mutant B2M11.2*** | **Optimized Mutant B2M11.3*** | **B2M11 target cleavage** |
|---|---|---|
| | I-CreI 30Q33G38C68N70S77R/**19S72F** | + |
| | I-CreI 30Q33G38C68N70S77R/**31L83Q87L** | + |
| | I-CreI 30Q33G38C68N70S77R/**43L117G** | + |
| | I-CreI 30Q33G38C68N70S77R/**49A** | + |
| | I-CreI 30Q33G38C68N70S77R/**50R107R** | + |
| | I-CreI 30Q33G38C68N70S77R/**54L** | + |
| | I-CreI 30Q33G38C68N70S77R/**56E** | + |
| | I-CreI 30Q33G38C68N70S77R/**57N** | + |
| | I-CreI 30Q33G38C68N70S77R/**59A60E163L** | + |
| I-CreI 32G33H44A68Y70S75Y77K **/2I96R105A** | I-CreI 30Q33G38C68N70S77R/**60G100R155Q165T** | + |
| | I-CreI 30Q33G38C68N70S77R/**60N** | + |
| | I-CreI 30Q33G38C68N70S77R/**64A** | + |
| | I-CreI 30Q33G38C68N70S77R/**64D69G** | + |
| Or | I-CreI 30Q33G38C68N70S77R/**69E82E** | + |
| | I-CreI 30Q33G38C68N70S77R/**69G** | + |
| I-CreI 32A33H44A68Y70S75Y77K /**132V** | I-CreI 30Q33G38C68N70S77R/**72P154G** | + |
| | I-CreI 30Q33G38C68N70S77R/**73I** | + |
| | I-CreI 30Q33G38C68N70S77R/**73I156N** | + |
| | I-CreI 30Q33G38C68N70S77R/**103S** | + |
| Or | I-CreI 30Q33G38C68N70S77R/**103S147N** | + |
| | I-CreI 30Q33G38C68N70S77R/**105A** | + |
| I-CreI 32A33H44A68Y70S75Y77K /**2Y53R66C** | I-CreI 30Q33G38C68N70S77R/**110D** | + |
| | I-CreI 30Q33G38C68N70S77R/**110G153V** | + |
| | I-CreI 30Q33G38C68N70S77R/**111L** | + |
| | I-CreI 30Q33G38C68N70S77R/**142R161P** | + |
| Or | I-CreI 30Q33G38C68N70S77R/**153G** | + |
| | I-CreI 30Q33G38C68N70S77R/**153V** | + |
| I-CreI 32G33H44A68Y70S75Y77K /**120G** | I-CreI 30Q33G38C68N70S77R/**156N** | + |
| | I-CreI 30Q33G38C68N70S77R/**156R** | + |
| | I-CreI 30Q33G38C68N70S77R/**157V** | + |
| | I-CreI 30Q33G38C68N70S77R/**158N** | + |
| | I-CreI 30Q33G38C68N70S77R/**80G94Y** | + |
| | I-CreI 30Q33G38C68N70S77R/**81T83A117G** | + |
| | I-CreI 30Q33G38C68N70S77R/**81V159Q** | + |
| | I-CreI 30Q33G38C68N70S77R/**82E107R** | + |
| | I-CreI 30Q33G38C68N70S77R/**85R** | + |
| | I-CreI 30Q33G38C68N70S77R/**87L** | + |
| | I-CreI 30Q33G38C68N70S77R/**92L135P142R164G165P** | + |
| | I-CreI 30Q33G38C68N70S77R/**96R** | + |
| | I-CreI 30Q33G38C68Y70S77R/**72T140M** | + |
| | I-CreI 30Q33G**38S**68N70S77R | + |

| | | |
|---|---|---|
| + indicates that the heterodimeric variant is cleaving the B2M11 target. * mutations resulting from random-mutagenesis are in bold. | | |

Several different mutations were found in the improved mutants, among which the G19S and V105A mutations previously described. The V105A mutation was not associated with any other additional mutation, indicating that it is sufficient to improve the activity of the I-CreI 30Q33G38C68N70S77R protein. In example 5, it was already observed that the V105A mutation was sufficient to improve the activity of a completely different heterodimer cleaving the rosa1 target. Thus, the V105A mutation to behaves like a "portable" motif, able to enhance the activity of different I*-Cre*I derivatives by itself.

### Example 12: Making of meganucleases cleaving B2M11.2 with higher efficacy by random mutagenesis of meganucleases cleaving B2M11.3, co-expression with proteins cleaving B2M11. 2, and screening in CHO cells

I-*Cre*I mutants able to cleave the palindromic B2M11 target with a better efficiency were identified by co-expression, in yeast, of mutants- optimized or not- cleaving palindromic B2M11.2 and B2M11.3 targets (Example 11). However, it is very useful to have heterodimer which are functional in mammalian cells and efficiency and number of positive combinations able to cleave B2M11 in mammalian cell cells using an extrachromosomal assay could be different than in yeast cell.

Therefore, the best proteins cleaving B2M11.2 were mutagenized as in example 11, and variants cleaving B2M11with good efficiency when combined to optimized mutants for B2M11.3, were screened. According to the structure of the I-*Cre*I protein bound to its target, there is no contact between the residues used for the first combinatorial approach (28, 30, 32, 33, 38 and 40 vs 44, 68, 70, 75 and 77) in the *I-CreI* protein (Chevalier B. S. and B.L. Stoddard, Nucleic Acids Res. 2001, 29, 3757-74; Chevalier et al., Nat. Struct. Biol. 2001, 8, 312-316; Chevalier et al., J. Mol. Biol. 2003, 329, 253-269). Thus, it is difficult to rationally choose a set of positions to mutagenize, and mutagenesis was done on the C-terminal part of the protein (83 last amino acids) or on the whole protein.

### 1) Material and Methods

### a) Construction of libraries by random mutagenesis

Random mutagenesis libraries were created on pool of chosen mutants, by PCR using Mn²⁺ or derivatives of dNTPs as 8-oxo-dGTP and dPTP, in two-step PCR process as described in the protocol from JENA BIOSCIENCE GmbH in JBS dNTP-Mutageneis kit. Primers used are attB1-ICreIFor (5'-ggggacaagtttgtacaaaaaagcaggcttcgaaggagatagaaccatggccaataccaaatataacaaagagttcc-3'; SEQ ID NO: 28) and attB2-ICreIRev (5'-ggggaccactttgtacaagaaagctgggtttagtcggcc gccggggaggatttcttcttctcgc-3'; SEQ ID NO: 29). PCR products obtained were cloned *in vitro* in CHO Gateway expression vector pCDNA6.2 from INVITROGEN (pCLS1069, Figure 18). In parallel, chosen mutants used for libraries were cloned in same way in this vector. Cloned mutants and positives resulting clones of libraries were verified by sequencing (MILLEGEN).

### b) Construction of B2M11 target in a vector for CHO screen

The B2M11 target was amplified from yeast target vector (as described in example 1), by two steps PCR using primers : M1s (5'-aaaaagcaggctgattggcatacaagtt-3'; SEQ ID NO: 30) and M2s (5'-agaaagctgggtgattgacagacgattg-3'; SEQ ID NO: 31) followed by attB1adapbis (5'-ggggacaagtttgtacaaaaaagca-3'; SEQ ID NO: 32) and attB2adapbis (5'-ggggaccactttgtacaagaaagct-3'; SEQ ID NO: 33). Primers are from Proligo. Final PCR was cloned using the Gateway protocol (INVITROGEN) into CHO reporter vector (pCLS1058, Figure 19). Cloned target was verified by sequencing (MILLEGEN).

### c) Extrachromosomal assay in mammalian cells

CHO cells were transfected with Polyfect transfection reagent according to the supplier's protocol (QIAGEN). Per assay, 150 ng of target vector was cotransfected with 12.5 ng of each one of both mutants (12.5 ng of mutant cleaving palindromic B2M11.2 target and 12.5 ng of mutant cleaving palindromic B2M11.3 target). 72 hours after transfection, culture medium was removed and 150 µl of lysis/revelation buffer added for β-galactosidase liquid assay (1 liter of buffer containing: 100 ml of lysis buffer (Tris-HCl 10 mM pH 7.5, NaCl 150 mM, Triton X100 0.1 %, BSA 0.1 mg/ml, protease inhibitors), 10 ml of Mg 100 X buffer (MgCl₂ 100 mM, β-mercaptoethanol 35 %), 110 ml ONPG 8 mg/ml and 780 ml of sodium phosphate 0.1M pH 7.5). After incubation at 37°C, the optical density was measured at 420 nm. The entire process is performed on an automated Velocity11 BioCel platform.

### 2) Results

The optimized mutants cleaving B2M11.2 (I-*Cre*I 32G33H44A68Y70S75Y77K/120G, 32A33H44A68Y70S75Y77K/2Y53R66C, 32G33H44A68Y70S75Y77K/2I96R105A and 32A33H44A68Y70S75Y77K/132V as described into Table XII) were randomly mutagenized and transformed into Gateway vector (Figure 15). DNA plasmid of 1920 transformed clones were purified and then cotransfected with the CHO B2M11 target vector and an optimized variant cleaving the B2M11.3 target, chosen among those described in example 11. Sixty clones were found to trigger cleavage of the B2M11 target.

In a control experiment, none of these clones was found to trigger cleavage of B2M11 without cotransfection of an optimized variant cleaving the B2M11.3 target. It was thus concluded that 60 positives were containing proteins able to cleave B2M11 when forming heterodimers with optimized variant cleaving the B2M11.3 target. Examples of such heterodimeric mutants are listed in Table XIII, with the observed cleavage activity, as a measure of beta-galactosidase activity. Again, mutations G19S and V105A were found, alone (V105A) or in combination with other mutations (for G19S).

**Table XIII: Functional mutant combinations resulting in cleavage of the B2M11 target***

| | | Mutants cleaving B2M11.3 (Initial or Optimized) | | |
|---|---|---|---|---|
| | | 30Q33G38C68N 70S77R/ 43L115T117G | 30Q33G38C68N 70S77R/ 110D | 30Q33G38C68N 70S77R |
| Optimized Mutants cleaving B2M11.2 | 32A33H44A68Y70S75Y77K/132V | 0.56 | 0.43 | 0.23 |
| | 32A33H44A68Y70S75Y77K/19S120G | nd | 0.21 | 0.19 |
| | 32A33H44A68Y70S75Y77K/19S132V | 1.83 | 0.82 | 0.64 |
| | 32A33H44A68Y70S75Y77K/19S43L | 0.93 | 0.53 | 0.33 |
| | 32A33H44A68Y70S75Y77K/19S43L53R66C | 0.60 | 0.31 | 0.28 |
| | 32A33H44A68Y70S75Y77K/24F117G | 0.94 | 0.49 | 0.41 |
| | 32A33H44A68Y70S75Y77K/43L132V | 0.52 | 0.41 | 0.26 |
| | 32G33H44A68Y70S75Y77K/ | 0.16 | 0.31 | 0.17 |
| | 32G33H44A68Y70S75Y77K/105A | 0.73 | 0.43 | 0.33 |
| | 32G33H44A68Y70S75Y77K/105A128R | 0.33 | 0.14 | 0.17 |
| | 32G33H44A68Y70S75Y77K/105A162F | 0.48 | 0.14 | 0.15 |
| | 32G33H44A68Y70S75Y77K/117G 154G | 0.44 | 0.27 | 0.13 |
| | 32G33H44A68Y70S75Y77K/120G | 0.43 | 0.35 | 0.17 |
| | 32G33H44A68Y70S75Y77K/120G162F | 0.34 | 0.14 | 0.09 |
| | 32G33H44A68Y70S75Y77K/120G163Q | 0.42 | 0.22 | 0.13 |
| | 32G33H44A68Y70S75Y77K/19S43L105A | 0.59 | 0.49 | 0.29 |
| | 32G33H44A68Y70S75Y77K/19S96R105A | nd | 0.20 | 0.31 |
| | 32G33H44A68Y70S75Y77K/24F89A117G162F165 P | 0.92 | 0.51 | 0.38 |
| | 32G33H44A68Y70S75Y77K/2I96R105A | 0.18 | 0.34 | 0.20 |
| | 32G33H44A68Y70S75Y77K/4Q96R105A | 0.46 | 0.17 | 0.17 |
| | 32G33H44A68Y70S75Y77K/79G96R105A | 0.38 | 0.18 | 0.09 |
| | 32G33H44A68Y70S75Y77K/89A | 0.35 | 0.13 | 0.10 |
| | 32G33H44A68Y70S75Y77K/89I120G | 0.41 | 0.22 | 0.19 |
| | 32G33H44A68Y70S75Y77K/92R96R105A | 0.45 | 0.16 | 0.13 |
| | 32G33H44A68Y70S75Y77K/94L105A | 0.41 | 0.14 | 0.14 |
| | 32G33H44A68Y70S75Y77K/96R100Q105A161F | 0.44 | 0.16 | 0.15 |
| | 32G33H44A68Y70S75Y77K/96R105A | 0.75 | 0.40 | 0.36 |
| | 32G33H44A68Y70S75Y77K/96R105A117G | 0.32 | 0.13 | 0.14 |

| | | | | |
|---|---|---|---|---|
| * Values (absorbance unit) are average of experimental results of the extrachromosomal assay in CHO cells. | | | | |

### Example 13: Refinement of meganucleases cleaving the HprCH3 target site by site-directed mutagenesis resulting in the substitution of Glycine-19 with Serine (G19S)

To further validate the ability of the G19S substitution to increase the cleavage activity of *I-CreI* derived meganucleases, this mutation was incorporated into each of the two proteins of the heterodimer HprCH3.3 (KNSHQS/QRRDI/42A43L) / HprCH3.4 (KNTHQS/RYSNN/72T) that cleaves the 22 bp (non-palindromic) target sequence HprCH3 (tcgagatgtcatgaaagagatgga; SEQ ID NO:34 ). The HprCH3 target sequence is located in Exon 3 (positions 17 to 38) of the *Criteculus griseus* (Chinese Hamster) HPRT gene.

To evaluate the cleavage activity of the original and G19S derived mutants a chromosomal reporter system in CHO cells was used (Figure 20). In this system a single-copy LacZ gene driven by the CMV promoter is interrupted by the HprCH3 site and is thus non-functional. The transfection of the cell line with CHO expression plasmids for HprCH3.3 / HprCH3.4 and a LacZ repair plasmid allows the restoration of a functional LacZ gene by homologous recombination. It has previously been shown that double-strand breaks can induce homologous recombination; therefore the frequency with which the LacZ gene is repaired is indicative of the cleavage efficiency of the genomic HprCH3 target site.

### 1) Material and Methods

### a) Site-directed mutagenesis

To introduce the G19S substitution into the HprCH3.3 and HprCH3.4 coding sequences, two separate overlapping PCR reactions were carried out that amplify the 5' end (residues 1-24) or the 3' end (residues 14-167) of the I-*Cre*I coding sequence. For both the 5' and 3' end, PCR amplification is carried out using a primer with homology to the vector:
CCM2For 5'-aagcagagctctctggctaactagagaacccactgcttactggcttatcgaccatggccaatacca aatataacaaagagttcc-3' (SEQ ID NO: 35) or CCMRev 5'-tctgatcgattcaagtcagtgtctctctag atagcgagtcggccgccggggaggatttcttcttctcgc -3': SEQ ID NO: 36) and a primer specific to the I-*Cre*I coding sequence for amino acids 14-24 that contains the substitution mutation G19S : G19SF 5'-gccggctttgtggac**tct**gacggtagcatcatc-3' (SEQ ID NO:37) or G19SR 5'-gatgatgctaccgtc**aga**gtccacaaagccggc-3' (SEQ ID NO: 38).

The resulting PCR products contain 33 bp of homology with each other. Subsequently the fragments are assembled by PCR using an aliquot of each of the two fragments and the CCM2For and CCMRev primers.

### b) Cloning of mutants in a CHO expression vector

PCR products digested with *Soc*I-*Xba*I were cloned into the corresponding *Sac*I-*Xba*I sites of the plasmid pCLS1088 (Figure 21), a CHO gateway expression vector pCDNA6.2 (INVITROGEN) containing the I*-Cre*I N75 coding sequence. The substitution of the HprCH3.3-G19S or HprCH3.4-G19S coding sequence for the I*-Cre*I N75 coding sequence was verified by sequencing (MILLEGEN).

### c) Chromosomal assay in mammalian cells

CHO cell lines harbouring the reporter system were seeded at a density of 2x10⁵ cells per 10cm dish in complete medium (Kaighn's modified F-12 medium (F12-K), supplemented with 2 mM L-glutamine, penicillin (100 UI/ml), streptomycin (100 µg/ml), amphotericin B (Fongizone) (0.25 µg/ml) (INVITROGEN-LIFE SCIENCE) and 10% FBS (SIGMA-ALDRICH CHIMIE). The next day, cells were transfected with Polyfect transfection reagent (QIAGEN). Briefly, 2 µg of lacz repair matrix vector was co-transfected with various amounts of meganucleases expression vectors. After 72 hours of incubation at 37 °C, cells were fixed in 0.5 % glutaraldehyde at 4 °C for 10 min, washed twice in 100 mM phosphate buffer with 0.02 % NP40 and stained with the following staining buffer (10 mM Phosphate buffer, 1 mM MgCl₂, 33 mM K hexacyanoferrate (III), 33 mM K hexacyanoferrate (II), 0.1 % (v/v) X-Gal). After, an overnight incubation at 37 °C, plates were examined under a light microscope and the number of LacZ positive cell clones counted. The frequency of LacZ repair is expressed as the number of LacZ+ foci divided by the number of transfected cells (5x10⁵) and corrected by the transfection efficiency.

### 2) Results

The activities of heterodimers containing either the two initial mutants (HprCH3.3 / HprCH3.4) or one of the two G19S derived mutants combined with the corresponding initial mutant (HprCH3.3 / HprCh3.4 G19S or HprCH3.3 G19S / HprCh3.4) were tested using a chromosomal assay in a CHO cell line containing the HprCH3 target. This chromosomal assay has been extensively described in a recent publication (Arnoul et al., J. Mol. Biol. Epub May 10, 2007). Briefly, a CHO cell line carrying a single copy transgene was first created. The transgene contains a human EF1α promoter upstream an I-*Sce*I cleavage site (Figure 20, step 1). Second, the I-*Sce*I meganuclease was used to trigger DSB-induced homologous recombination at this locus, and insert a 5.5 kb cassette with a novel meganuclease cleavage site (Figure 20, step2). This cassette contains a non functional LacZ open reading frame driven by a CMV promoter, and a promoter-less hygromycin marker gene. The LacZ gene itself is inactivated by a 50 bp insertion containing the meganuclease cleavage site to be tested (here, the rosa1 cleavage site). This cell lines can in turn be used to evaluate DSB-induced gene targeting efficiencies (LacZ repair) with engineered I-*Cre*I derivatives cleaving the rosa1 target (Figure 20, step3).

This cell line was co-transfected with the repair matrix and various amounts of the vectors expressing the meganucleases. The frequency of repair of the LacZ gene increased from a maximum of 2.0 x10⁻³ with the initial engineered heterodimers (HprCH3.3 / HprCH3.4), to a maximum of 1.15 x 10⁻² with the HprCH3.3-G19S derived mutant and a maximum of 1.2 x 10⁻² with the HprCH3.4-G19S derived mutant (Figure 22).

These finding confirm that G19S mutation is able, by itself, to enhance the activity of a heterodimer, when found in only one of its monomers. These results confirm those obtained in example 5, wherein a single G19S substitution was shown to enhance the activity of a completely different heterodimer, cleaving the rosa1 target. In addition, G19S was found in several proteins with enhanced activity towards the B2M11 target, although in combination with other mutations (see examples 11 and 12). Thus, the G19S mutation behaves like a "portable" motif, able to enhance the activity of different I-CreI derivatives by itself, or in combinations with other mutations.

However, when the HprCH3.3 G19S / HprCH3.4 G19S heterodimer was transformed with the repair matrix, no LacZ+ foci were detected, indicating a recombination frequency of less than 6.0 x 10⁻⁶. These finding confirm those observed in example 6 with meganucleases cleaving the rosa1 target: whereas a single G19S substitution enhances the activity, a G19S substitution in each monomers of the heterodimer results in a very strong decrease of the activity.

### Example 14: Improvement of meganucleases cleaving the RAG1.10 DNA sequence by introduction of a single G19S substitution

Several optimization processes by random mutagenesis of I-*Cre*I mutants have shown that several improved mutants were carrying the same mutation G19S, which means the substitution at position 19 of the glycine by a serine residue. This mutation has a dual function because it not only improves the activity of the heterodimer but also abolishes the activity of the homodimer harboring the G19S mutation, hence enhancing the specificity of the I*-Cre*I derived meganucleases (see examples 5, 6, 11, 12 and 13).

Therefore, the G19S mutation was introduced into the KRSNQS/AYSDR mutant (noted M2 below) cleaving the RAG1.10.2 target and into the NNSSRR/YRSQV mutant (noted M3 below) cleaving the RAG1.10.3 target. These new proteins were then tested against the RAG1.10, RAG1.10.2 and RAG1.10.3 targets (Figure 23) in extrachromosomal and chromosomal assays in mammalian cells, as decribed in examples 12 and 13.

### 1) Material and Methods

### a) Introduction of the G19S mutation

Two overlapping PCR reactions were performed using two sets of primers: Gal10F (5'-gcaactttagtgctgacacatacagg-3'; SEQ ID NO: 12) and G19SRev (5'-gatgatgctaccgtcagagtccacaaagccggc-3'; SEQ ID NO: 38) for the first fragment and G19SFor (5'-gccggctttgtggactctgacggtagcatcatc-3'; SEQ ID NO: 37) and Gal10R (5'-acaacettgattggagacttgacc-3'; SEQ ID NO: 13) for the second fragment. Approximately 25 ng of each PCR fragment and 75 ng of vector DNA (pCLS0542) linearized by digestion with *Nco*I and *Eag*I were used to transform the yeast *Saccharomyces cerevisiae* strain FYC2-6A (MATα, trp1Δ63, leu2Δ1, his3Δ200) using a high efficiency LiAc transformation protocol (Gietz, R.D. and R.A. Woods; Methods Enzymol. 2002, 350, 87-96). An intact coding sequence containing the G19S mutation is generated by *in vivo* homologous recombination in yeast.

### b) Sequencing of the mutants

To recover the mutant expressing plasmids, yeast DNA was extracted using standard protocols and used to transform *E. coli.* Sequence of mutant ORF were then performed on the plasmids by MILLEGEN SA.

### c) Cloning of the RAG1.10 G19S mutants into a mammalian expression vector

Each mutant ORF was amplified by PCR using the primers CCM2For: 
(5'-aagcagagctctctggctaactagagaacccactgcttactggcttatcgaccatggccaataccaaatataacaaag
agttcc-3'; SEQ ID NO:39)
 and CCMRevBis:
(5'-ctgctctagattagtcggccgccggggaggatttcttc-3'; SEQ ID NO: 40).

The PCR fragment was digested by the restriction enzymes *SacI* and *XbaI,* and was then ligated into the vector pCLS1088 (Figure 21) digested also by *SacI* and *XbaI.* Resulting clones were verified by sequencing (MILLEGEN).

### d) Cloning of the different RAG1.10 targets in a vector for extrachromosomal assay

The target of interest was cloned as follows: oligonucleotide corresponding to the target sequence flanked by gateway cloning sequence was ordered from Proligo. Double-stranded target DNA, generated by PCR amplification of the single stranded oligonucleotide, was cloned using the Gateway protocol (INVITROGEN) into CHO reporter vector (pCLS1058, Figure 19).

### e) Extrachromosomal assay in CHO cells

See example 12

### f) Chromosomal assay in CHO cells

See example 13

### 2) Results

The activity of the M2 and M3 I-*Cre*I mutants harboring the G19S mutation (M2 G19S and M3 G19S) against their respective targets RAG1.10.2 and RAG1.10.3 (Figure 23) was monitored using the extrachromosomal assay in CHO cells. The mutants were tested either in a pure homodimeric way or in cotransfecting the mutants with and without the G19S mutation, which allowed the detection of the activity of both heterodimers M2/M2 G19S and M3/M3 G19S against their respective RAG1.10.2 and RAG1.10.3 targets (Figure 24A). Then the different heterodimers M2/M3, M2 G19S/M3 and M2/M3 G19S were tested against the RAG1.10 target (Figure 24B). As can be seen in Figures 23A and 23B, both aspects of the G19S mutation are observed. Firstly, this mutation abolishes the activity of the homodimers (M2 G19S and M3 G19S) against their palindromic targets. Secondly, introduction of the G19S mutation in the M3 mutant greatly increases the activity of the RAG1.10.3 target cleavage by the M3/M3 G19S heterodimer. This effect can not be really evidenced for the M2 mutant because it already cleaves the RAG1.10.2 target at saturating levels in this assay. The same remark can be made for the RAG1.10 target, which is cleaved at saturating levels by the M2/M3 heterodimer as well as the M2 G19S/M3 and M2/M3 G19S heterodimers.

These three last heterodimers were then tested in a chromosomal assay in CHO cells. The system used is described in example 13, and in Figure 20, but in this example, the RAG1.10 cleavage site was introduced in the reporter strain instead of rosa1 and 0.1 µg of each vector expressing each meganuclease was used to transfect the cells. Table XIV summarizes the experimental results. A more than two fold increase of the frequency of gene targeting was observed when the G19S was introduced in one of the two monomers (M2 or M3).

**Table XIV: Frequency of LacZ repair in function of the nature of the heterodimer used to transfect the CHO cell line carrying the single copy of the cassette CMV-LacZ, in which the LacZ gene has been interrupted by the RAG1.10 DNA sequence.**

| **Heterodimer** | **Frequency of LacZ repair** |
|---|---|
| M2/M3 | 2.4x10⁻³ |
| M2 G19S/M3 | 5.8x10⁻³ |
| M2/M3 G19S | 5.2x10⁻³ |
| M2 G19S/M3 G19S | 0 |

These results confirm the data described in examples 5, 6, 11, 12 and 13, showing that the G19S mutation is a portable motif, able to increase the activity of several, totally different heterodimeric I-*Cre*I engineered derivatives. Furthermore, they also confirm that this mutation abolishes the formation of functional homodimers, as already observed in examples 6 and 13 with different meganucleases. This effect is solely due to the presence of a G19S mutation in each monomer, since the M3/M3 G19S is not only functional, but even more active than the M3/M3 homodimer. Altogether, the various examples shown here define the G19S as a portable motif able to increase the activity and specificity of I-*Cre*I derived engineered meganucleases.

### Example 15: Making of RAG1.10 obligatory heterodimers in introducing the K7E, E8K and G19S mutations.

Examples 6, 13 and 14 show that the G19S mutation not only increases the heterodimeric activity but also abolishes almost completely the activity of G19S homodimers. It is therefore a tool of choice to use in the obligatory heterodimer design. Taking the two RAG1.10 heterodimers described above in example 14 (M2 and M3), the K7E mutation was introduced in the M2 G19S and M3 G19S variants and the E8K mutation was introduced in the M2 and M3 variants. Activity of the two heterodimers M2 K7E,G19S / M3 E8K and M2 E8K / M3 K7E,G19S was then monitored against the three RAG1.10 targets using the CHO extrachromosomal assay described in example 12.

### 1) Material and Methods

### a) Introduction of the K7E and E8K mutations

The *I-CreI* derived mutants M2 and M3 were already cloned in the pCLS1088 mammalian expression vector (figure 21). Each mutation was introduced using two overlapping PCR reactions carried out on the DNA of the M2 and M3 mutants. For the K7E mutation, the first PCR reaction was done with the primers CMVFor (5'-cgcaaatgggcggtaggcgtg-3'; SEQ ID NO: 53) and K7ERev (5'-gtacagcaggaactcttcgttatatttggtattgg-3'; SEQ ID NO: 54) and the second reaction with the primers K7EFor (5'-aataccaaatataacgaagagttcctgctgtacc-3'; SEQ ID NO: 55) and V5epitopeRev (5'-cgtagaatcgagaccgaggagagg-3'; SEQ ID NO: 56). The two PCR fragments were gel purified, mixed and a third assembly PCR was conducted using the CMVFor and V5epitopeRev primers. The obtained PCR fragment contains the open reading frame of the I-*Cre*I mutant with the K7E mutation. The PCR fragment was then purified, digested with the restriction enzymes *Sac*I and *Xba*I and ligated into the pCLS1088 (Figure 21) also digested by *Sac*I and *Xba*I*.* The resulting clones M2 K7E or M3 K7E were verified by sequencing (MILLEGEN).

Introduction of the E8K mutation in the M2 and M3 mutants was carried out using absolutely the same protocol but the two primers E8KRev (5'-caggtacagcaggaactttttgttatatttgg-3'; SEQ ID NO: 57) and E8KFor (5'-accaaatataacaaaaagttcctgctgtacctgg-3'; SEQ ID NO: 58).

### b) Introduction of the G19S mutation

The G19S mutation was introduced using two overlapping PCR reactions carried out on the mutant DNA. The first PCR reaction was done with the primers CMVFor (5'-cgcaaatgggcggtaggcgtg-3'; SEQ ID NO: 53) and G19SRev (5'-gatgatgctaccgtcagagtccacaaagccggc-3'; SEQ ID NO: 59) and the second reaction with the primers G19SFor (5'-gccggctttgtggactctgacggtagcatcatc -3'; SEQ ID NO: 60) and V5epitopeRev (5'-cgtagaatcgagaccgaggagagg-3'; SEQ ID NO: 56). The two PCR fragments were gel purified, mixed and a third assembly PCR was conducted using the CMVFor and V5epitopeRev primers. The obtained PCR fragment contains the open reading frame of the I-*Cre*I mutant with the G19S mutation. The PCR fragment was then purified, digested with the restriction enzymes *Sac*I and XbaI and ligated into the pCLS1088 also digested by SacI and XbaI*.* The resulting clones M2 K7E, G19S and M3 K7E,G19S were verified by sequencing (MILLEGEN).

### 2) Results

Activity of the two heterodimers M2 K7E,G19S / M3 E8K (Het1) and M2 E8K / M3 K7E,G19S (Het2) against the three RAG1.10 targets was compared to that of the initial M2 / M3 heterodimer in CHO cells using the extrachromosomal assay previously described (example 12). The figure 25 shows that the two heterodimers Het1 and Het2 behave as obligatory heterodimers, because homodimeric activities have been decreased to almost non detectable levels. In addition, Het1 and Het2 have the same activity level toward the RAG1.10 target as the initial M2 / M3 heterodimer. The K7E/E8K and G19S mutations can therefore be used to strongly favour the formation of functional heterodimers versus functional homodimers.

### Example 16: Use of the K7E, E8K and G19S mutations in the single chain molecule design

To further validate the use of the G19S and K7E/E8K mutations to improve a meganuclease specificity, these mutations were introduced in a a single chain molecule able to cleave the C1 target (cgagatgtcacacagaggtacg; SEQ ID NO: 61), a DNA sequence found in the human *Xeroderma Pigmentosum* (XPC) gene. The engineering of I-*Cre*I derived mutants able to cleave the C1 target has been described previously (Arnould et al., J. Mol. Biol., Epub 10 May 2007; International PCT Application WO 2007/093836 and WO 2007/093918). The single chain construct is X2-L1-H33, where X2 is a I-*Cre*I Y33H, Q38A, S40Q, Q44K, R68Q, R70S and D75N derived mutant able to cleave the palindromic C4 target, H33 is the I-*Cre*I Y33H mutant, which cleaves the palindromic C3 target and Ll is a 22 amino acids linker (-AA(GGGGS)₄-; SEQ ID NO: 68), which connects the X2 mutant C-terminus to the H33 mutant N-terminus. The K7E/E8K and G19S mutations were introduced in the XPC single chain molecule to create two new single chain molecules SCX1 and SCX2, which are respectively X2(K7E)-L1-H33(E8K,G19S) and X2(E8K)-L1-H33(K7E,G19S). Activity of the three single chain molecules against the three XPC targets was then monitored using the previously described yeast screening assay.

### 1) Material and Methods

### a) Introduction of the K7E, E8K and G19S mutations in the XPC X2-L1-H33 Single Chain molecule

First, the G19S mutation was introduced in the X2-L1-H33 molecule. Two overlapping PCR reactions were performed on the single chain molecule cloned in the pCLS0542 yeast expression vector. The first PCR reaction uses the primers: Gall0F (5'-gcaactttagtgctgacacatacagg-3'; SEQ ID NO: 12) and G19SRev60 (5'-gcaatgatggagccatcagaatccacaaatccagc-3'; SEQ ID NO: 62) and the second PCR reaction, the primers G19SFor60 (5'-gctggatttgtggattctgatggctccatcattgc-3'; SEQ ID NO: 63) and Gall0R (5'-acaaccttgattggagacttgacc-3'; SEQ ID NO: 13). Approximately 25 ng of each PCR fragment and 75 ng of vector DNA (pCLS0542) linearized by digestion with *Nco*I and *Eag*I were used to transform the yeast *Saccharomyces cerevisiae* strain FYC2-6A (MATα, trp1Δ63, leu2Δ1, his3Δ200) using a high efficiency LiAc transformation protocol (Gietz R D and Woods R A Transformation of yeast by lithium acetate/single-stranded carrier DNA/polyethylene glycol method. Methods Enzymol. 2002; 350:87-96). An intact coding sequence containing the G19S mutation is generated by *in vivo* homologous recombination in yeast.

In a second step, the K7E and E8K mutations were introduced in the X2-L2-H33(G19S) molecule by performing three overlapping mutations. For the SCX1 molecule, the 3 PCR reactions use three primers set, which are respectively: Gall0F and K7ERev (5'-gtacagcaggaactcttcgttatatttggtattgg-3'; SEQ ID NO: 54), K7EFor (5'-aataccaaatataacgaagagttcctgctgtacc-3'; SEQ ID NO: 55) and E8KRevSC (5'-aagatacagcaggaactttttgttagagccacc-3'; SEQ ID NO: 64), E8KForSc (5'-ggtggctctaacaaaaagttcctgctgtatctt-3'; SEQ ID NO: 65) and Gall0R. For the SCX2 molecule, the 3 PCR reactions use three primers set, which are respectively: Gal10F and E8KRev (5'-caggtacagcaggaactttttgttatatttgg-3'; SEQ ID NO: 57), E8KFor (5'-accaaatataacaaaaagttcctgctgtacctgg-3'; SEQ ID NO: 58) and K7ERevSC (5'-aagatacagcaggaactcttcgttagagccacc-3' SEQ ID NO: 66), K7EForSc (5'-ggtggctctaacgaagagttcctgctgtatctt-3'; SEQ ID NO: 67) and Gall0R. For both constructs, approximately 25ng of each PCR fragment and 75ng of vector DNA (pCLS0542) linearized by digestion with *Nco*I and *Eag*I were used to transform the yeast *Saccharomyces cerevisiae* strain FYC2-6A (MATα, trp1Δ63, leu2Δ1, his3Δ200) using a high efficiency LiAc transformation protocol (Gietz R D and Woods R A Transformation of yeast by lithium acetate/single-stranded carrier DNA/polyethylene glycol method. Methods Enzymol. 2002; 350:87-96). An intact coding sequence for the SCX1 or SCX2 constructs is generated by *in vivo* homologous recombination in yeast.

### b) Mating of meganucleases coexpressing clones and screening in yeast

Mating was performed using a colony gridder (QpixII, GENETIX). Mutants were gridded on nylon filters covering YPD plates, using a low gridding density (about 4 spots/cm²). A second gridding process was performed on the same filters to spot a second layer consisting of different reporter-harbouring yeast strains for each target. Membranes were placed on solid agar YPD rich medium, and incubated at 30 °C for one night, to allow mating. Next, filters were transferred to synthetic medium, lacking leucine and tryptophan, adding G418, with galactose (1 %) as a carbon source, and incubated for five days at 37 °C, to select for diploids carrying the expression and target vectors. After 5 days, filters were placed on solid agarose medium with 0.02 % X-Gal in 0.5 M sodium phosphate buffer, pH 7.0, 0.1 % SDS, 6 % dimethyl formamide (DMF), 7 mM β-mercaptoethanol, 1 % agarose, and incubated at 37 °C, to monitor β-galactosidase activity. Results were analyzed by scanning and quantification was performed using appropriate software.

### 2) Results

Figure 26 shows the activity of the three single chain molecules X2-L1-H33, SCX1 and SCX2 against the three XPC targets in a yeast screening assay. The initial single chain molecule has a strong cleavage activity against the C1 and C3 target but introduction of the K7E/E8K and G19S mutations to generate the SCX1 and SCX2 molecules promotes an increased cleavage activity toward the C1 target and a complete abolition of the cleavage activity toward the C3 target. Thus, the mutations K7E/E8K and G19S that were described in example 15 in the obligatory heterodimer design can also be successfully introduced in a single chain molecule to improve its specificity without affecting its cleavage activity toward the DNA target of interest.

### Example 17: Improvement of the meganucleases cleaving the HBB5 DNA sequence by introduction of the V105A and I132V mutations, alone or in combination.

The HBB5 DNA sequence (5'-ttggtctccttaaacctgtcttga-3'; SEQ ID NO: 69) belongs to the HBB gene, which codes for the haemoglobin β-chain. The HBB5 DNA sequence is located at the beginning of the HBB gene intron 1. The HBB5 DNA target was divided into two half-palindromic 24bp DNA targets that were called HBB5.3 (5'-ttggtctcctgtacaggagaccaa-3'; SEQ ID NO: 70) and HBB5.4 (5'-tcaagacagggtaccctgtcttga-3'; SEQ ID NO: 71). By a semi-rational combinatorial process, which has been thoroughly described in examples 1 and 2, we were able to obtain an I-*Cre*I derived mutant able to cleave the HBB5.3 target. This mutant called H3 has the following mutations in comparison to the I-*Cre*I wild-type sequence: 30S33H38R44K68Y70S77T. Using the same process followed with a mutant optimization led by random mutagenesis as shown in example 4, two I-*Cre*I derived mutants were obtained that are able to cleave the HBB5.4 target. The two mutants called H4a and H4b have respectively the following mutations in comparison to the I-*Cre*I wild-type sequence: 19S32T44A70S75E77R80K96R and 24V43L44A70S75E77R80K87L96R. Yeast coexpression of mutant H3 with either H4a or H4b led then to the cleavage of the HBB5 DNA sequence by the heterodimeric meganuclease.

To assess the influence of multiple optimization mutations on the activity, the mutations V105A and I132V were introduced on the H3 mutant, either alone or in combination. The obtained mutants were then tested with the two H4a and H4b partners in the yeast recombination assay on the HBB5.1 target.

### 1) Material and Methods

### a) Introduction the V105A mutation

Two overlapping PCR reactions were performed on the DNA coding for the H3 mutant using two sets of primers: Gall0F (5'-gcaactttagtgctgacacatacagg-3'; SEQ ID NO: 12) and V105ARev (5'-ttcgataattttcagagccaggtttgcctgttt-3'; SEQ ID NO: 72) for the first fragment and V105AFor (5'- aaacaggcaaacctggctctgaaaattatcgaa-3'; SEQ ID NO: 73) and Gal10R (5'-acaacettgattggagacttgacc-3'; SEQ ID NO: 13) for the second fragment. Approximately 25 ng of each PCR fragment and 75 ng of vector DNA (pCLS0542) linearized by digestion with *Nco*I and *Eag*I were used to transform the yeast *Saccharomyces cerevisiae* strain FYC2-6A (MATα, trp1Δ63, leu2Δ1, his3Δ200) using a high efficiency LiAc transformation protocol (Gietz, R.D. and R.A. Woods; Methods Enzymol. 2002, 350, 87-96). An intact coding sequence containing the V105A mutation is generated by *in vivo* homologous recombination in yeast.

### b) Introduction the I132V mutation

Two overlapping PCR reactions were performed on the DNA coding for the H3 mutant using two sets of primers: Gal10F (5'-gcaactttagtgctgacacatacagg-3'; SEQ ID NO: 12) and I132VRev (5'-atcgttcagagctgcaacctgatccacccaggt-3'; SEQ ID NO: 74) for the first fragment and I132VFor (5'-acctgggtggatcaggttgcagctctgaacgat-3'; SEQ ID NO: 75) and Gal10R (5'-acaaccttgattggagacttgacc-3'; SEQ ID NO: 13) for the second fragment. Approximately 25 ng of each PCR fragment and 75 ng of vector DNA (pCLS0542) linearized by digestion with *Nco*I and *Eag*I were used to transform the yeast *Saccharomyces cerevisiae* strain FYC2-6A (MATα, trp1Δ63, leu2Δ1, his3Δ200) using a high efficiency LiAc transformation protocol (Gietz, R.D. and R.A. Woods; Methods Enzymol. 2002, 350, 87-96). An intact coding sequence containing the I132V mutation is generated by *in vivo* homologous recombination in yeast. The double mutant H3 V105 I132V was obtained by doing these same PCR reactions on the DNA coding for the H3 mutant carrying the V105A mutation.

### c) Sequencing of the mutants

To recover the mutant expressing plasmids, yeast DNA was extracted using standard protocols and used to transform *E*. *coli.* Sequence of mutant ORF were then performed on the plasmids by MILLEGEN SA.

### 2) Results

The three H3 derived mutants (H3 V105A, H3 I132V and H3 V105A I132V) were coexpressed in the yeast with either H4a or H4b and cleavage of the HBB5 target was monitored using the yeast recombination assay that has been already described in the previous examples (see example 3). Values indicated in Table XV are directly correlated to the β-galactodisase activity and hence to the cleavage activity of the heterodimeric HBB5 meganuclease.

**Table XV: Yeast cleavage of the HBB5 target by a heterodimeric meganuclease, where one partner carries the V105A and I132V mutations, alone or in combination.**

| **HBB5.3 Mutants** | **HBB5.4 Mutants** | **Value** |
|---|---|---|
| H3 | H4a | 0.34 |
| H3 V105A | H4a | 0.47 |
| H3 I132V | H4a | 0.49 |
| H3 V105A I132V | H4a | 0.77 |
| H3 | H4b | 0.29 |
| H3 V105A | H4b | 0.37 |
| H3 I132V | H4b | 0.37 |
| H3 V105A I132V | H4b | 0.56 |

In both cases, either with H4a or H4b, mutations V105A or I132V introduced on the H3 mutant increase the cleavage activity toward the HBB5 target. Activity is further increased when the two mutations are introduced simultaneously, showing that they can act independently of each other.

## Claims

1. A method for enhancing the cleavage activity of a functional I-*Cre*I meganuclease variant or a single-chain meganuclease derivative, **characterized in that** it comprises the site-specific mutation of at least the valine at position 105 (V105) and/or the isoleucine at position 132 (I132) of I*-Cre*I.

2. The method of claim 1, **characterized in that** the valine at position 105 is changed to alanine (V105A), and/or the isoleucine at position 132 is changed to valine (I132V).

3. The method of claim 1 or 2, **characterized in that** the V105 and I132 residues are mutated in the same monomer of the I-*Cre*I meganuclease variant or single-chain derivative.

4. The method of any one of claims 1 to 3, **characterized in that** both monomers of the I-*Cre*I meganuclease variant or single-chain derivative are mutated.

5. The method of claim 4, **characterized in that** one monomer has the V105A and/or I132V mutation and the other monomer has the G19S or F87L mutation.

6. An I-*Cre*I meganuclease variant, **characterized in that** it is obtainable by the method of any one of claims 1 to 5 and it comprises at least one mutation selected from the group consisting of V105A and I132V, with the exclusion of the I-*Cre*I variants selected in the group consisting of:
- I-*Cre*I Y33R, S40Q, Q44A, R70H, D75N, F87L, I132T, V151A,
- I-*Cre*I Y33H, F54L, N86D, K100R, L104M, V105A, N136S, K159R,
- I-*Cre*I S32T, Y33H, Q44K, R68Y, R70S, I77R, Q92R, K96R, K107R, I132V, T140A, T143A,
- I-*Cre*I S32A, Y33H, Q44A, R68Y, R70S, D75Y, I77K, I132V,
- I-*Cre*I N2I, S32G, Y33H, Q44A, R68Y, R70S, D75Y, I77K, K96R, V105A,
- I*-Cre*I S32A, Y33H, F43L, Q44A, R68Y, R70S, D75Y, I77K, V105A, K159R, and
- I*-Cre*I N30Q, Y33G, Q38C, R68N, R70S, I77R, V105A.

7. The I-*Cre*I meganuclease variant of claim 6, **characterized in that** it is a heterodimeric meganuclease wherein each monomer comprises different mutations at positions 26 to 40 and/or 44 to 77 of I-*Cre*I, and said meganuclease being able to cleave a non-palindromic genomic DNA target sequence of interest.

8. The I-*Cre*I meganuclease variant of claim 6 or claim 7, **characterized in that** it is a heterodimeric meganuclease wherein one monomer comprises the K7E mutation and the other monomer comprises the E8K mutation.

9. A single-chain meganuclease, **characterized in that** it comprises the first and the second monomer of an I-*Cre*I meganuclease variant according to any one of claims 6 to 8, connected by a peptidic linker.

10. A recombinant vector, **characterized in that** it comprises at least one polynucleotide fragment encoding the meganuclease variant of any one of claims 6 to 8 or the single-chain meganuclease of claim 9.

11. A host cell, **characterized in that** it is modified by a vector according to claim 10.

12. A pharmaceutical composition, **characterized in that** it comprises at least one meganuclease of any one of claims 6 to 9, or one vector of claim 10.


SEQUENCE LISTING

<110>  CELLECTIS

<120>  METHOD FOR ENHANCING THE CLEAVAGE ACTIVITY OF I-CREI DERIVED
       MEGANUCLEASES

<130>  HLPcp1546/18

<160>  75

<170>  PatentIn version 3.3

<210>  1
<211>  163
<212>  PRT
<213>  Chlamydomonas reinhardtii

<400>  1

Met Asn Thr Lys Tyr Asn Lys Glu Phe Leu Leu Tyr Leu Ala Gly Phe
1               5                   10                  15

Val Asp Gly Asp Gly Ser Ile Ile Ala Gln Ile Lys Pro Asn Gln Ser
            20                  25                  30

Tyr Lys Phe Lys His Gln Leu Ser Leu Ala Phe Gln Val Thr Gln Lys
        35                  40                  45

Thr Gln Arg Arg Trp Phe Leu Asp Lys Leu Val Asp Glu Ile Gly Val
    50                  55                  60

Gly Tyr Val Arg Asp Arg Gly Ser Val Ser Asp Tyr Ile Leu Ser Glu
65                  70                  75                  80

Ile Lys Pro Leu His Asn Phe Leu Thr Gln Leu Gln Pro Phe Leu Lys
                85                  90                  95

Leu Lys Gln Lys Gln Ala Asn Leu Val Leu Lys Ile Ile Trp Arg Leu
            100                 105                 110

Pro Ser Ala Lys Glu Ser Pro Asp Lys Phe Leu Glu Val Cys Thr Trp
        115                 120                 125

Val Asp Gln Ile Ala Ala Leu Asn Asp Ser Lys Thr Arg Lys Thr Thr
    130                 135                 140

Ser Glu Thr Val Arg Ala Val Leu Asp Ser Leu Ser Glu Lys Lys Lys
145                 150                 155                 160

Ser Ser Pro

<210>  2
<211>  24
<212>  DNA
<213>  Artificial

<220>
<223>  I-CreI C1221 DNA target

<400>  2
tcaaaacgtc gtacgacgtt ttga                                              24


<210>  3
<211>  167
<212>  PRT
<213>  Artificial

<220>
<223>  I-CreI N75 scaffold

<400>  3

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Tyr Lys Phe Lys His Gln Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Arg Gly Ser Val Ser Asn Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  4
<211>  24
<212>  DNA
<213>  Artificial

<220>
<223>  10GGG_P DNA target

<400>  4
tcgggacgtc gtacgacgtc ccga                                              24


<210>  5
<211>  24
<212>  DNA
<213>  Artificial

<220>
<223>  5GAT_P DNA target

<400>  5
tcaaaacgat gtacatcgtt ttga                                              24


<210>  6
<211>  24
<212>  DNA
<213>  Artificial

<220>
<223>  5TAT_P DNA target

<400>  6
tcaaaactat gtacatagtt ttga                                              24


<210>  7
<211>  22
<212>  DNA
<213>  Artificial

<220>
<223>  rosal DNA target

<400>  7
caacatgatg ttcataatcc ca                                                22


<210>  8
<211>  22
<212>  DNA
<213>  Artificial

<220>
<223>  rosa1.2 DNA target

<400>  8
caacatgatg tacataatcc ca                                                22

<210>  9
<211>  22
<212>  DNA
<213>  Artificial

<220>
<223>  rosa1.3 DNA target

<400>  9
caacatgatg tacatcatgt tg                                                22


<210>  10
<211>  22
<212>  DNA
<213>  Artificial

<220>
<223>  rosa1.4 DNA target

<400>  10
tgggattatg tacataatcc ca                                                22


<210>  11
<211>  50
<212>  DNA
<213>  Artificial

<220>
<223>  oligonucleotide

<400>  11
tggcatacaa gtttcaacat gatgtacatc atgttgacaa tcgtctgtca                  50


<210>  12
<211>  26
<212>  DNA
<213>  Artificial

<220>
<223>  Gal10F primer

<400>  12
gcaactttag tgctgacaca tacagg                                            26


<210>  13
<211>  24
<212>  DNA
<213>  Artificial

<220>
<223>  Gal10R primer

<400>  13
acaaccttga ttggagactt gacc                                              24


<210>  14
<211>  15

<212>  DNA
<213>  Artificial

<220>
<223>  AssF primer


<220>
<221>  misc_feature
<222>  (4)..(6)
<223>  n is a, c, g, or t

<400>  14
ctannnttga ccttt                                                        15


<210>  15
<211>  15
<212>  DNA
<213>  Artificial

<220>
<223>  AssR primer


<220>
<221>  misc_feature
<222>  (10)..(12)
<223>  n is a, c, g, or t

<400>  15
aaaggtcaan nntag                                                        15


<210>  16
<211>  59
<212>  DNA
<213>  Artificial

<220>
<223>  preATGCreFor primer

<400>  16
gcataaatta ctatacttct atagacacgc aaacacaaat acacagcggc cttgccacc      59


<210>  17
<211>  48
<212>  DNA
<213>  Artificial

<220>
<223>  ICreIpostRev primer

<400>  17
ggctcgagga gctcgtctag aggatcgctc gagttatcag tcggccgc                  48


<210>  18
<211>  18
<212>  DNA
<213>  Artificial

<220>
<223>  AA78a83For primer

<400>  18
ttaagcgaaa tcaagccg                                                    18


<210>  19
<211>  18
<212>  DNA
<213>  Artificial

<220>
<223>  AA78a83Rev

<400>  19
cggcttgatt tcgcttaa                                                    18


<210>  20
<211>  24
<212>  DNA
<213>  Artificial

<220>
<223>  10GAA_P DNA target

<400>  20
tcgaaacgtc gtacgacgtt tcga                                             24


<210>  21
<211>  24
<212>  DNA
<213>  Artificial

<220>
<223>  10CTG_P DNA target

<400>  21
tcctgacgtc gtacgacgtc agga                                             24


<210>  22
<211>  24
<212>  DNA
<213>  Artificial

<220>
<223>  5TAG_P DNA target

<400>  22
tcaaaactag gtacctagtt ttga                                             24


<210>  23
<211>  24
<212>  DNA
<213>  Artificial

<220>
<223>  5TTT_P DNA target

<400>  23
tcaaaacttt gtacaaagtt ttga                                              24


<210>  24
<211>  22
<212>  DNA
<213>  Artificial

<220>
<223>  B2M11 P DNA target

<400>  24
tgaaattagg tacaaagtca ga                                                22


<210>  25
<211>  22
<212>  DNA
<213>  Artificial

<220>
<223>  B2M11.2 DNA target

<400>  25
tgaaattagg tacctaattt ca                                                22


<210>  26
<211>  22
<212>  DNA
<213>  Artificial

<220>
<223>  B2M11.3 DNA target

<400>  26
tctgactttg tacaaagtca ga                                                22


<210>  27
<211>  50
<212>  DNA
<213>  Artificial

<220>
<223>  oligonucleotide

<400>  27
tggcatacaa gttttgttct caggtacctg agaacaacaa tcgtctgtca                  50


<210>  28
<211>  77
<212>  DNA
<213>  Artificial

<220>
<223>  attB1-ICreIFor primer

<400>  28
ggggacaagt ttgtacaaaa aagcaggctt cgaaggagat agaaccatgg ccaataccaa      60

atataacaaa gagttcc                                                    77

<210>  29
<211>  64
<212>  DNA
<213>  Artificial

<220>
<223>  attB2-ICreIRev primer

<400>  29
ggggaccact ttgtacaaga aagctgggtt tagtcggccg ccggggagga tttcttcttc      60

tcgc                                                                   64

<210>  30
<211>  28
<212>  DNA
<213>  Artificial

<220>
<223>  M1s primer

<400>  30
aaaaagcagg ctgattggca tacaagtt                                         28

<210>  31
<211>  28
<212>  DNA
<213>  Artificial

<220>
<223>  M2s primer

<400>  31
agaaagctgg gtgattgaca gacgattg                                         28

<210>  32
<211>  25
<212>  DNA
<213>  Artificial

<220>
<223>  attB1adapbis primer

<400>  32
ggggacaagt ttgtacaaaa aagca                                            25

<210>  33
<211>  25
<212>  DNA
<213>  Artificial

<220>
<223>  attB2adapbis primer

<400>  33
ggggaccact ttgtacaaga aagct                                            25

<210>  34
<211>  24
<212>  DNA
<213>  Artificial

<220>
<223>  HprCH3 DNA target

<400>  34
tcgagatgtc atgaaagaga tgga                                              24


<210>  35
<211>  83
<212>  DNA
<213>  Artificial

<220>
<223>  CCM2For primer

<400>  35
aagcagagct ctctggctaa ctagagaacc cactgcttac tggcttatcg accatggcca      60

ataccaaata taacaaagag ttc                                               83


<210>  36
<211>  69
<212>  DNA
<213>  Artificial

<220>
<223>  CCMRev primer

<400>  36
tctgatcgat tcaagtcagt gtctctctag atagcgagtc ggccgccggg gaggatttct      60

tcttctcgc                                                               69


<210>  37
<211>  33
<212>  DNA
<213>  Artificial

<220>
<223>  G19SF primer

<400>  37
gccggctttg tggactctga cggtagcatc atc                                    33


<210>  38
<211>  33
<212>  DNA
<213>  Artificial

<220>
<223>  G19SR primer

<400>  38

gatgatgcta ccgtcagagt ccacaaagcc ggc 33

<210> 39
<211> 84
<212> DNA
<213> Artificial

<220>
<223> CCM2For primer

<400> 39
aagcagagct ctctggctaa ctagagaacc cactgcttac tggcttatcg accatggcca 60

ataccaaata taacaaagag ttcc 84

<210> 40
<211> 38
<212> DNA
<213> Artificial

<220>
<223> CCMRevBis primer

<400> 40
ctgctctaga ttagtcggcc gccggggagg atttcttc 38

<210> 41
<211> 24
<212> DNA
<213> Artificial

<220>
<223> 10GTT_P DNA target

<400> 41
tcgttacgtc gtacgacgta acga 24

<210> 42
<211> 24
<212> DNA
<213> Artificial

<220>
<223> 10TGG_P DNA target

<400> 42
tctggacgtc gtacgacgtc caga 24

<210> 43
<211> 24
<212> DNA
<213> Artificial

<220>
<223> 5CAG_P DNA target

<400> 43
tcaaaaccag gtacctggtt ttga 24

<210>  44
<211>  24
<212>  DNA
<213>  Artificial

<220>
<223>  5GAG_P DNA target

<400>  44
tcaaaacgag gtacctcgtt ttga                                              24


<210>  45
<211>  22
<212>  DNA
<213>  Artificial

<220>
<223>  RAG1.10 DNA target

<400>  45
tgttctcagg tacctcagcc ag                                                22


<210>  46
<211>  22
<212>  DNA
<213>  Artificial

<220>
<223>  RAG1.10.2 DNA target

<400>  46
tgttctcagg tacctgagaa cg                                                22


<210>  47
<211>  22
<212>  DNA
<213>  Artificial

<220>
<223>  RAG1.10.3 DNA target

<400>  47
ttggctgagg tacctcagcc ag                                                22


<210>  48
<211>  163
<212>  PRT
<213>  Chlamydomonas reinhardtii

<400>  48

Met Asn Thr Lys Tyr Asn Lys Glu Phe Leu Leu Tyr Leu Ala Gly Phe
1               5                   10                  15


Val Asp Gly Asp Gly Ser Ile Ile Ala Gln Ile Lys Pro Asn Gln Ser
            20                  25                  30

Tyr Lys Phe Lys His Gln Leu Ser Leu Thr Phe Gln Val Thr Gln Lys
        35                  40                  45

Thr Gln Arg Arg Trp Phe Leu Asp Lys Leu Val Asp Glu Ile Gly Val
    50                  55                  60

Gly Tyr Val Arg Asp Arg Gly Ser Val Ser Asp Tyr Ile Leu Ser Glu
65                  70                  75                  80

Ile Lys Pro Leu His Asn Phe Leu Thr Gln Leu Gln Pro Phe Leu Lys
                85                  90                  95

Leu Lys Gln Lys Gln Ala Asn Leu Val Leu Lys Ile Ile Glu Gln Leu
            100                 105                 110

Pro Ser Ala Lys Glu Ser Pro Asp Lys Phe Leu Glu Val Cys Thr Trp
        115                 120                 125

Val Asp Gln Ile Ala Ala Leu Asn Asp Ser Lys Thr Arg Lys Thr Thr
    130                 135                 140

Ser Glu Thr Val Arg Ala Val Leu Asp Ser Leu Ser Glu Lys Lys Lys
145                 150                 155                 160

Ser Ser Pro

<210>  49
<211>  9
<212>  PRT
<213>  artificial sequence

<220>
<223>  HA-tag

<400>  49

Tyr Pro Tyr Asp Val Pro Asp Tyr Ala
1               5

<210>  50
<211>  15
<212>  PRT
<213>  artificial sequence

<220>
<223>  S-tag

<400>  50

Lys Glu Thr Ala Ala Ala Lys Phe Glu Arg Gln His Met Asp Ser

1               5                   10                  15


<210>  51
<211>  5
<212>  PRT
<213>  artificial sequence

<220>
<223>  NLS

<400>  51

Lys Lys Lys Arg Lys
1               5


<210>  52
<211>  30
<212>  PRT
<213>  artificial sequence

<220>
<223>  peptidic linker

<400>  52

Gly Gly Ser Asp Lys Tyr Asn Gln Ala Leu Ser Lys Tyr Asn Gln Ala
1               5                   10                  15


Leu Ser Lys Tyr Asn Gln Ala Leu Ser Gly Gly Gly Gly Ser
            20                  25                  30


<210>  53
<211>  21
<212>  DNA
<213>  artificial sequence

<220>
<223>  primer

<400>  53
cgcaaatggg cggtaggcgt g                                                21


<210>  54
<211>  35
<212>  DNA
<213>  artificial sequence

<220>
<223>  primer

<400>  54
gtacagcagg aactcttcgt tatatttggt attgg                                 35


<210>  55
<211>  34
<212>  DNA
<213>  artificial sequence

<220>
<223>  primer

<400>  55
aataccaaat ataacgaaga gttcctgctg tacc                                34


<210>  56
<211>  24
<212>  DNA
<213>  artificial sequence

<220>
<223>  primer

<400>  56
cgtagaatcg agaccgagga gagg                                           24


<210>  57
<211>  32
<212>  DNA
<213>  artificial sequence

<220>
<223>  primer

<400>  57
caggtacagc aggaactttt tgttatattt gg                                  32


<210>  58
<211>  34
<212>  DNA
<213>  artificial seqeunce

<220>
<223>  primer

<400>  58
accaaatata acaaaaagtt cctgctgtac ctgg                                34


<210>  59
<211>  33
<212>  DNA
<213>  artificial sequence

<220>
<223>  primer

<400>  59
gatgatgcta ccgtcagagt ccacaaagcc ggc                                 33


<210>  60
<211>  33
<212>  DNA
<213>  artificial sequence

<220>
<223>  primer

<400>  60
gccggctttg tggactctga cggtagcatc atc                                    33


<210>  61
<211>  22
<212>  DNA
<213>  artificial sequence

<220>
<223>  C1 DNA target

<400>  61
cgagatgtca cacagaggta cg                                                22


<210>  62
<211>  35
<212>  DNA
<213>  artificial sequence

<220>
<223>  primer

<400>  62
gcaatgatgg agccatcaga atccacaaat ccagc                                  35


<210>  63
<211>  35
<212>  DNA
<213>  artificial sequence

<220>
<223>  primer

<400>  63
gctggatttg tggattctga tggctccatc attgc                                  35


<210>  64
<211>  33
<212>  DNA
<213>  artificial sequence

<220>
<223>  primer

<400>  64
aagatacagc aggaactttt tgttagagcc acc                                    33


<210>  65
<211>  33
<212>  DNA
<213>  artificial sequence

<220>
<223>  primer

<400>  65
ggtggctcta acaaaaagtt cctgctgtat ctt                                    33

<210>  66
<211>  33
<212>  DNA
<213>  artificial sequence

<220>
<223>  primer

<400>  66
aagatacagc aggaactctt cgttagagcc acc                                    33


<210>  67
<211>  33
<212>  DNA
<213>  artificial sequence

<220>
<223>  primer

<400>  67
ggtggctcta acgaagagtt cctgctgtat ctt                                    33


<210>  68
<211>  22
<212>  PRT
<213>  artificial sequence

<220>
<223>  peptidic linker

<400>  68

Ala Ala Gly Gly Gly Gly Ser Gly Gly Gly Gly Ser Gly Gly Gly Gly
1               5                   10                  15


Ser Gly Gly Gly Gly Ser
            20


<210>  69
<211>  24
<212>  DNA
<213>  artificial sequence

<220>
<223>  HBB5 target

<400>  69
ttggtctcct taaacctgtc ttga                                              24


<210>  70
<211>  24
<212>  DNA
<213>  artificial sequence

<220>
<223>  HBB5.3 target

<400>  70
ttggtctcct gtacaggaga ccaa                                              24


<210>  71
<211>  24
<212>  DNA
<213>  artificial sequence

<220>
<223>  HBB5.4 target

<400>  71
tcaagacagg gtaccctgtc ttga                                              24


<210>  72
<211>  33
<212>  DNA
<213>  artificial sequence

<220>
<223>  V105ARev primer

<400>  72
ttcgataatt ttcagagcca ggtttgcctg ttt                                    33


<210>  73
<211>  33
<212>  DNA
<213>  artificial sequence

<220>
<223>  V105AFor primer

<400>  73
aaacaggcaa acctggctct gaaaattatc gaa                                    33


<210>  74
<211>  33
<212>  DNA
<213>  artificial sequence

<220>
<223>  I132V Rev primer

<400>  74
atcgttcaga gctgcaacct gatccaccca ggt                                    33


<210>  75
<211>  33
<212>  DNA
<213>  artificial sequence

<220>
<223>  I132V For primer

<400>  75
acctgggtgg atcaggttgc agctctgaac gat                                    33